# EUROPÄISCHE PATENTSCHRIFT

(11) **EP 2 054 044 B1**
(45) Veröffentlichungstag und Bekanntmachung des Hinweises auf die Patenterteilung: **23.07.2014**
(21) Anmeldenummer: 07722473.1
(22) Anmeldetag: 26.05.2007
(51) Int. Cl.: A61K 31/04, A61K 31/661, A61K 31/6615, A61K 39/08, A61K 31/336, C07K 14/33, C07K 16/12, C07K 16/40

(54) **ARZNEIMITTEL GEGEN LCT-VERGIFTUNGEN**
MEDICAMENT FOR LCT POISONING
MÉDICAMENT CONTRE LES EMPOISONNEMENTS DUS À DES TOXINES LCT

(30) Priorität: 02.08.2006 DE 102006036373; 26.01.2007 DE 102007004938
(43) Veröffentlichungstag der Anmeldung: 06.05.2009
(73) Patentinhaber: Johannes Gutenberg-Universität Mainz, 55122 Mainz (DE)
(72) Erfinder: VON EICHEL-STREIBER, Christoph, 55444 Schweppenhausen (DE); REINEKE, Jessica, 65307 Bad Schwalbach (DE); TENZER, Stefan, 55130 Mainz (DE); SCHILD, Hansjörg, 55270 Schwabenheim (DE); RUPNIK, Maja, 3241 Limbus (SI)
(74) Vertreter: Rudolph, Ulrike
(86) Internationale Anmeldenummer: PCT/DE2007/000957
(87) Internationale Veröffentlichungsnummer: WO 2008/014733

(56) Entgegenhaltungen:
- WO-A1-99/24461
- RODE K ET AL: "The presence of manganese and potassium activates the Clostridium difficile Toxin B." NAUNYN-SCHMIEDEBERG'S ARCHIVES OF PHARMACOLOGY, Bd. 367, Nr. Supplement 1, März 2003 (2003-03), Seite R54, XP002453403 & 44TH SPRING MEETING OF THE DEUTSCHE GESELLSCHAFT FUER EXPERIMENTELLE UND KLINISCHE PHARMAKOLOGIE UND; MAINZ, GERMANY; MARCH 17-20, 2003 ISSN: 0028-1298
- FARUKI H ET AL: "EFFECT OF CALCIUM ON IN-VITRO ACTIVITY OF LY-146032 AGAINST CLOSTRIDIUM-DIFFICILE" ANTIMICROBIAL AGENTS AND CHEMOTHERAPY, Bd. 31, Nr. 3, 1987, Seiten 461-462, XP002453404 ISSN: 0066-4804
- REINEKE JESSICA ET AL: "Autocatalytic cleavage of Clostridium difficile toxin B." NATURE 22 MAR 2007, Bd. 446, Nr. 7134, 22. März 2007 (2007-03-22), Seiten 415-419, XP002453402 ISSN: 1476-4687
- MARTIN ET AL: "Metallopeptidase inhibitors of tetanus toxin: A combinatorial approach", JOURNAL OF MEDICINAL CHEMISTRY, AMERICAN CHEMICAL SOCIETY, US, vol. 42, 1 January 1999 (1999-01-01), pages 515-525, XP002160420, ISSN: 0022-2623, DOI: 10.1021/JM981066W
- J. Chrzanowska ET AL: "Aspartic proteinase from Penicillium camemberti: Purification, properties, and substrate specificity", Enzyme and Microbial Technology, vol. 17, no. 8, 1 August 1995 (1995-08-01) , pages 719-724, XP55042411, ISSN: 0141-0229, DOI: 10.1016/0141-0229(94)00129-F

## Beschreibung

Die Erfindung betrifft Wirkstoffe zur Verwendung bei der Behandlung zur Vermeidung oder Linderung einer Vergiftung durch Large Clostridial Cytotoxins (LCTs), insbesondere Clostridium difficile Toxine A und B (TcdA und TcdB), Clostridium sordellii letales Toxin (TcsL) und Clostridium novyi α-Toxin (Tcnα).

Clostridium difficile ist ein strikt anaerob wachsender, Sporen bildender, grampositiver Keim, der erst Ende der 70er Jahre als etiologisches Agenz der Antibiotika-assoziierten Diarrhoe und der Pseudomembranösen Kolitis identifiziert wurde. Seit den 90er Jahren wird C. difficile als der bedeutendste Krankenhauskeim der entwickelten Länder angesehen. Als Konsequenz des sich ständig weiter ausbreitenden Einsatzes von Breitbandantibiotika steigt die Inzidenz von C. difficile Infektionen vor allem bei stationär behandelten Patienten weiter stetig an.

Für die C. difficile-assozierten Erkrankungen sind die von C. difficile produzierten Exotoxine Toxin A (TcdA) und Toxin B (TcdB) verantwortlich. Es existieren verschiedene Stämme mit unterschiedlicher Virulenz und Toxinproduktion. Ungefähr ein Viertel aller Stämme produziert keine Toxine. Toxinbildende Stämme produzieren fast immer beide Toxine. TcdA ist ein Enterotoxin, das durch zytotoxische Schädigung der Enterozyten die Permeabilität der Darmschleimhaut erhöht und damit eine Diarrhoe auslöst. TcdB ist ein Zytotoxin, das den Elektrolyttransport stört und für Flüssigkeitsverlust und Funktionsstörungen des Darmes verantwortlich ist. Die Toxine TcdA und TcdB gehören zur Gruppe der sogenannten Large Clostridial Cytotoxins (LCTs) und bestehen jeweils aus einer Peptidkette mit drei funktionalen Domänen, nämlich der C-terminalen Domäne, die für das Anbinden des Toxins an die Wirtszellmembran verantwortlich ist, der hydrophoben mittleren Domäne, die für den Translokationsprozess durch die zellulären Membranen (mit) verantwortlich gemacht wird, und die N-terminale Domäne, die eine Glykosyltransferase-Funktion aufweist und die toxische Aktivität des Moleküls vermittelt.

Zwar ist der Aufnahmeprozess der Toxine in die Wirtszelle noch nicht vollständig aufgeklärt, es gilt jedoch als Tatsache, dass die Toxine nach dem Anbinden an einen Wirtszellrezeptor per Endozytose in die Wirtszelle gelangen, und dass zur Entfaltung ihrer Toxizität die N-terminale katalytische Domäne abgespalten und in das Cytosol der Wirtszelle befördert wird. Dort glykosiliert die katalytische Domäne spezifisch GTPasen der Rho-Subfamilie (Rho, Rac und Cdc42), welche ihrerseits an einer Fülle von Signaltransduktionskaskaden beteiligt sind, und blockiert auf diese Weise die betreffenden Signaltransduktionsprozesse, was letztendlich zur Disaggregation des Cytoskeletts und zum Zelltod führt.

Im Stand der Technik wurde bisher davon ausgegangen, dass die Abspaltung der katalytischen N-terminalen Domäne der Toxin-Peptidketten von TcdA und TcdB und auch anderer "Large Clostridial Toxines" LCT von einer zellulären Protease katalysiert wird (Rupnik et al., 2005 und Pfeiffer et al., 2003). Ein entsprechender Nachweis konnte jedoch nicht erbracht werden.

Rupnik et al. 2005 beschreiben in-vitro-Experimente die zeigen, dass TcdB in einem einzigen proteolytischen Schritt in zwei Fragmente gespalten wird, von denen das kleinere, N-terminale Fragment die zytotoxische Funktion und die Glucosyltransferase-Funktion aufweist. Die Autoren vertreten die Auffassung, dass der Spaltungsprozess von einer eukaryontischen Pepstatin-sensitiven Protease der Wirtszelle katalysiert wird, die im Zytoplasma und an der zytoplasmischen Oberfläche intrazellulärer Membranen lokalisiert ist. Es wird gezeigt, dass bereits abgespaltete N-terminale Fragmente nicht in eukaryonte Zellen aufgenommen werden. In den beschriebenen "in-vitro assay" zur Identifizierung von Substanzen, die die proteolytische Spaltungsreaktion hemmen oder fordern, ist die Protease in der "cytosolic fraction" der Wirtszelle das Target für die Testsubstanzen.

Rode et al. 2003 beschreiben Untersuchungen zum Einfluss von zweiwertigen Ionen direkt auf die Glykosyltransferase-Aktivität in der toxischen Domäne der LCTs am Beispiel der Interaktion von TcdB mit Kalzium. Ihr Test-System ist eine zellfreie Mischung aus LCTs, kleinen G-Proteinen und radioaktiv-markierter UDP-Glukose.

Faruki et al 1987 beschreiben die Wirkung des Antibiotikums LY146032 auf das Wachstum von *Clostridium difficile* und den Einfluss von Kalzium-Ionen auf diese antimikrobielle Aktivität dieses Antibiotikums. Test-System sind C. *difficile* Bakterien in vitro auf Petrischalen. Es wird gezeigt, dass der Zusatz von Kalzium-Ionen die Effizienz des Antibiotikums LY146032 als Wachstumshemmer von C. *difficile* signifikant verstärkt.

Supuran et al. 2002 beschreiben in ihrem Übersichtsartikel die gegenwärtige Situation der Antibiotika gegen Bakterien, die zunehmende bakterielle Resistenzentwicklung und Möglichkeiten der Generierung neuer Generationen von antibakteriellen Antibiotika mit anderen Wirkungsmechanismen als den bekannten. Es wird der Stand der Technik im Design solcher Enzym-Inhibitoren mit potenziellen therapeutischen Anwendungen präsentiert sowie jüngste Fortschritte beim Einsatz von einigen dieser Proteasen in der Therapie. Die Aspartat-Protease von LCTs ist nicht erwähnt.

Martin et al. 1999 beschreiben Untersuchungen zum bakteriellen Protein Tetanustoxin (TeNt), das von dem Bakterium Clostridium tetani gebildet wird, zur Familie der Zink-Endopeptidasen gehört, und die Spaltung des Proteins Synaptobrevin im Neurotransmitter-Exocytose-Apparat eukaryontischer Zellen bewirkt. Tetanustoxin ist kein Toxine der "Large clostridial Toxin (LCT) family".

Die WO 99/24461) beschreibt eine Verbindung der allgemeinen chemischen Strukturformel (I) und diese enthaltende Arzneimittel, die zur Hemmung der Aktivität der Clostridien-Toxine Tetanustoxin und Botulinum-Neurotoxin (Serotyp A bis G) vorgeschlagen werden. (siehe Beschreibung S.1 Abs.1). Tetanustoxin und Botulinum-Neurotoxin sind keine Toxine der "Large clostridial Toxin (LCT) family".

Chrzanowska et al. 1995 beschreiben eine saure Proteinase von *Penicillium camemberti.* Als Substrat dieser Pilz-Protease sind ausschließlich eukaryontische Proteine bekannt.

Im Verlauf der Untersuchungen, die der vorliegenden Erfindung zugrunde liegen, wurde nun aber überraschend gefunden, dass die Spaltung von TcdA und TcdB ein autokatalytischer Prozess ist, der von Inositolphosphat (IP) initiiert wird, und dass folglich die Toxine von Clostridium difficile neben ihrer katalytischen Funktion der Glykosyltransferase auch noch die Funktion einer Protease zur Selbstspaltung bzw. autokatalytischen Spaltung besitzen.

Diese Proteasefunktion wurde als Aspartat-Protease identifiziert. Als katalytisches Zentrum der Protease funktion wurde die Proteinregion identifiziert, die die Aminosäuresequenz von Aminosäureposition AS 1653 bis AS 1678 des TcdB gemäß Sequenz Nr P18177 (SwissProt/TrEMBL) umfasst. Das für Aspartat-Proteasen charakteristische Motiv DXG (Rao et al. 1998) liegt an der Aminosäureseposition 1665.

Als Inositolphosphat-Bindungsstelle wurde die Proteinregion identifiziert, die die Aminosäuresequenz von Aminosäureposition AS 1517 bis AS 2142 des TcdB-Proteins gemäß Sequenz Nr. P18177 (SwissProt/TrEMBL) umfasst. Dieses Aminosäuresequenz stellt ein Inosin-5-Monophosphat-Dehydrogenase (IMPDH) Motiv dar, das sich aus zwei Bereichen, nämlich AS 1517 - AS 1593 und AS 1918 - AS 2142 zusammensetzt, die durch einen 325 Aminosäure langen Proteinabschnitt ohne Sequenzhomologie getrennt sind.

Zur Behandlung von Patienten mit C. difficile Infektionen wird zunächst das auslösende Antibiotikum abgesetzt, soweit dies möglich. Die weitere Behandlung erfolgt ausschließlich symptomatisch. Bei länger andauerndem oder schwerwiegendem Krankheitsverlauf, sowie wenn ein Absetzen des auslösenden Antibiotikums aus anderen Gründen nicht möglich ist, wird zur Therapie Metrondiazol oder Vancomycin verabreicht.

Die Nachteile der gängigen antimikrobiellen Therapie sind vielfältig. Entscheidend ist hierbei vor allem, daß sich eine Erkrankung, die infolge der Behandlung einer anderen Infektionssituation mit Antibiotika ausgelöst wurde, nicht effektiv mit einer antimikrobiellen Therapie heilen läßt. Hintergrund dafür ist die Tatsache, daß C. difficile im Darm gesunder Menschen nur verhältnismäßig selten vorkommt und sich gegenüber der normalen Darmflora nicht durchsetzen kann. Wird die normale Darmflora durch Antibiotika-Therapie zerstört, kann sich C. difficile durchsetzen und den Darm effektiv besiedeln. Die gegen C. difficile gerichtete Antibiotika-Therapie führt wiederum zur Zerstörung der Darmflora und verhindert damit ursächlich auch das Entstehen einer gesunden Darmflora. Dies erklärt auch die hohe Anzahl an Remissionen, die nach Beendigung der antimikrobiellen Therapie zu beobachten sind. Ein zusätzlicher Nachteil der gängigen Therapie ist das zunehmende Auftreten von multiresistenten C. difficile Stämmen in jüngster Zeit. Damit droht, daß auch die bislang einzige Therapie für C. difficile induzierte Erkrankungen nutzlos wird und die Anzahl an Todesfällen infolge von C. difficile Erkrankungen steigt. Hinzu kommt, daß die zur Behandlung einer C. difficile Infektion nötigen Antibiotika sehr teuer sind und normalerweise nur in begründeten Fällen als Reserveantibiotika eingesetzt werden.

Es besteht daher ein dringender Bedarf nach Medikamenten, die zur spezifischen Bekämpfung (Vermeidung, Beseitigung, Linderung) von C. difficile Infektionen geeignet sind, ohne dass das Risiko der Entwicklung von Resistenzen bei den Clostridien oder auch anderen Bakterien besteht, und ohne die natürliche Bakterienflora der betroffenen Patienten - insbesondere deren Darmflora - zu schädigen.

Aufgabe der vorliegenden Erfindung ist die Bereitstellung eines solchen Medikaments.

Eine Lösung der genannten Aufgabe besteht in der Bereitstellung von Wirkstoffen der eingangs genannten Art, die Inhibitoren oder Aktivatoren der autokatalytischen Proteaseaktivität von LCTs (Large Clostridial Cytotoxins), insbesondere der autokatalytischen Proteaseaktivität von Clostridium difficile Toxin A (TcdA) und/oder Clostridium difficile Toxin B (TcdB) und/oder Clostridium sordellii letales Toxin (TcsL) und/oder Clostridium novyi α-Toxin (Tcnα) darstellen und in den Ansprüchen spezifiziert sind.

Als Effektoren der autokatalytischen Proteaseaktivität von LCTs werden im folgenden sowohl Aktivatoren als auch Inhibitoren der autokatalytischen Proteaseaktivität von LCTs bezeichnet.

Ist der Wirkstoff bzw. Effektor ein Inhibitor, so beruht seine antitoxische Wirkung darauf, dass er die Proteaseaktivität des intakten Toxins, insbesondere des TcdB oder TcdA oder TcsL oder Tcnα, hemmt und somit die Abspaltung des cytotoxisch wirksamen Fragments mit Glucosyltranferasefunktion (im Fall von TcdB und TcdA ist das das 63kDa-Fragment) unterbindet.

Geeignete Inhibitoren sind chemische Substanzen, welche die Protease-Aktivität der Toxine inhibieren.

Der Begriff "chemische Substanz" bezeichnet in den vorstehenden und nachfolgenden Ausführungen sowohl anorganische als auch organische Verbindungen, Ionen und Peptide oder Proteine.

Bevorzugte Inhibitoren sind solche chemischen Substanzen, die die Protease-Aktivität irreversibel inhibieren. Ein Beispiel hierfür ist die Substanz EPNP (1,2-Epoxy-3-(p-Nitrophenoxy)-Propan). Die Substanz reagiert irreversibel mit Aspartatresten im katalytischen Zentrum von Proteasen und inhibiert so die proteolytische Wirkung. Weitere Protease-Inhibitoren sind dem Fachmann bekannt oder können von ihm mit bekannten Methoden (Computer-Modelling, High-Throughput-Screening) leicht identifizieren werden. Beispielsweise kann zur Durchführung eines "High-Troughput-Screenings" ein Peptid synthetisiert werden, dessen Aminosäure-Sequenz der Sequenz der Proteasespaltstelle der LCTs entspricht. Durch Kopplung dieses Peptids z.B. mit dem Farbstoff AMC (7-Amino,4-Methyl-Cumarin) mit Methoden, die dem Fachmann bekannt sind, kann eine Sonde generiert werden. Zur Durchführung des "High-Troughput-Screenings" wird anschließend die gelabelte Sonde mit dem Toxin und den Kandidaten-Substanzen zusammengebracht. Wird die Sonde gespalten, so kommt es zu Änderungen im Fluoreszenz-Spektrums. Diese Änderungen sind mit dem Fachmann geläufigen Methoden (Fluoreszenzdetektoren) leicht zu erfassen. Ansätze, in denen keine Änderung des Fluoreszenz-Spektrum zu beobachten sind, enthalten dann potentielle Protease-Inhibitoren.

Beispielhaft sei noch ein weiteres Verfahren zur Identifizierung von Substanzen, die die Aktivierung der autokatalytischen Protease-Aktivität der LCTs beeinflussen, beschrieben. Dazu können das Holotoxin oder auch geeignete Toxinfragmente verwendet werden, die z.B. mit einem Farbstoff gekoppelt sind, dessen Fluoreszenz im ungespaltenen Toxin oder Toxin-Fragment gequencht ist. Durch die autokatalytische Spaltung des Toxins bzw. der Toxinfragmente wird die quenchende Wirkung aufgehoben. Die Änderungen im Fluoreszenz-Spektrum können, wie beschrieben, leicht erfasst werden.

Besonders geeignete Inhibitoren, d.h. Effektoren mit Inhibitorfunktion sind chemische Substanzen, insbesondere Proteine und hierunter vor allem Antikörper, die die autokatalytische Proteaseaktivität der LCTs hemmen, indem sie mit dem aktive Zentrum der Protease interagieren.

Der Begriff "interagieren" bedeutet im vorliegenden Kontext jede Art von Wechselwirkung zwischen den LCTs und einer chemischen Substanz, insbesondere einem Protein, und umfasst insbesondere kovalente Bindungen wie z.B. DisulfidBindungen und nichtkovalente Bindungen wie z.B. van-der-Waals-Kräfte, hydrophobe oder elektrostatische Wechselwirkungen sowie Wasserstoffbrücken-Bindungen.

Bevorzugt sind dabei Proteine und hierunter vor allem Antikörper, die mit der TcdB-Proteinregion von AS 1500 bis AS 1800 , insbesondere von AS 1653 bis AS 1678 und vor allem mit dem DXG-Motiv an Position 1665 - jeweils gemäß TcdB-Aminosäuresequenz Nr. P18177 (SwissProt/TrEMBL) oder den hierzu äquivalenten bzw. homologen Proteinregionen von TcdA oder TcsL oder Tcnα interagieren. Bei diesen äquivalenten/homologen Proteinregionen handelt es sich im Fall von TcdA um den Aminosäuresequenzabschnitt von AS 1651 bis AS 1675 gemäß TcdA-Aminosäuresequenz Nr. P16154 (SwissProt/TrEMBL) mit dem DXG-Motiv an Aminosäureposition AS 1662, im Fall von TcsL um den Aminosäuresequenzabschnitt von AS 1654 bis AS 1679 gemäß TcsL-Aminosäuresequenz Nr. Q46342 (SwissProt/TrEMBL) mit dem DXG-Motiv an Aminosäureposition AS 1666, und im Fall von Tcnα um den Aminosäuresequenzabschnitt von AS 1641 bis AS 1665 gemäß Tcnα -Aminosäuresequenz Nr. Q46149 (SwissProt/TrEMBL).

Weitere geeignete Inhibitoren (Effektoren mit Inhibitorfunktion) sind chemische Substanzen, insbesondere Proteine und hierunter vor allem Antikörper, die die autokatalytische Proteaseaktivität der LCTs hemmen, indem sie die Interaktion des Inositolphosphates mit dem Toxin inhibieren. Indem die IP-Bindung verhindert wird, unterbleibt die proteolytische Spaltung der Toxine.

Bevorzugt sind dabei Proteine und hierunter vor allem Antikörper, die mit der TcdB-Proteinregion von AS 1400 bis AS 2300 , insbesondere von AS 1517 bis AS 2142 und vor allem von AS 1517 bis AS 1593 oder AS 1918 bis AS 2142 - jeweils gemäß TcdB-Aminosäuresequenz Nr. P18177 (SwissProt/TrEMBL) - oder mit den hierzu äquivalenten bzw. homologen Proteinregionen der Toxine TcdA oder TcsL oder Tcnα interagieren.

Ebensogut können auch Antikörper oder andere Proteine generiert werden, die nicht direkt mit der Inositolphosphat-Bindungsstelle, insbesondere den vorgenannten Proteinregionen interagieren, sondern gegen benachbarte Regionen gerichtet sind und die IP-Bindung sterisch behindern und damit die proteolytische Spaltung der Toxine verhindern. Außerdem können Antikörper oder andere Proteine generiert werden, die nicht direkt mit dem DXG-Motiv der Proteasefunktion der LCTs interagieren, sondern durch Bindung in benachbarten Proteinabschnitten die proteolytische Spaltung verhindern.

Geeignete Aktivatoren, d.h. Effektoren mit Aktivatorfunktion, sind chemische Substanzen, welche die Protease-Aktivität der Toxine aktivieren.

Die antitoxische Wirkung eines Aktivators beruht darauf, dass er die Proteaseaktivität des intakten Toxins, insbesondere des TcdB oder TcdA oder TcsL oder Tcnα initiert, noch bevor das Toxin derart an die Wirtszelle gebunden hat, dass das abgespaltenen Fragment in das Zellinnere (Cytosol) gelangen könnte. Der Aktivator bewirkt folglich eine Abspaltung des cytotoxisch wirksamen Fragments mit Glucosyltranferasefunktion (im Fall von TcdB und TcdA ist das das 63kDa-Fragment) außerhalb der Wirtszelle. Das cytotoxisch wirksame Fragment kann dann nicht mehr in das Zellinnere gelangen und dort seine cytotoxische Wirkung entfalten.

Ein besonders geeigneter Aktivator (Effektor mit Aktivatorfunktion) ist isoliertes (im Unterschied zu cytosolischem) Inositolphosphat (IP), vorzugsweise Inositolhexaphosphat (IP6).

Ein Medikament mit diesem Wirkstoff hat den Vorteil, dass im Patientendarm vorhandenes LCT, insbesondere TcdB und/oder TcdA und/oder TcsL und/oder Tcnα, durch das medikamentös zugeführte IP schon im Darm zur Spaltung veranlasst wird, das heißt noch bevor es an Darmzellen oder andere Körperzellen binden und toxisch wirken kann.

Die katalytischen Zentren der Proteasefunktion der LCTs können erfindungsgemäß auch als gezielt verabreichte Antigene (Impfungstoffe) zur Erzeugung einer Immunisierung gegen die Toxine verwendet werden. Gegenstand der vorliegenden Erfindung ist deshalb auch die Verwendung von LCT-Proteinfragmenten als Vaccine-Wirkstoff bei der Behandlung zur Vermeidung oder Linderung einer Vergiftung durch LCT (= Large Clostridial Cytotoxins), die dadurch gekennzeichnet ist, dass der oder die antigene(n) Wirkstoff(e) aus der folgenden Gruppe von Proteinfragmenten ausgewählt ist/sind :
- DXG-Motiv an Position 1665 der TcdB-Aminosäuresequenz Nr. P18177 (SwissProt/TrEMBL),
- die Aminosäurepositionen AS 1653 bis AS 1678 der TcdB-Aminosäuresequenz Nr. P 18177 (SwissProt/TrEMBL),
- die Aminosäurepositionen AS 1500 bis AS 1800 der TcdB-Aminosäuresequenz Nr. P 18177 (SwissProt/TrEMBL),
- das DXG-Motiv an Position 1662 der TcdA-Aminosäuresequenz Nr. P16154 (SwissProt/TrEMBL),
- die Aminosäurepositionen AS 1651 bis AS 1675 der TcdA-Aminosäuresequenz Nr. P 16154 (SwissProt/TrEMBL)
- das DXG-Motiv an Position 1666 der TcsL-Aminosäuresequenz Nr. Q46342 (SwissProt/TrEMBL)
- die Aminosäurepositionen AS 1654 bis AS 1679 der TcsL-Aminosäuresequenz Nr. Q46342 (SwissProt/TrEMBL),
- die Aminosäurepositionen AS 1641 bis AS 1665 der Tcnα-Aminosäuresequenz Nr. Q46149 (SwissProt/TrEMBL).

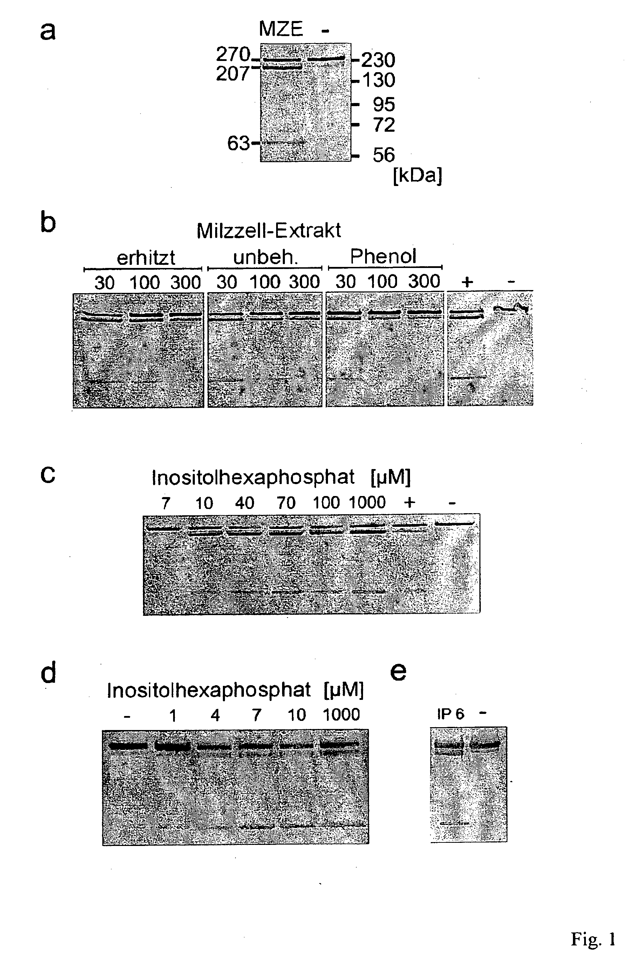

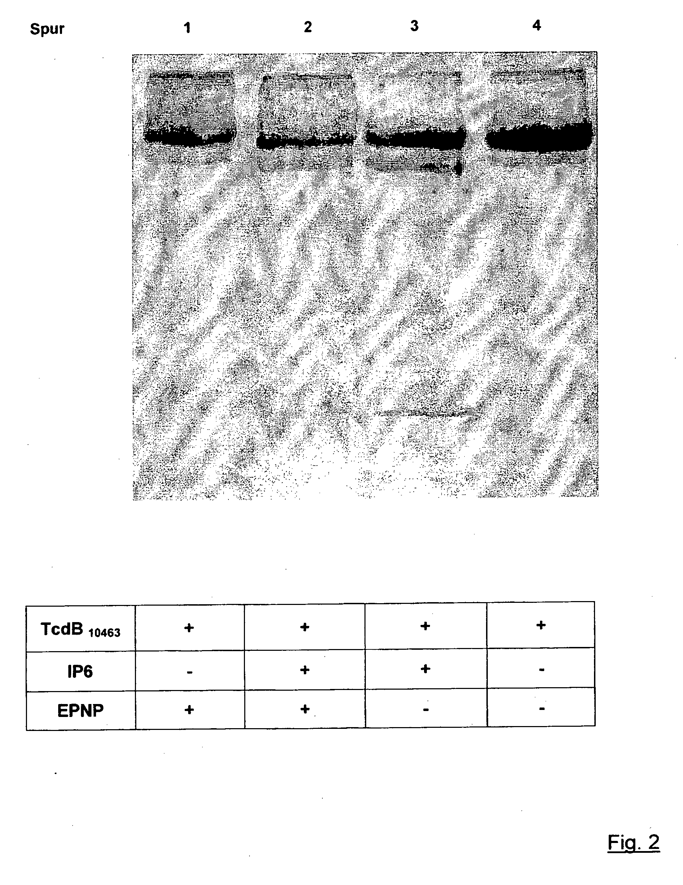

Die Erfindung wird im folgenden anhand von Ausführungsbeispielen und Figuren näher erläutert. Es zeigen
- **Figur 1:**: Aufspaltung des TcdB₁₀₄₆₃ (270 kDa) Holotoxins in die Translokations/Ligandendomäne (207 kDa) und die N-terminale katalytische Domäne (63 kDa) in der SDS-PAGE, durchgeführt mit
a: einer Mischung aus Cy3-markiertem TcdB₁₀₄₆₃ und Schweine-Milzzell-Extrakt (Beispiel 1 A);
b: einer Mischung aus Cy3-markiertem TcdB₁₀₄₆₃ und von Protein befreitem Schweine-Milzzell-Extrakt (Beispiel 1 B);
c: einer Mischung aus Cy3-markiertem TedB₁₀₄₆₃. und Inositolphosphat;
d: einer Mischung aus unmarkiertem TedB₁₀₄₆₃. und Inositolphosphat;
e: einer Mischung aus unmarkiertem (mittels Affinitätschromatographie gereinigtem) TedB₁₀₄₆₃ und Inositolphosphat;
a-e jeweils im Anschluß an eine Inkubation bei Raumtemperatur für 1 Stunde
- **Figur 2:**: SDS-PAGE einer Mischung von TcdB₁₀₄₆₃ und/oder IP6, mit oder ohne Vorbehandlung des Toxins mit EPNP;
Spur 1: TcdB₁₀₄₆₃ nach Vorbehandlung mit EPNP und ohne IP6, keine Bande im 63kDa Bereich nachweisbar;
Spur 2: TcdB₁₀₄₆₃ nach Vorbehandlung mit EPNP und nach Inkubation mit IP6, die typische 63kD Bande ist nur schwach ausgeprägt;
Spur 3: TcdB₁₀₄₆₃ ohne Vorbehandlung mit EPNP und nach Inkubation mit IP6, es ist eine deutlich ausgeprägte Bande im Molekulargewichtsbereich von 63kDa erkennbar;
Spur 4: TcdB₁₀₄₆₃ ohne Vorbehandlung mit EPNP und ohne IP6, keine Bande im 63kDa Bereich nachweisbar.

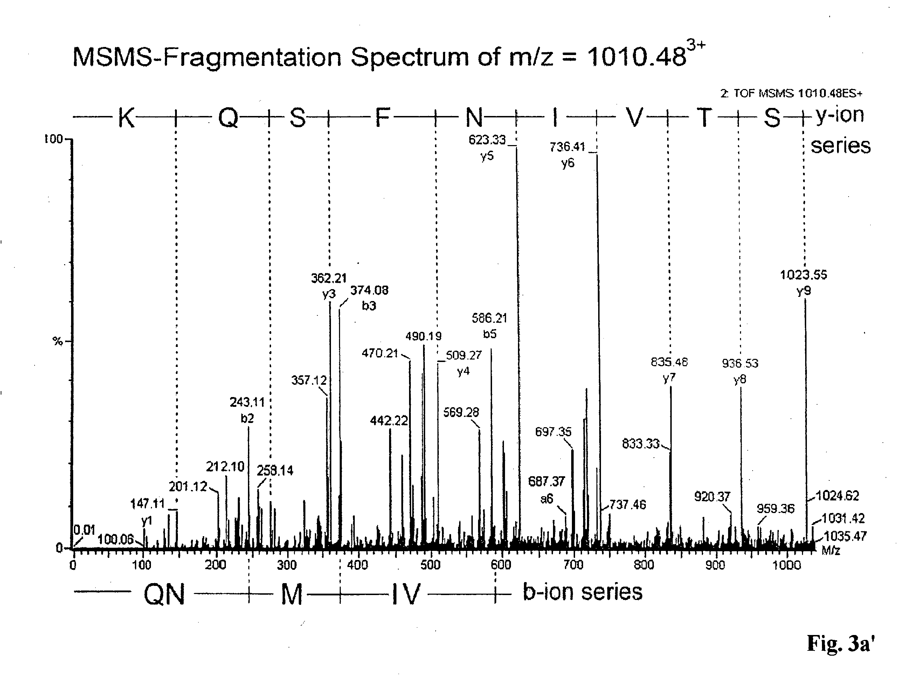

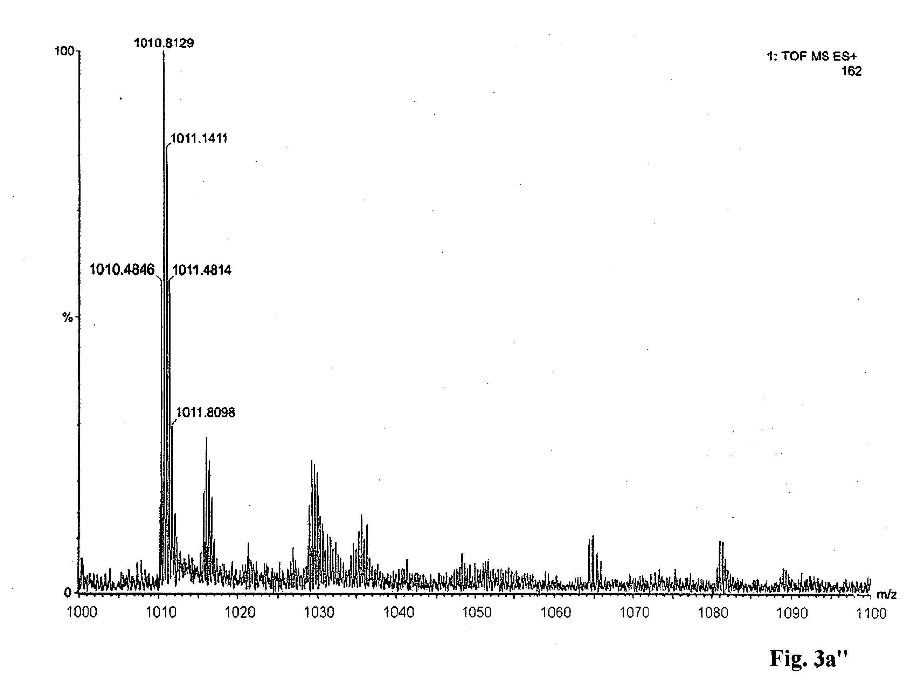

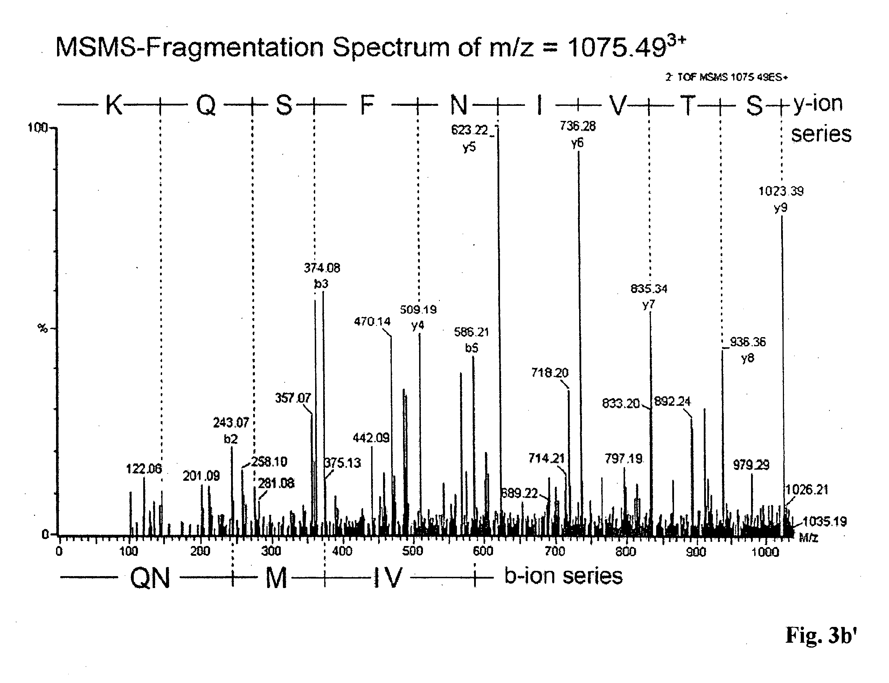

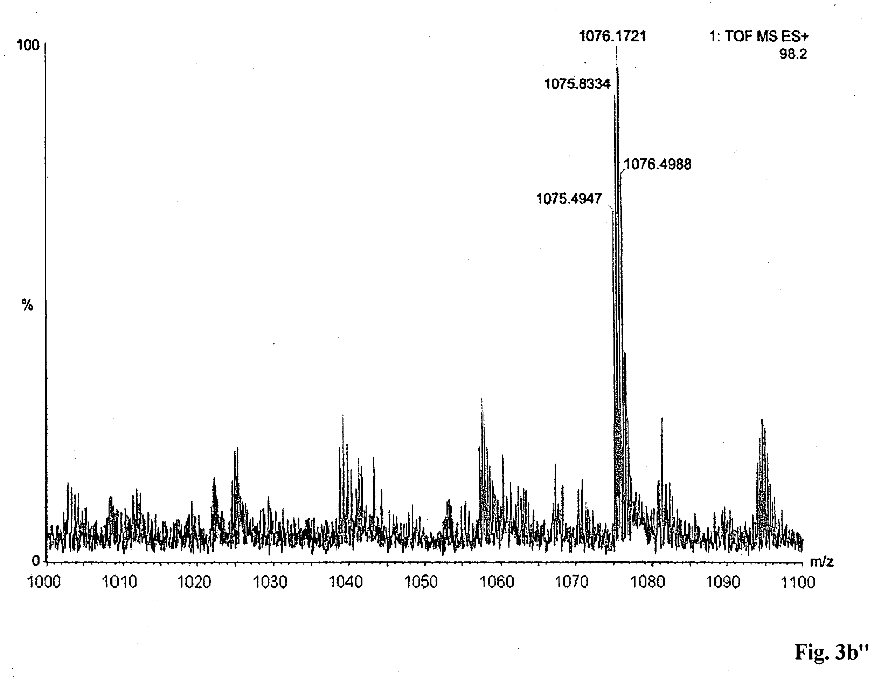

- **Figur 3:**: ESI-LCMSMS-Analyse nach tryptischem Verdau des nativen (a) und des EPNP-mödifizierten (b) TcdB-Proteins. MS-Survey Scans (großes Bild) und Fragmentation Spektren (Einschub).

Alle in den folgenden Beispielen genannten Verfahren sind dem Fachmann bekannt und z.B. in Ausubel et al. (2003) beschrieben.

### Beispiel 1: Nachweis der autokatalytischen Proteaseaktivität von TcdB

Clostridium difficile Toxin B (270 kDa) des Referenzstammes VPI10463, im folgenden kurz TedB₁₀₄₆₃ benannt, wurde zunächst mit Cy3 fluoreszenzmarkiert.

Hierfür wurden 200-400 µg TedB₁₀₄₆₃ (tgcBIOMICS, Mainz, Germany) mit dem Farbstoff Cy3 nach Angaben des Herstellers (Amersham Biosciences) markiert, indem das Toxin zusammen mit dem in Dimethylformamid gelösten Farbstoff für 1 Stunde bei 4°C inkubiert wurde. Ungebundener Farbstoff wurde anschließend mittels Größenausschlußchromatographie (Size Exclusion Chromatographie = SEC) entfernt, wobei 10 mM Tris-HCl pH 8.5 als Laufpuffer diente. Das molare Verhältnis zwischen Farbstoff und Cy3-markiertem TcdB₁₀₄₆₃ betrug 0.8 - 1.6. Markiertes TedB₁₀₄₆₃ wurde aliquotiert und bei -80 °C bis zur weiteren Verwendung gelagert.

(A) Für die Durchführung des im Stand der Technik bekannten "In-vitro-cleavage-assay" (siehe hierzu insbesondere Rupnik et al (2005); auf den Inhalt dieser Publikation wird hiermit ausdrücklich Bezug genommen) wurde ein auf Raumtemperatur aufgetautes Aliquot Cy3-markiertes TcdB₁₀₄₆₃ für 1 Stunde bei Raumtemperatur mit Schweine-Milzzell-Extrakt inkubiert.

Dieser Schweine-Milzzell-Extrakt wurde wie folgt hergestellt: Frisch gewonnene Schweinemilz wurde in Phosphat Puffer (PBS) aufgenommen und zu einer Einzelzellsuspension zerkleinert. Durch Zugabe von Niedrigsalzpuffer (Low Salt Buffer), - nämlich 150 mM NH₄Cl, 1 mM KHCO₃, 0.1 mM EDTA, pH 7.6 -, wurden vorhandene Erythrozyten lysiert und somit entfernt. Anschließend wurden die Milzzellen zweimal mit 10 mM Tris-HCl pH 8.5 gewaschen und sofort bei -80 °C tiefgefroren. Für den gewünschten Zellextrakt wurde bei Bedarf ein Aliquot dieser tiefgefrorenen Milzzellen in einem Aliquot 10 mM Tris-HCl pH 8.5 aufgetaut und suspensiert und diese Suspension anschließend einer Ultraschallbehandlung unterworfen. Das erhaltene Lysat wurde 1 Stunde bei 200 000 x g und 4 °C zentrifugiert und der Überstand für die anstehenden Experimente verwendet.

Am Ende der Inkubationsphase wurde die Mischung aus Cy3-markiertem TcdB₁₀₄₆₃ und Schweine-Milzzell-Extrakt einer SDS-PAGE unterworfen, um die während der Inkubation entstandenen TcdB-Fragmente zu separieren und nachzuweisen. Das Ergebnis dieser SDS-PAGE ist in **Fig. 1a** dargestellt: Es wurden erwartungsgemäß die beiden bekannten und charakteristischen 63 kDa- und 207 kDa-Fragmente des Clostridium difficile Toxins B - hier des TcdB₁₀₄₆₃ - erhalten.

Als Negativ-Kontrollen dienten Aliquots von TcdB₁₀₄₆₃ ohne Beimischung von Milzzell-Extrakt (siehe Fig. 1 "-").

(B) In einem Parallelversuch wurde der in (A) beschriebene Milzell-Extrakt vor der Inkubation mit dem Cy3-markierten TedB₁₀₄₆₃ von Protein gereinigt. Zu diesem Zweck wurde ein Aliquot des gemäß (A) hergestellten Schweine-Milzzell-Extrakts im Anschluß an die Untraschallbehandlung sechs Phenol-Chloroform-Extraktionen unterworfen, die wie folgt durchgeführt wurden: Schweine-Milzzell-Extrakt wurde mit Phenol-Chloroform-Isoamylalkohol (25/24/1) im Volumenverhältnis 1:1 gemischt. Diese Mischung wurde 10 Minuten bei 1.700 x g, und 4°C zentrifugiert. Die wässrige oberste Schicht wurde in eine frisches Zentrifugengefäß dekantiert und Zentrifugation und Dekantierung noch fünf mal wiederholt. Um auch letzte Reste von Phenol zu entfernen, wurde abschließend eine Chloroform-Exktraktion durchgeführt.

Aliquots der auf diese Weise gewonnenen wässrigen und proteinfreien Fraktion des Milzzell-Extraktes wurden im Volumenverhältnis 1:30 (30), 1:100 (100) oder 1:300 (300) mit 10mM Tris-HCL pH8,5 verdünnt und wie unter (A) beschrieben mit auf Raumtemperatur aufgetauten Aliquots von Cy3-markiertem TcdB₁₀₄₆₃ gemischt und für 1 Stunde bei Raumtemperatur inkubiert. Am Ende der Inkubationsphase wurden diese Mischungen einer SDS-PAGE unterworfen, um die während der Inkubation entstandenen TcdB-Fragmente zu separieren und nachzuweisen. Das Ergebnis dieser SDS-PAGE wurde mit Hilfe des Gel Doc EQ System Image Readers (BIO-RAD München, Deutschland) sichtbar gemacht und ist in **Fig. 1b** dargestellt: Es wurden bei allen Testansätzen die beiden charakteristischen 63 kDa- und 207 kDa-Fragmente von TcdB₁₀₄₆₃ erhalten. Das zeigt, dass die wässrige und proteinfreie Fraktion des Milzzell-Extraktes nach wie vor die Eigenschaft besitzt bzw. besaß, TcdB₁₀₄₆₃ in seine beiden charakteristischen Teilfragmente zu spalten.

In einem weiteren Parallelversuch wurde der in (A) beschriebene Milzzell-Extrakte mit Hitze (96°C, 30 Minuten) behandelt, bevor er wie in (A) beschrieben mit Cy3-markiertem TcdB₁₀₄₆₃ inkubiert und der SDS-PAGE unterworfen wurde. Das Ergebnis dieser SDS-PAGE ist ebenfalls in **Fig. 1b** dargestellt: Es wurden wiederum die beiden charakteristischen 63 kDa- und 207 kDa-Fragmente von TcdB₁₀₄₆₃ erhalten.

Das Ergebnis zeigt, dass der hitzeinduzierte Milzzell-Extrakt weiterhin die Eigenschaft besitzt, TcdB₁₀₄₆₃ in seine charakteristischen Teilfragmente zu spalten.

(C) In einer weiteren Versuchsreihe wurde Cy3-markiertes TcdB₁₀₄₆₃ und unmarkiertes TcdB₁₀₄₆₃ allein (d.h. ohne Beimischung von Milzzell-Extrakt) mit verschiedenen Inositolphosphaten für 1 Stunde bei Raumtemperatur inkubiert und die jeweiligen Mischungen anschließend einer SDS-PAGE unterworfen. Das Ergebnis dieser Untersuchungen ist in Tabelle 1 und den **Figuren 1c****-e** dargestellt. Es lieferte die überraschenden Kernaussagen, dass die proteolytische Spaltung von TcdB₁₀₄₆₃ allein durch eine chemische Substanz wie IP initiierbar ist, und dass es sich bei dieser proteolytischen Spaltung folglich um einen autokatalytischen Prozess des ToxinProteins handelt.

Aus Tabelle 1 ist ersichtlich, dass eine Reihe von Inositolphosphaten die autokatalytische Spaltung von TcdB₁₀₄₆₃ initieren können. Inositolhexaphosphat (IP₆) ruft unter den getesteten Inositolphosphaten die stärkste Spaltungsaktivität hervor, und das heißt, IP₆ besitzt die höchste Initiatoraktivität (siehe auch **Fig. 1c****-e**).

Strukturanaloga oder andere den Inositolphosphaten verwandte Substanzen, die eine Initiatoraktivität besitzen, sind dem Fachmann bekannt oder können mit bekannten Methoden (z.B. Computer-Modelling) leicht ermittelt werden. Beispielsweise können auch die vorstehend beschriebenen "High-Troughput-Assays" verwendet werden.

Die Austestung unterschiedlicher Konzentrationen an IP₆ im Inkubationsversuch mit TcdB₁₀₄₆₃ zeigt (siehe Fig. 1c und 1d), daß zur Spaltung von fluoreszenzmarkiertem Toxin B eine IP-Konzentrationen von 10 µM (**Fig 1c**) ausreichend ist, während zur Spaltung von unmarkiertem (und erst in der SDS-PAGE durch Zinkfärbung (Zinc Stain and Destain Kit, Biorad, Hercules, USA) sichtbar gemachtem) Toxin B noch niedrigere Konzentrationen bis zu 1 µM (**Fig 1d**) genügen.

Analoge Experimente wurden auch mit den LCTs TcdA₁₀₄₆₃, TcsL von C. sordellii und Tcnα durchgeführt. Dabei zeigte sich, daß Inositolphosphate aktivierend auf die autokatalytische Spaltung aller untersuchter Toxine der LCT-Familie wirken.

(D) Um auszuschließen, dass das in den Versuchen eingesetzte, aus Kulturüberstand von C. difficile aufgereinigte TcdB₁₀₄₆₃ mit Proteasen kontaminiert war, wurde ein Kontrollversuch mit speziell gereinigtem TcdB₁₀₄₆₃ durchgeführt. Diese Reinigung des TcdB₁₀₄₆₃ erfolgte mittels Affinitätschromatographie unter Einsatz des monoklonalen Antikörpers 2CV (DSM ACC 2321) wie folgt:

7mg des TcdB spezifischen monoklonalen Antikörper 2CV (ProteinG-gereinigter Überstand einer serumfreien Hybridoma-Kultur) wurde an eine HiTrap NHS Sepharose Säule (kommerziell erhältlich bei GE Healthcare, Freiburg, FRG) gekoppelt. Kopplung und Elution wurden gemäß Herstellerangaben durchgeführt. Die fertige Säule wurde mit ca. 4 mg TcdB₁₀₄₆₃ beschickt und ungebundene Proteine durch dreimaliges Waschen mit 50mM Tris/HCl, pH 7.0; 125 mM NaCl entfernt. Die Elution erfolgte in einem Schritt mit 0.1 M Triethanolamin-HCl pH11. Das Toxin eluierte in drei 4ml Fraktionen mit Konzentrationen zwischen 450-185 µg/ml Das eluierte Toxin wurde sofort mit 1M Tris-HCl pH 7.5 im Volumenverhältnis 1/10 neutralisiert, was dadurch gewährleistet war, daß diese Neutralisierungslösung bereits vor dem Start der Elution in den Auffangröhrchen für die Fraktionen vorgelegt worden war. Anschließend wurde die Abwesenheit von kontaminierenden Proteinen durch SDS-PAGE und nachfolgende Zinkfärbung nachgewiesen (siehe **Fig. 1** **e** "-").

Der Kontrollversuch bestand aus der Inkubation von derart gereinigtem unmarkiertem TcdB₁₀₄₆₃ mit IP₆ und nachfolgender SDS-PAGE und Nachweis des Toxins oder der Toxinfragmente mittels Zinkfärbung. Das Ergebnis dieses Versuchs ist in **Figur le** dargestellt und zeigt die vollständige Aufspaltung des Holotoxins in die beiden bekannten Fragmente 63 kDa und 207 kDa.

### Beispiel 2: Inaktivierung von TcdB-10463 durch Inkubation mit einem Protease-Inhibitor

TcdB₁₀₄₆₃ wurde wie in Beispiel 1 (D) beschrieben mittels Affinitätschromatographie unter Einsatz des monoklonalen Antikörpers 2CV gereinigt und anschließend entweder (i) mit dem Protease-Inhibitor EPNP (10mM 1,2-Epoxy-3-(p-Nitrophenoxy)-Propan) oder (ii) - als Kontrolle - mit Puffer (50mM HEPES, 1M NaCl, 1mM EDTA, pH 8,0) 60 Minuten bei Raumtemperatur vorbehandelt.

Anschließend wurde ein in-vitro-Cleavage-Assay analog Beispiel 1 (A) durchgeführt.. Die Testansätze umfaßten jeweils ein Volumen von 10 µl, und enthielten jeweils 50-100 ng unmarkiertes TcdB₁₀₄₆₃, 100 µM IP6 und 10 mM Tris-HCl pH 8.5. Diese Testansätze wurden im Anschluß an die Inkubation (1 h bei Raumtemperatur) einer SDS-PAGE (10%) unterworfen und die Toxine und Toxinfragmente anschließend mittels Zinkfärbung sichtbar gemacht.

Fig. 2 zeigt das Ergebnis dieses Experimentes: Die Inkubation von TcdB₁₀₄₆₃ allein mit IP6 (Spur 3) zeigt eine deutlich ausgeprägte Bande im Molekulargewichtsbereich von 63kDa. Wenn das Toxin vorher mit EPNP vorbehandelt wird (Spur 2), ist die typische 63kD Bande nur schwach ausgeprägt. Dieser Befund zeigt, dass durch die Zugabe von EPNP die proteolytische Aktivität (Protease-Aktivität) des TcdB₁₀₄₆₃ nahezu vollständig gehemmt ist.

Das mit EPNP vorbehandelte Toxin wurde außerdem im CHO-Test nach Moos et al. (2000) auf seine Restaktivität (zytotoxische Wirkung) hin untersucht.

Der CHO-Test wurde wie folgt durchgeführt: In einer 96well-Mikrotiterplatte wurden CHO-Zellen (= Chinese-Hamster-Ovarialcells) ausgesät (5000 Zellen/well) und 16 Stunden unter Standardbedingungen (5% CO₂, 37°C, DMEM F12 ergänzt mit 2mM L-Glutamine, 5% FCS) inkubiert. Anschließend wurden die Toxine nach stufenweiser Verdünnung in Wachstumsmedium zu den Zellen gegeben. Verdünnungstufen zwischen 10° und 10⁻⁸ wurden ausgetestet. Die Zellen wurden 3 Stunden unter Standardbedingungen inkubiert. Anschließend wurde der Anteil an abgerundeten Zellen mikroskopisch bestimmt, indem mehrere repräsentative Ausschnitte des Wells photographiert und die ausgestreckten, sowie die abgerundeten Zellen ausgezählt wurden. (Siehe auch Moos et al., Meth Enzymol. 2000, 325: 114-125. Auf den Inhalt dieser Publikation wird hier ausdrücklich Bezug genommen.)

Die Ergebnisse dieses CHO-Tests zeigen, daß das mit EPNP vorbehandelte TcdB₁₀₄₆₃ eine wesentlich schwächere zytotoxische Aktivität aufweist als das unbehandelte TcdB₁₀₄₆₃ (vgl. Tabelle 2). Da die inhibierende Wirkung von EPNP bekanntermaßen darauf beruht, daß EPNP mit katalytischen Aspartatresten in kovalente Wechselwirkungen tritt und dadurch eine irreversible Inaktivierung der Protease herbeiführt (Salto et al. 1994), zeigen die Ergebnisse des vorliegenden Versuchs, dass die Hemmung der TcdB₁₀₄₆₃-Aktivität auf der Hemmung einer Protease-Aktivität dieses Toxinmoleküls beruht.

Das hier beschriebene Experiment mit EPNP belegt, daß die toxische Wirkung von TcdB-₀₄₆₃ und anderer LCTs durch Vorbehandlung der Toxine mit einem geeigneten Protease-Inhibitor signifikant reduziert wird.

EPNP stellt eine Modellstubstanz für einen kovalenten Inhibitor der LCTs dar. Weitere vergleichbar kovalent wirkende oder kompetitiv hemmende Inhibitoren sind dem Fachmann bekannt oder können von diesem mit bekannten Methoden, beispielsweise mit den bereits beschriebenen "High-Troughput-Assays" ermittelt werden.

### Beispiel 3: Inaktivierung der zytotoxischen Wirkung von TcdB₁₀₄₆₃ durch extrazelluläre Aktivierung der Protease-Aktivität mit IP6

TcdB₁₀₆₃ wurde wie in Beispiel 1 (C) beschrieben mit 100 µM IP6 inkubiert. Im Anschluß an die Inkubation wurde das durch die Protease-Aktivität abgespaltene kleinere, 63kDa Fragment des Toxinproteins abgetrennt, indem der Ansatz über Microcon-Röhrchen (Millipore, Ausschlußgröße 100kD) aufgereinigt wurde. Dieses 63kDa Fragment von TcdB₁₀₄₆₃ wurde anschließend in dem in Beispiel 2 beschriebenen CHO-Test nach Moos et al. (2000) auf Abrundung der Zellen untersucht. Dabei wurde das Protein unverdünnt und in Verdünnungsstufen zu den Zellen gegeben.

Es zeigte sich in diesem Versuch, dass das 63 kDa-Fragment, welches die Glucosyltransferasefunktion von TcdB₁₀₄₆₃ aufweist, unter den gegebenen Bedingungen alleine nicht in der Lage ist/war, eine zytotoxische Wirkung hervorzurufen. Weder in verdünntem noch in unverdünntem Zustand konnte eine Abrundung der Zellen (infolge einer Glukosilierung spezifischer GTPasen der Rho-Subfamilie, daraus resultierender Blockierung von Signaltransduktionsprozessen und daraus resultierender Disaggregation des Cytoskeletts) beobachtet werden. Dieser Befund, dass das mittels Autokatalyse generierte Toxinfragment extrazellulär inaktiv ist, bestätigt die Ergebnisse von Pfeifer et al. (2003) und Rupnik et al (2005), die zeigen, dass die abgespaltene katalytische Domäne von TedB₁₀₄₆₃ nicht in eukaryonte Zellen aufgenommen wird und daher im Zellmedium inaktiv ist. (Während in diesen Arbeiten jedoch eine autokatalytische Aktivität der LCTs explizit ausgeschlossen wird [Pfeiffer et al., 2003] bzw. über eine zelluläre Protease zur Aktivierung der LCTs spekuliert wird [Rupnik et al., 2005], zeigen die im Zusammenhang mit der vorliegenden Erfindung gewonnenen Versuchsergebnisse erstmals, dass das N-terminale Toxinfragment autokatalytisch abgespalten wird.)

Die zytotoxische Wirkung von TcdB₁₀₄₆₃ und anderen LCTs kann folglich dadurch gehemmt werden, dass die proteolytische Spaltung der Toxine bereits vor Eindringen der Toxine in die Zellen induziert wird.

### Beispiel 4: Nachweis des aktiven Zentrums der Protease von TcdB

EPNP-inaktiviertes (vergl. Beispiel 2) und unbehandeltes TcdB 10463 wurden mittels SDS-Gel aufgetrennt und mit Zinkfärbung dargestellt. Anschließend wurden die den Proteinen entsprechenden Banden ausgeschnitten und in kleine Stücke zerteilt. Diese wurde entfärbt und getrocknet, anschließend in 2mM DTT reduziert und mit 20mM Iodacetamid alkyliert. Nach dem Waschen und erneuten Trocknen der Gelfragmente wurden diese mit Trypsin über Nacht bei 37°C verdaut. Die resultierenden Peptide wurden anschließend über HPLC (NanoAcquity ultraperformance Liquid Chromatography, Waters, Milford, USA) aufgetrennt. Dazu wurden 2µl der Proben auf eine reverse-phase Säule (NanoEase BEH C₁₈ (75µm x 10cm) von Waters, Milford, USA) in 2% mobile Phase B-Puffer (0,1% Ameisensäure in Acetonitril) aufgetragen. Mobile Phase A-Puffer enthielt 0,1% Ameisensäure in H₂O. Anschließend wurden die Fragmente durch einen Gradienten von 3-40% mobile Phase B-Puffer (90min bei 300nl/min) von der Säule eluiert.

Die eluierten Fragmente wurden anschließend massenspektrometrisch untersucht. Dazu wurde ein Q-Tof Premier Massenspektrometer der Firma Waters verwendet. Das Gerät wurde mit einer [Glu-1]-Fibrinogenpeptid-Lösung (500fmol/µl bei 300nl/min) über den Referenz-Sprayer der NanoLockSpray-Quelle (Waters) kalibiert. Zur Analyse der Ergebnisse wurde die MassLnyx4.1 Software (Waters) verwendet.

Die Analyse der Ergebnisse zeigt, dass sich das unbehandelte TcdB von dem EPNPbehandelten Toxin nur in einem tryptischen Fragment unterscheidet (Abb. 3). Dieses Fragment umfasst die Aminosäuren AS 1653 bis 1678 des TcdB-Proteins gemäß TcdB-Aminosäuresequenz Nr. P 18177 (SwissProt/TrEMBL) mit dem für Aspartat-Proteasen charakteristischen DXG-Motiv an Position 1665.

Ein Vergleich der Aminosäuresequenz AS 1653 bis AS 1678 des katalytischen Zentrums von TcdB mit den entsprechenden katalytischen Zentren und Proteinregionen der Toxine TcdA, TcsL und Tcnα zeigt, dass die Region hoch konserviert ist (siehe Tab. 3).

Der Fachmann kann daher mit bekannten Methoden Antikörper oder andere Proteine generieren, die spezifisch mit dem aktiven Zentrum der Protease-Domäne der Toxine interagieren und somit beispielsweise die autokatalytische Spaltung der LCTs verhindern. Ebensogut können auch Antikörper oder Proteine generiert werden, die nicht direkt das aktive Zentrum blockieren, sondern gegen benachbarte Regionen gerichtet sind und somit die autoproteolytische Spaltung der Toxin sterisch behindern.

### Beispiel 5: Inhibition der TcdB-Wirkung durch Antikörper infolge Immunisierung mit inaktiviertem TcdB

Um einen Schutz gegen die zytotoxischen Effekte des TcdB zu induzieren, wurden folgende TcdB-Präparationen hergestellt und zur Immunisierung in Kaninchen eingesetzt:
Präparation A: TcdB, inaktiviert mit Formalin
Präparation B: TcdB, inaktiviert mit EPNP (vergl. Beispiel 2)
Präparation C: TcdB-Fragment AS1601-1716 (DSG-Motiv)
Präparation D: TcdB-Fragment AS1508-1601 (Teil des Inosin-bindenden Motiv)
Präparation E: TcdB-Fragmente AS 1508-2157
   (DSG-Motiv und gesamtes Inosin-bindendes Motiv)

Die TcdB-Fragmente wurden mit dem Fachmann bekannten Methoden im Plasmid pET-19 (Novagen) exprimiert und über den angehängten His-Tag gereinigt. Anschließend wurde der His-Tag mittels Enterokinase-Verdau abgespalten. Die Reinheit des Proteins wurde im SDS-Gel überprüft (Daten nicht gezeigt)

Zur Immunisierung wurden Kaninchen mit dem Antigen zunächst grundimmunisiert und nachfolgend mehreren Boosterimmunisierungen unterworfen. Aus dem Blut der Kaninchen wurden schließlich polyklonale Antiseren erhalten.

Um die neutralisierende Wirkung der polyklonalen Antiseren zu überprüfen, wurde zunächst TcdB mit polyklonalem Antiserum vorbehandelt (Verdünnungsstufe 1:100) und 1h bei Raumtemperatur inkubiert. Anschließend wurde die neutralisierende Wirkung der Antiseren wie in Beispiel 2 beschrieben anhand des CHO-Tests überprüft. Das Maß für die neutralisierende Wirkung der Seren war, wie lange die Zellen vor der zytotoxischen Wirkung des TcdB geschützt waren.

Die mit Präparation A immunisierten Kaninchen wiesen nur einen geringen Antikörper-Titer auf (vergl. Tab. 4), zudem wirkte das Serum dieser Kaninchen nicht neutralisierend. Dieser Befund entspricht den dem Fachmann bekannten Ergebnissen von Immunisierungsversuchen mit formalisierten LCTs.

Die Tiere, die mit Präparation B immunisiert wurden, entwickelten zwar einen deutlichen Titer (ausverdünnbar bis 1:1500), allerdings wies auch dieses Antiserum keine neutralisierende Wirkung im CHO-Test auf (Tab. 4).

Die Kaninchen, die mit den Präparationen C bis E immunisiert worden waren, wiesen ebenfalls einen Antikörper-Titer auf, und zudem zeigten ihre polyklonalen Seren im CHO-Test eine deutlich neutralisierende Wirkung (Tab. 4). Das polyklonale Serum, das durch Immuniserung mit Präparation E generiert wurde, wies dabei die besten neutralisierenden Eigenschaften auf.

Die Seren, die durch separate Immunisierung mit den Präparationen C und D hergestellt wurden, zeigten demgegenüber eine geringere neutralisierende Wirkungen.

Der Erfolg der Immunisierung mit Fragment D, welches einen Teil der Inositolbindende Region umfasst, beweist, dass es sich bei diesem Abschnitt der LCTs um eine für die Aktivierung der Toxine wichtige Region handelt. Die Bindung des IP6 in diesem Toxin-Abschnitt führt zur Aktivierung der autokatalytischen Protease-Aktivität und somit auch zur Aktivierung der LCTs

Die neutralisierende Wirkung der Region um das DSG-Motif beweist, dass Antikörper, die gegen das aktive Zentrum der Protease gerichtet sind, die proteolytische Aktivität inhibieren können. Mit solchen Antikörpern kann somit auch in vivo ein Schutz vor den toxischen Effekten der LCTs erreicht werden.

Bei der Immunisierung mit Präparation E wurden nur die beiden Fragmente der erfolgreichen Präparationen C und D zusammen eingesetzt worden. Der Erfolg der Immunisierung mit beiden TcdB-Fragmenten beruht darauf, dass in den Tieren Antikörper induziert werden, die sowohl gegen das aktive Zentrum der Protease gerichtet sind, als auch solche Antikörper, die an die Inositol-Phosphat-bindende Region binden. Die Wirkung des Antiserums beruht also darauf, dass erstmals spezifische Antikörper induziert werden konnten, die gezielt die Aktivierung des Toxins verhindern.

Für die natürliche Aufnahme der LCTs in ihre Zielzellen ist die autokatalytische Spaltung der Toxine wichtig, da nur so das N-terminale, die eigentliche toxische Aktivität vermittelnde Fragment in den Zielzellen freigesetzt wird. Die Bindung spezifischer Antikörper in der Umgebung des DSG-Motifs der Aspartat-Protease und der Inositol-Phosphat-Bindungsstelle verhindert die autokatalytische Spaltung der Toxine. In Patienten kann somit durch Einsatz von Toxin-Fragmenten, die für die autokatalytische Spaltung der LCTs notwendig sind, eine wirksame Immunisierung gegen LCTs erreicht werden.

### Literatur:

Ausubel, F.M. et al.: "Current Protocols in Molecular Biology" (2003), John Wiley and Sons. Inc.
Rupnik et al. (2005) "Characterization of the cleavage site and function of resulting cleavage fragments after limited proteolysis of Clostridium difficile toxin B(TcdB) by host cells." Mikrobiol 151, 199-208.
Moos et al. (2000) "Purification and evaluation of large clostridial cytotoxins that inhibit small GTPases of Rho and Ras subfamilies" Meth Enzymol. 325: 114-125.
Pfeifer et al. (2003) "Cellular Uptake of Clostridium difficile toxins B" J. Biol. Chem. 278: 44535-41.
Rao et al. (1998) "Molecular and biotechnological aspects of microbial proteases" Microbiol. Mol. Biol. Rev. 62: 597-635.
Salto et al. (1994) "In vitro characterization of nonpeptide irreversible inhibitors of HIV proteases", J. Biol. Chem. 269: 10691-8.
Tang (1971) "Specific and irreversible inactivation of pepsin by substrate-like Epoxides", J. Biol. Chem. 246: 4510-17.

**Tabelle 1: Autokatalytische Spaltung von TcdB₁₀₄₆₃ unter Zugabe definierter Inositolphosphate**

| **IP** | **Konzentration** | | |
|---|---|---|---|
| | **1mM** | **100µM** | **10µM** |
| **1,5** | - | - | - |
| **1,4** | - | - | - |
| **4,5** | - | - | - |
| **1,4,5** | - | - | - |
| **2,3,5** | - | - | - |
| **1,3,5** | - | - | - |
| **1,3,5,6** | - | - | - |
| **1,2,3,4,6** | - | - | - |
| **1,3,4** | + | - | - |
| **1,3,4,5** | + | - | - |
| **3,4,6** | + | - | - |
| **1,2,3,4** | + | - | - |
| **1,2,3,4,5** | + | - | - |
| **1,2,3,5,6** | + | + | - |
| **1,3,4,5,6** | + | + | - |
| **3,4,5,6** | + | + | - |
| **2,3,4,5,6** | + | + | - |
| **1,4,5,6** | + | + | - |
| **1,2,3,4,5,6** | + | + | + |

**Tabelle 2: Zellabrundung (in %) von CHO-Zellen nach Inkubation mit TedB₁₀₄₆₃ - mit oder ohne EPNP-Vorbehandlung**

| **Verdünnung** | **Abgerundete Zellen [%] nach 3h** | |
|---|---|---|
| | **TcdB-10463** | **TcdB-10463 + EPNP** |
| 10⁻³ | 100% | 100% |
| 10⁻⁴ | 100% | 100% |
| 10⁻⁵ | 100% | 50% |
| 10⁻⁶ | 100% | 10% |
| 10⁻⁷ | 10% | <5% |
| 10⁻⁸ | <5% | <5% |

**Tabelle 3: Vergleich der Aminosäuresequenz AS 1653 bis AS 1678 des katalytischen Zentrums der Proteasefunktion von TcdB mit den entsprechenden katalytischen Zentren und Proteinregionen der Toxine TcdA, TcsL und Tcnα.**

| **Toxin** | **Homologer Sequenz-Bereich** |
|---|---|
| **TcdB-10463** | 1653-QNMIVEPNYDLD**DSG**DISSTVINFSQ-1678 |
| **TcdA-10463** | 1651-RNVVVEPIYNP**DTG**EDISTSL-DFSY-1675 |
| **TcsL** | 1654-QNLIVEPSYHLD**DSG**NISSTVINFSQ-1679 |
| **Tcnα** | 1641-CNVIVSGSNKLNSEGDLADT-IDVLD-1665 |

**Tabelle 4: Beginn der Zellabrundung (in h) von CHO-Zellen nach Inkubation mit TcdB, das mit polyklonalem Antiserum vorbehandelt worden war.**

| **Präparation** | **Antikörper-Titer** | **Beginn der Zellabrundung nach** |
|---|---|---|
| A | 1:100 | 1,5h |
| B | 1:1500 | 3h |
| C | 1:750 | 12-15h |
| D | 1:500 | 9-12h |
| E | 1:1000 | >24h |

## Patentansprüche

1. 1,2-Epoxy-3-(p-Nitrophenoxy)-Propan (EPNP) zur Verwendung bei der Behandlung zur Vermeidung oder Linderung einer Vergiftung durch LCT (= Large Clostridial Cytotoxins), nämlich durch Clostridium difficile Toxin A (TcdA) und/oder Clostridium difficile Toxin B (TcdB) und/oder Clostridium sordellii letales Toxin (TcsL) und/oder Clostridium novyi α-Toxin (Tcnα).

2. Inositolphosphat zur Verwendung bei der Behandlung zur Vermeidung oder Linderung einer Vergiftung durch LCTs (= Large Clostridial Cytotoxins), nämlich durch Clostridium difficile Toxin A (TcdA) und/oder Clostridium difficile Toxin B (TcdB) und/oder Clostridium sordellii letales Toxin (TcsL) und/oder Clostridium novyi α-Toxin (Tcnα).

3. Inositolhexaphosphat (IP6) zur Verwendung nach Anspruch 2.

4. Antikörper, der mit der TcdB-Proteinregion von AS 1653 bis AS 1678 gemäß TcdB-Aminosäuresequenz Nr. P18177 (SwissProt/TrEMBL) interagiert, zur Verwendung bei der Behandlung zur Vermeidung oder Linderung einer Vergiftung durch LCTs.

5. Antikörper, der mit dem DXG-Motiv an der Aminosäureposition AS 1665 des TcdB-Proteins gemäß TcdB-Aminosäuresequenz Nr. P18177 (SwissProt/TrEMBL) interagiert zur Verwendung bei der Behandlung zur Vermeidung oder Linderung einer Vergiftung durch LCTs.

6. Antikörper, der mit dem aktive Zentrum der Protease in der TcdA-Proteinregion von AS 1651 bis AS 1675 gemäß TcdA-Aminosäuresequenz Nr. P16154 (SwissProt/TrEMBL) interagiert zur Verwendung bei der Behandlung zur Vermeidung oder Linderung einer Vergiftung durch LCTs.

7. Antikörper, der mit dem DXG-Motiv an Aminosäureposition AS 1662 des TcdA-Proteins gemäß TcdA-Aminosäuresequenz Nr. P16154 (SwissProt/TrEMBL) interagiert zur Verwendung bei der Behandlung zur Vermeidung oder Linderung einer Vergiftung durch LCTs.

8. Antikörper, der mit dem aktive Zentrum der Protease in der TcsL -Proteinregion von AS 1654 bis AS 1679 gemäß TcsL -Aminosäuresequenz Nr Q46342 (SwissProt/TrEMBL) interagiert zur Verwendung bei der Behandlung zur Vermeidung oder Linderung einer Vergiftung durch LCTs.

9. Antikörper, der mit dem DXG-Motiv an Aminosäureposition AS 1666 des TcsL - Proteins gemäß TcsL -Aminosäuresequenz Nr. Q46342 (SwissProt/TrEMBL) interagiert zur Verwendung bei der Behandlung zur Vermeidung oder Linderung einer Vergiftung durch LCTs.

10. Antikörper, der mit dem aktive Zentrum der Protease in der Tcnα -Proteinregion von AS 1641 bis AS 1665 gemäß Tcnα-Aminosäuresequenz Nr Q46149 (SwissProt/TrEMBL) interagiert zur Verwendung bei der Behandlung zur Vermeidung oder Linderung einer Vergiftung durch LCTs.

11. Antikörper, der mit der TcdB-Proteinregion von AS 1517 bis AS 2142 gemäß TcdB-Aminosäuresequenz Nr. P 18177 (SwissProt/TrEMBL) oder mit der hierzu äquivalenten bzw. homologen Proteinregionen von TcdA oder TcsL oder Tcnα interagiert zur Verwendung bei der Behandlung zur Vermeidung oder Linderung einer Vergiftung durch LCTs.

12. Antikörper, der mit der TcdB-Proteinregion von AS 1517 bis AS 1593 gemäß TcdB-Aminosäuresequenz Nr. P18177 (SwissProt/TrEMBL) oder mit einer hierzu äquivalenten bzw. homologen Proteinregionen von TcdA oder TcsL oder Tcnα interagiert zur Verwendung bei der Behandlung zur Vermeidung oder Linderung einer Vergiftung durch LCTs.

13. Antikörper, der mit der TcdB-Proteinregion von AS 1918 bis AS 2142 gemäß TcdB-Aminosäuresequenz Nr. P 18177 (SwissProt/TrEMBL) oder mit einer hierzu äquivalenten bzw. homologen Proteinregionen von TcdA oder TcsL oder Tcnα interagiert zur Verwendung bei der Behandlung zur Vermeidung oder Linderung einer Vergiftung durch LCTs.

14. LCT-Proteinfragment (a) und/oder (b) und/oder (c) und/oder (d) zur Verwendung als Vaccine-Wirkstoff bei der Behandlung zur Vermeidung oder Linderung einer Vergiftung durch LCTs (= Large Clostridial Cytotoxins), nämlich durch Clostridium difficile Toxin A (TcdA) und/oder Clostridium difficile Toxin B (TcdB) und/oder Clostridium sordellii letales Toxin (TcsL) und/oder Clostridium novyi α-Toxin (Tcnα),
- wobei
(a) dasjenige TcdB-Proteinfragment der TcdB-Aminosäuresequenz Nr. P18177 (SwissProt/TrEMBL) ist, das wenigstens das DXG-Motiv an Position 1665 oder die Aminosäuresequenz von Aminosäurepositionen AS 1653 bis AS 1678 umfasst,
(b) dasjenige TcdA-Proteinfragment der TcdA-Aminosäuresequenz Nr. P16154 (SwissProt/TrEMBL) ist, das wenigstens das DXG-Motiv an Position 1662 oder die Aminosäuresequenz von Aminosäurepositionen AS 1651 bis AS 1675 umfasst,
(c) dasjenige TcsL-Proteinfragment der TcsL-Aminosäuresequenz Nr. Q46342 (SwissProt/TrEMBL) ist, das wenigstens das DXG-Motiv an Position 1666 oder die Aminosäuresequenz von Aminosäurepositionen AS 1654 bis AS 1679 umfasst, und
(d) dasjenige Tcnα-Proteinfragment der Tcnα-Aminosäuresequenz Nr. Q46149 (SwissProt/TrEMBL) ist, das wenigstens die Aminosäuresequenz von Aminosäurepositionen AS 1641 bis AS 1665 umfasst.

15. LCT-Autoprotease zur Verwendung als Target in einem Screening-Verfahren zur Identifizierung von Substanzen, die die autokatalytische Proteasefunktion von LCTs aktivieren oder inhibieren.

## Claims

1. 1,2-epoxy-3-(p-nitrophenoxy)-propan (EPNP) for use in the treatment to prevent or relief poisoning by LCTs (= Large Clostridial Cytotoxins), namely by Clostridium difficile Toxin A (TcdA) and/or Clostridium difficile Toxin B (TcdB) and/or Clostridium sordellii letales Toxin (TcsL) and/or Clostridium novyi α-Toxin (Tcnα).

2. Inositolphosphat for use in the treatment to prevent or relief poisoning by LCTs (= Large Clostridial Cytotoxins), namely by Clostridium difficile Toxin A (TcdA) and/or Clostridium difficile Toxin B (TcdB) and/or Clostridium sordellii letales Toxin (TcsL) and/or Clostridium novyi α-Toxin (Tcnα).

3. Inositolhexaphosphat (IP6) for use according to claim 2.

4. Antibody which interacts with the TcdB protein region of AS 1653 to AS 1678 according to TcdB amino acid sequence No. P18177 (SwissProt/TrEMBL) for use in the treatment to prevent or relief poisoning by LCTs.

5. Antibody which interacts with the DXG motif at the amino acid position AS 1665 of the TcdB protein according to TcdB amino acid sequence No. P18177 (SwissProt/TrEMBL) for use in the treatment to prevent or relief poisoning by LCTs.

6. Antibody which interacts with the active site of the protease in the TcdA protein region of AS 1651 to AS 1675 according to TcdA amino acid sequence No. P16154 (SwissProt/TrEMBL) for use in the treatment to prevent or relief poisoning by LCTs.

7. Antibody which interacts with the DXG motif at amino acid position AS 1662 of the TcdA protein according to TcdA amino acid sequence No. P16154 (SwissProt/TrEMBL) for use in the treatment to prevent or relief poisoning by LCTs.

8. Antibody which interacts with the active site of the protease in the TcsL protein region of AS 1654 to AS 1679 according to TcsL amino acid sequence No. Q46342 (SwissProt/TrEMBL) for use in the treatment to prevent or relief poisoning by LCTs.

9. Antibody which interacts with the DXG motif at amino acid position AS 1666 of the TcsL protein according to TcsL amino acid sequence No. Q46342 (SwissProt/TrEMBL) for use in the treatment to prevent or relief poisoning by LCTs.

10. Antibody which interacts with the active site of the protease in the Tcnα protein region of AS 1641 to AS 1665 according to Tcnα amino acid sequence No. Q46149 (SwissProt/TrEMBL) for use in the treatment to prevent or relief poisoning by LCTs.

11. Antibody which interacts with the TcdB protein region of AS 1517 to AS 2142 according to TcdB amino acid sequence No. P18177 (SwissProt/TrEMBL) or with the protein regions of TcdA or TcsL or Tcnα equivalent or homologous thereto, for use in the treatment to prevent or relief poisoning by LCTs.

12. Antibody which interacts with the TcdB protein region of AS 1517 to AS 1593 according to TcdB amino acid sequence No. P18177 (SwissProt/TrEMBL) or with a protein region of TcdA or TcsL or Tcnα equivalent or homologous thereto, for use in the treatment to prevent or relief poisoning by LCTs.

13. Antibody which interacts with the TcdB protein region of AS 1918 to AS 2142 according to TcdB amino acid sequence No. P18177 (SwissProt/TrEMBL) or with a protein region of TcdA or TcsL or Tcnα equivalent or homologous thereto, for use in the treatment to prevent or relief poisoning by LCTs.

14. LCTproteine fragment (a) and/or (b) and/or (c) and/or (d) for use as active component of a vaccine in the treatment to prevent or relief poisoning by LCTs (= Large Clostridial Cytotoxins), namely by Clostridium difficile Toxin A (TcdA) and/or Clostridium difficile Toxin B (TcdB) and/or Clostridium sordellii letales Toxin (TcsL) and/or Clostridium novyi α-Toxin (Tcnα),
- wherein
(a) is the TcdB protein fragment of TcdB amino acid sequence No. P18177 (SwissProt/TrEMBL) which comprises at least the DXG motif at position 1665, or the amino acid sequence of amino acid positions AS 1653 to AS 1678,
(b) is the TcdA protein fragment of the TcdA amino acid sequence No. P16154 (SwissProt/TrEMBL), which comprises at least the DXG motif at position 1662 or the amino acid sequence of amino acid positions AS 1651 to AS 1675
(c) is the TcsL protein fragment of the TcsL amino acid sequence No. Q46342 (SwissProt/TrEMBL), which comprises at least the DXG motif at position 1666, or the amino acid sequence of amino acid positions AS 1654 to AS 1679; and
(d) is the Tcnα protein fragment of the Tcnα amino acid sequence No. Q46149 (SwissProt/Tr EMBL), which comprises at least the amino acid sequence of AS 1641 to AS 1665.

15. LCT-autoprotease for use as target in a screening procedure for identifying a substance which activates or inhibits the autocatalytic protease activity of the LCT-autoprotease.

## Revendications

1. 1,2-époxy-3-(p-nitrophénoxy)-propane (EPNP) pour une utilisation dans le traitement en vue d'éviter ou d'atténuer un empoisonnement dû à des cytotoxines de Clostridium de poids moléculaire élevé (LCT = Large Clostridial Cytotoxins), à savoir par la toxine A de Clostridium difficile (TcdA) et/ou la toxine B de Clostridium difficile (TcdB) et/ou la toxine létale de Clostridium sordellii (TcsL) et/ou la toxine α de Clostridium novyi (Tcna).

2. Inositol phosphate pour une utilisation dans le traitement en vue d'éviter ou d'atténuer un empoisonnement dû à des cytotoxines de Clostridium de poids moléculaire élevé (LCT = Large Clostridial Cytotoxins), à savoir par la toxine A de Clostridium difficile (TcdA) et/ou la toxine B de Clostridium difficile (TcdB) et/ou la toxine létale de Clostridium sordellii (TcsL) et/ou la toxine α de Clostridium novyi (Tcnα).

3. Inositol héxaphosphate (IP6) pour une utilisation selon la revendication 2.

4. Anticorps qui interagit avec la région de la protéine TcdB comprise entre AS 1653 à AS 1678 selon la séquence d'acides aminés TcdB Nr P18177 (SwissProt/TrEMBL), pour une utilisation dans le traitement en vue d'éviter ou d'atténuer un empoisonnement dû aux LCT.

5. Anticorps qui interagit avec le motif DXG à la position de l'acide aminé AS 1665 de la protéine TcdB selon la séquence d'acides aminés TcdB Nr. P18177 (SwissProt/TrEMBL) pour une utilisation dans le traitement en vue d'éviter ou d'atténuer un empoisonnement dû aux LCT.

6. Anticorps qui interagit avec le site actif de la protéase situé dans la région de la protéine TcdA comprise entre AS 1651 et AS 1675 selon la séquence d'acides aminés TcdA Nr. P16154 (SwissProt/TrEMBL) pour une utilisation dans le traitement en vue d'éviter ou d'atténuer un empoisonnement dû aux LCT.

7. Anticorps qui interagit avec le motif DXG à la position de l'acide aminé AS 1662 de la protéine TcdA selon la séquence d'acides aminés TcdA Nr. P16154 (SwissProt/TrEMBL) pour une utilisation dans le traitement en vue d'éviter ou d'atténuer un empoisonnement dû aux LCT.

8. Anticorps qui interagit avec le site actif de la protéase situé dans la région de la protéine TcsL comprise entre AS 1654 et AS 1679 selon la séquence d'acides aminés TcsL Nr. Q46342 (SwissProt/TrEMBL) pour une utilisation dans le traitement en vue d'éviter ou d'atténuer un empoisonnement dû aux LCT.

9. Anticorps qui interagit avec le motif DXG à la position de l'acide aminé AS 1666 de la protéine TcsL selon la séquence d'acides aminés TcsL Nr. Q46342 (SwissProt/TrEMBL) pour une utilisation dans le traitement en vue d'éviter ou d'atténuer un empoisonnement dû aux LCT.

10. Anticorps qui interagit avec le site actif de la protéase situé dans la région de la protéine Tcnα comprise entre AS 1641 et AS 1665 selon la séquence d'acides aminés Tcnα Nr. Q46149 (SwissProt/TrEMBL) pour une utilisation dans le traitement en vue d'éviter ou d'atténuer un empoisonnement dû aux LCT.

11. Anticorps qui interagit avec la région de la protéine TcdB comprise entre AS 1517 à AS 2142 selon la séquence d'acides aminés TcdB Nr P18177 (SwissProt/TrEMBL) ou avec la région équivalente ou homologue de la protéine TcdA ou TcsL ou Tcnα pour une utilisation dans le traitement en vue d'éviter ou d'atténuer un empoisonnement dû aux LCT.

12. Anticorps qui interagit avec la région de la protéine TcdB comprise entre AS 1517 à AS 1593 selon la séquence d'acides aminés TcdB Nr P18177 (SwissProt/TrEMBL) ou avec une région équivalente ou homologue de la protéine TcdA ou TcsL ou Tcnα pour une utilisation dans le traitement en vue d'éviter ou d'atténuer un empoisonnement dû aux LCT.

13. Anticorps qui interagit avec la région de la protéine TcdB comprise entre AS 1918 à AS 2142 selon la séquence d'acides aminés TcdB Nr P18177 (SwissProt/TrEMBL) ou avec une région équivalente ou homologue de la protéine TcdA ou TcsL ou Tcnα pour une utilisation dans le traitement en vue d'éviter ou d'atténuer un empoisonnement dû aux LCT.

14. Fragment de protéine LCT (a) et/ou (b) et/ou (c) et/ou (d) pour une utilisation comme substance active d'un vaccin dans le traitement en vue d'éviter ou d'atténuer un empoisonnement dû aux cytotoxines de Clostridium de poids moléculaire élevé (Large Clostridial Cytotoxins / LCT), à savoir par la toxine A de Clostridium difficile (TcdA) et/ou la toxine B de Clostridium difficile (TcdB) et/ou la toxine létale de Clostridium sordellii (TcsL) et/ou la toxine α de Clostridium novyi (Tcnα),
- (a) étant le fragment de protéine de TcdB de la séquence d'acides aminés TcdB Nr P18177 (SwissProt/TrEMBL) qui comprend au moins le motif DXG à la position 1665 ou la séquence d'acides aminés des positions d'acides aminés comprises entre AS 1653 et AS 1678,
- (b) étant le fragment de protéine TcdA de la séquence d'acides aminés TcdA Nr. P16153 (SwissProt/TrEMBL) qui comprend au moins le motif DXG à la position 1662 ou la séquence d'acides aminés des positions d'acides aminées comprises entre AS 1651 et AS 1675,
- (c) étant le fragment de protéine TscL de la séquence d'acides aminés TcsL Nr. Q46342 (SwissProt/TrEMBL) qui comprend au moins le motif DXG à la position 1666 ou la séquence d'acides aminés des positions d'acides aminés comprises entre AS 1654 et AS 1679, et
- (d) étant le fragment de protéine Tcnα de la séquence d'acides aminés Tcnα Nr. Q46149 (SwissProt/TrEMBL) qui comprend au moins la séquence d'acides aminés des positions d'acides aminés comprises entre AS 1641 et AS 1665.

15. Autoprotéase LCT pour une utilisation comme cible dans un procédé de screening pour identifier des substances qui activent ou inhibent la fonction autocatalytique de la protéase.


### SEQUENCE LISTING

<110> Johannes Gutenberg-universität Mainz
<120> Arzneimittel gegen LCT-vergiftungen
<130> PCT/DE2007/000957
<140> EP07722473.1
   <141> 2007-05-26
<150> DE102006036373.6
   <151> 2006-08-02
<150> DE102007004938.4
   <151> 2007-01-26
<160> 19
<170> PatentIn version 3.3
<210> 1
   <211> 3
   <212> PRT
   <213> Clostridium difficile
<300>
   <308> P16154
   <309> 1990-04-01
   <313> (1662)..(1664)
<400> 1
   Asp Thr Gly
   1
<210> 2
   <211> 25
   <212> PRT
   <213> Clostridium difficile
<300>
   <308> P16154
   <309> 1990-04-01
   <313> (1651)..(1675)
<400> 2
   Arg Asn Val Val Val Glu Pro Ile Tyr Asn Pro Asp Thr Gly Glu Asp
   1               5                   10                  15
   Ile Ser Thr Ser Leu Asp Phe Ser Tyr
               20                  25
<210> 3
   <211> 2710
   <212> PRT
   <213> Clostridium sordellii
<220>
   <221> MISC_FEATURE
   <222> (1662)..(1664)
   <223> DXG motive
<300>
   <308> P16154
   <309> 1990-04-01
   <313> (1)..(2710)
<400> 3
   Met Ser Leu Ile Ser Lys Glu Glu Leu Ile Lys Leu Ala Tyr Ser Ile
   1               5                   10                  15
   Arg Pro Arg Glu Asn Glu Tyr Lys Thr Ile Leu Thr Asn Leu Asp Glu
               20                  25                  30
   Tyr Asn Lys Leu Thr Thr Asn Asn Asn Glu Asn Lys Tyr Leu Gln Leu
           35                  40                  45
   Lys Lys Leu Asn Glu Ser Ile Asp Val Phe Met Asn Lys Tyr Lys Thr
       50                  55                  60
   Ser Ser Arg Asn Arg Ala Leu Ser Asn Leu Lys Lys Asp Ile Leu Lys
   65                  70                  75                  80
   Glu Val Ile Leu Ile Lys Asn Ser Asn Thr Ser Pro Val Glu Lys Asn
                   85                  90                  95
   Leu His Phe Val Trp Ile Gly Gly Glu Val Ser Asp Ile Ala Leu Glu
               100                 105                 110
   Tyr Ile Lys Gln Trp Ala Asp Ile Asn Ala Glu Tyr Asn Ile Lys Leu
           115                 120                 125
   Trp Tyr Asp Ser Glu Ala Phe Leu Val Asn Thr Leu Lys Lys Ala Ile
       130                 135                 140
   Val Glu Ser Ser Thr Thr Glu Ala Leu Gln Leu Leu Glu Glu Glu Ile
   145                 150                 155                 160
   Gln Asn Pro Gln Phe Asp Asn Met Lys Phe Tyr Lys Lys Arg Met Glu
                   165                 170                 175
   Phe Ile Tyr Asp Arg Gln Lys Arg Phe Ile Asn Tyr Tyr Lys Ser Gln
               180                 185                 190
   Ile Asn Lys Pro Thr Val Pro Thr Ile Asp Asp Ile Ile Lys Ser His
           195                 200                 205
   Leu Val Ser Glu Tyr Asn Arg Asp Glu Thr Val Leu Glu Ser Tyr Arg
       210                 215                 220
   Thr Asn Ser Leu Arg Lys Ile Asn Ser Asn His Gly Ile Asp Ile Arg
   225                 230                 235                 240
   Ala Asn Ser Leu Phe Thr Glu Gln Glu Leu Leu Asn Ile Tyr Ser Gln
   245 250 255
   Glu Leu Leu Asn Arg Gly Asn Leu Ala Ala Ala Ser Asp Ile Val Arg
   260 265 270
   Leu Leu Ala Leu Lys Asn Phe Gly Gly Val Tyr Leu Asp Val Asp Met
   275 280 285
   Leu Pro Gly Ile His Ser Asp Leu Phe Lys Thr Ile Ser Arg Pro Ser
   290 295 300
   Ser Ile Gly Leu Asp Arg Trp Glu Met Ile Lys Leu Glu Ala Ile Met
   305 310 315 320
   Lys Tyr Lys Lys Tyr Ile Asn Asn Tyr Thr Ser Glu Asn Phe Asp Lys
   325 330 335
   Leu Asp Gln Gln Leu Lys Asp Asn Phe Lys Leu Ile Ile Glu Ser Lys
   340 345 350
   Ser Glu Lys Ser Glu Ile Phe Ser Lys Leu Glu Asn Leu Asn Val Ser
   355 360 365
   Asp Leu Glu Ile Lys Ile Ala Phe Ala Leu Gly Ser Val Ile Asn Gln
   370 375 380
   Ala Leu Ile Ser Lys Gln Gly Ser Tyr Leu Thr Asn Leu Val Ile Glu
   385 390 395 400
   Gln Val Lys Asn Arg Tyr Gln Phe Leu Asn Gln His Leu Asn Pro Ala
   405 410 415
   Ile Glu Ser Asp Asn Asn Phe Thr Asp Thr Thr Lys Ile Phe His Asp
   420 425 430
   Ser Leu Phe Asn Ser Ala Thr Ala Glu Asn Ser Met Phe Leu Thr Lys
   435 440 445
   Ile Ala Pro Tyr Leu Gln Val Gly Phe Met Pro Glu Ala Arg Ser Thr
   450 455 460
   Ile Ser Leu Ser Gly Pro Gly Ala Tyr Ala Ser Ala Tyr Tyr Asp Phe
   465 470 475 480
   Ile Asn Leu Gln Glu Asn Thr Ile Glu Lys Thr Leu Lys Ala Ser Asp
   485 490 495
   Leu Ile Glu Phe Lys Phe Pro Glu Asn Asn Leu Ser Gln Leu Thr Glu
   500 505 510
   Gln Glu Ile Asn Ser Leu Trp Ser Phe Asp Gln Ala Ser Ala Lys Tyr
           515                 520                 525
   Gln Phe Glu Lys Tyr Val Arg Asp Tyr Thr Gly Gly Ser Leu Ser Glu
       530                 535                 540
   Asp Asn Gly Val Asp Phe Asn Lys Asn Thr Ala Leu Asp Lys Asn Tyr
   545                 550                 555                 560
   Leu Leu Asn Asn Lys Ile Pro Ser Asn Asn Val Glu Glu Ala Gly Ser
                   565                 570                 575
   Lys Asn Tyr Val His Tyr Ile Ile Gln Leu Gln Gly Asp Asp Ile Ser
               580                 585                 590
   Tyr Glu Ala Thr Cys Asn Leu Phe Ser Lys Asn Pro Lys Asn Ser Ile
           595                 600                 605
   Ile Ile Gln Arg Asn Met Asn Glu Ser Ala Lys Ser Tyr Phe Leu Ser
       610                 615                 620
   Asp Asp Gly Glu Ser Ile Leu Glu Leu Asn Lys Tyr Arg Ile Pro Glu
   625                 630                 635                 640
   Arg Leu Lys Asn Lys Glu Lys Val Lys Val Thr Phe Ile Gly His Gly
                   645                 650                 655
   Lys Asp Glu Phe Asn Thr Ser Glu Phe Ala Arg Leu Ser Val Asp Ser
               660                 665                 670
   Leu Ser Asn Glu Ile Ser Ser Phe Leu Asp Thr Ile Lys Leu Asp Ile
           675                 680                 685
   Ser Pro Lys Asn Val Glu Val Asn Leu Leu Gly Cys Asn Met Phe Ser
       690                 695                 700
   Tyr Asp Phe Asn Val Glu Glu Thr Tyr Pro Gly Lys Leu Leu Leu Ser
   705                 710                 715                 720
   Ile Met Asp Lys Ile Thr Ser Thr Leu Pro Asp Val Asn Lys Asn Ser
                   725                 730                 735
   Ile Thr Ile Gly Ala Asn Gln Tyr Glu Val Arg Ile Asn Ser Glu Gly
               740                 745                 750
   Arg Lys Glu Leu Leu Ala His Ser Gly Lys Trp Ile Asn Lys Glu Glu
           755                 760                 765
   Ala Ile Met Ser Asp Leu Ser Ser Lys Glu Tyr Ile Phe Phe Asp Ser
       770                 775                 780
   Ile Asp Asn Lys Leu Lys Ala Lys Ser Lys Asn Ile Pro Gly Leu Ala
   785 790 795 800
   Ser Ile Ser Glu Asp Ile Lys Thr Leu Leu Leu Asp Ala Ser Val Ser
   805 810 815
   Pro Asp Thr Lys Phe Ile Leu Asn Asn Leu Lys Leu Asn Ile Glu Ser
   820 825 830
   Ser Ile Gly Asp Tyr Ile Tyr Tyr Glu Lys Leu Glu Pro Val Lys Asn
   835 840 845
   Ile Ile His Asn Ser Ile Asp Asp Leu Ile Asp Glu Phe Asn Leu Leu
   850 855 860
   Glu Asn Val Ser Asp Glu Leu Tyr Glu Leu Lys Lys Leu Asn Asn Leu
   865 870 875 880
   Asp Glu Lys Tyr Leu Ile Ser Phe Glu Asp Ile Ser Lys Asn Asn Ser
   885 890 895
   Thr Tyr Ser Val Arg Phe Ile Asn Lys Ser Asn Gly Glu Ser Val Tyr
   900 905 910
   Val Glu Thr Glu Lys Glu Ile Phe Ser Lys Tyr Ser Glu His Ile Thr
   915 920 925
   Lys Glu Ile Ser Thr Ile Lys Asn Ser Ile Ile Thr Asp Val Asn Gly
   930 935 940
   Asn Leu Leu Asp Asn Ile Gln Leu Asp His Thr Ser Gln Val Asn Thr
   945 950 955 960
   Leu Asn Ala Ala Phe Phe Ile Gln Ser Leu Ile Asp Tyr Ser Ser Asn
   965 970 975
   Lys Asp Val Leu Asn Asp Leu Ser Thr Ser Val Lys Val Gln Leu Tyr
   980 985 990
   Ala Gln Leu Phe Ser Thr Gly Leu Asn Thr Ile Tyr Asp Ser Ile Gln
   995 1000 1005
   Leu Val Asn Leu Ile Ser Asn Ala Val Asn Asp Thr Ile Asn Val
   1010 1015 1020
   Leu Pro Thr Ile Thr Glu Gly Ile Pro Ile Val Ser Thr Ile Leu
   1025 1030 1035
   Asp Gly Ile Asn Leu Gly Ala Ala Ile Lys Glu Leu Leu Asp Glu
   1040 1045 1050
   His Asp Pro Leu Leu Lys Lys Glu Leu Glu Ala Lys Val Gly Val
   1055 1060 1065
   Leu Ala Ile Asn Met Ser Leu Ser Ile Ala Ala Thr Val Ala Ser
   1070 1075 1080
   Ile Val Gly Ile Gly Ala Glu Val Thr Ile Phe Leu Leu Pro Ile
   1085 1090 1095
   Ala Gly Ile Ser Ala Gly Ile Pro Ser Leu Val Asn Asn Glu Leu
   1100 1105 1110
   Ile Leu His Asp Lys Ala Thr Ser Val Val Asn Tyr Phe Asn His
   1115 1120 1125
   Leu Ser Glu Ser Lys Lys Tyr Gly Pro Leu Lys Thr Glu Asp Asp
   1130 1135 1140
   Lys Ile Leu Val Pro Ile Asp Asp Leu Val Ile Ser Glu Ile Asp
   1145 1150 1155
   Phe Asn Asn Asn Ser Ile Lys Leu Gly Thr Cys Asn Ile Leu Ala
   1160 1165 1170
   Met Glu Gly Gly Ser Gly His Thr Val Thr Gly Asn Ile Asp His
   1175 1180 1185
   Phe Phe Ser Ser Pro Ser Ile Ser Ser His Ile Pro Ser Leu Ser
   1190 1195 1200
   Ile Tyr Ser Ala Ile Gly Ile Glu Thr Glu Asn Leu Asp Phe Ser
   1205 1210 1215
   Lys Lys Ile Met Met Leu Pro Asn Ala Pro Ser Arg Val Phe Trp
   1220 1225 1230
   Trp Glu Thr Gly Ala Val Pro Gly Leu Arg Ser Leu Glu Asn Asp
   1235 1240 1245
   Gly Thr Arg Leu Leu Asp Ser Ile Arg Asp Leu Tyr Pro Gly Lys
   1250 1255 1260
   Phe Tyr Trp Arg Phe Tyr Ala Phe Phe Asp Tyr Ala Ile Thr Thr
   1265 1270 1275
   Leu Lys Pro Val Tyr Glu Asp Thr Asn Ile Lys Ile Lys Leu Asp
   1280 1285 1290
   Lys Asp Thr Arg Asn Phe Ile Met Pro Thr Ile Thr Thr Asn Glu
   1295 1300 1305
   Ile Arg Asn Lys Leu Ser Tyr Ser Phe Asp Gly Ala Gly Gly Thr
   1310 1315 1320
   Tyr Ser Leu Leu Leu Ser Ser Tyr Pro Ile Ser Thr Asn Ile Asn
   1325 1330 1335
   Leu Ser Lys Asp Asp Leu Trp Ile Phe Asn Ile Asp Asn Glu Val
   1340 1345 1350
   Arg Glu Ile Ser Ile Glu Asn Gly Thr Ile Lys Lys Gly Lys Leu
   1355 1360 1365
   Ile Lys Asp Val Leu Ser Lys Ile Asp Ile Asn Lys Asn Lys Leu
   1370 1375 1380
   Ile Ile Gly Asn Gln Thr Ile Asp Phe Ser Gly Asp Ile Asp Asn
   1385 1390 1395
   Lys Asp Arg Tyr Ile Phe Leu Thr Cys Glu Leu Asp Asp Lys Ile
   1400 1405 1410
   Ser Leu Ile Ile Glu Ile Asn Leu Val Ala Lys Ser Tyr Ser Leu
   1415 1420 1425
   Leu Leu Ser Gly Asp Lys Asn Tyr Leu Ile Ser Asn Leu Ser Asn
   1430 1435 1440
   Thr Ile Glu Lys Ile Asn Thr Leu Gly Leu Asp Ser Lys Asn Ile
   1445 1450 1455
   Ala Tyr Asn Tyr Thr Asp Glu Ser Asn Asn Lys Tyr Phe Gly Ala
   1460 1465 1470
   Ile Ser Lys Thr Ser Gln Lys Ser Ile Ile His Tyr Lys Lys Asp
   1475 1480 1485
   Ser Lys Asn Ile Leu Glu Phe Tyr Asn Asp Ser Thr Leu Glu Phe
   1490 1495 1500
   Asn Ser Lys Asp Phe Ile Ala Glu Asp Ile Asn Val Phe Met Lys
   1505 1510 1515
   Asp Asp Ile Asn Thr Ile Thr Gly Lys Tyr Tyr Val Asp Asn Asn
   1520 1525 1530
   Thr Asp Lys Ser Ile Asp Phe Ser Ile Ser Leu Val Ser Lys Asn
   1535 1540 1545
   Gln Val Lys Val Asn Gly Leu Tyr Leu Asn Glu Ser Val Tyr Ser
   1550 1555 1560
   Ser Tyr Leu Asp Phe Val Lys Asn Ser Asp Gly His His Asn Thr
   1565 1570 1575
   Ser Asn Phe Met Asn Leu Phe Leu Asp Asn Ile Ser Phe Trp Lys
   1580 1585 1590
   Leu Phe Gly Phe Glu Asn Ile Asn Phe Val Ile Asp Lys Tyr Phe
   1595 1600 1605
   Thr Leu Val Gly Lys Thr Asn Leu Gly Tyr Val Glu Phe Ile Cys
   1610 1615 1620
   Asp Asn Asn Lys Asn Ile Asp Ile Tyr Phe Gly Glu Trp Lys Thr
   1625 1630 1635
   Ser Ser Ser Lys Ser Thr Ile Phe Ser Gly Asn Gly Arg Asn Val
   1640 1645 1650
   Val Val Glu Pro Ile Tyr Asn Pro Asp Thr Gly Glu Asp Ile Ser
   1655 1660 1665
   Thr Ser Leu Asp Phe Ser Tyr Glu Pro Leu Tyr Gly Ile Asp Arg
   1670 1675 1680
   Tyr Ile Asn Lys Val Leu Ile Ala Pro Asp Leu Tyr Thr Ser Leu
   1685 1690 1695
   Ile Asn Ile Asn Thr Asn Tyr Tyr Ser Asn Glu Tyr Tyr Pro Glu
   1700 1705 1710
   Ile Ile Val Leu Asn Pro Asn Thr Phe His Lys Lys Val Asn Ile
   1715 1720 1725
   Asn Leu Asp Ser Ser Ser Phe Glu Tyr Lys Trp Ser Thr Glu Gly
   1730 1735 1740
   Ser Asp Phe Ile Leu Val Arg Tyr Leu Glu Glu Ser Asn Lys Lys
   1745 1750 1755
   Ile Leu Gln Lys Ile Arg Ile Lys Gly Ile Leu Ser Asn Thr Gln
   1760 1765 1770
   Ser Phe Asn Lys Met Ser Ile Asp Phe Lys Asp Ile Lys Lys Leu
   1775 1780 1785
   Ser Leu Gly Tyr Ile Met Ser Asn Phe Lys Ser Phe Asn Ser Glu
   1790 1795 1800
   Asn Glu Leu Asp Arg Asp His Leu Gly Phe Lys Ile Ile Asp Asn
   1805 1810 1815
   Lys Thr Tyr Tyr Tyr Asp Glu Asp Ser Lys Leu Val Lys Gly Leu
   1820 1825 1830
   Ile Asn Ile Asn Asn Ser Leu Phe Tyr Phe Asp Pro Ile Glu Phe
   1835 1840 1845
   Asn Leu Val Thr Gly Trp Gln Thr Ile Asn Gly Lys Lys Tyr Tyr
   1850 1855 1860
   Phe Asp Ile Asn Thr Gly Ala Ala Leu Thr Ser Tyr Lys Ile Ile
   1865 1870 1875
   Asn Gly Lys His Phe Tyr Phe Asn Asn Asp Gly Val Met Gln Leu
   1880 1885 1890
   Gly Val Phe Lys Gly Pro Asp Gly Phe Glu Tyr Phe Ala Pro Ala
   1895 1900 1905
   Asn Thr Gln Asn Asn Asn Ile Glu Gly Gln Ala Ile Val Tyr Gln
   1910 1915 1920
   Ser Lys Phe Leu Thr Leu Asn Gly Lys Lys Tyr Tyr Phe Asp Asn
   1925 1930 1935
   Asn Ser Lys Ala Val Thr Gly Trp Arg Ile Ile Asn Asn Glu Lys
   1940 1945 1950
   Tyr Tyr Phe Asn Pro Asn Asn Ala Ile Ala Ala Val Gly Leu Gln
   1955 1960 1965
   Val Ile Asp Asn Asn Lys Tyr Tyr Phe Asn Pro Asp Thr Ala Ile
   1970 1975 1980
   Ile Ser Lys Gly Trp Gln Thr Val Asn Gly Ser Arg Tyr Tyr Phe
   1985 1990 1995
   Asp Thr Asp Thr Ala Ile Ala Phe Asn Gly Tyr Lys Thr Ile Asp
   2000 2005 2010
   Gly Lys His Phe Tyr Phe Asp Ser Asp Cys Val Val Lys Ile Gly
   2015 2020 2025
   Val Phe Ser Thr Ser Asn Gly Phe Glu Tyr Phe Ala Pro Ala Asn
   2030 2035 2040
   Thr Tyr Asn Asn Asn Ile Glu Gly Gln Ala Ile Val Tyr Gln Ser
   2045 2050 2055
   Lys Phe Leu Thr Leu Asn Gly Lys Lys Tyr Tyr Phe Asp Asn Asn
   2060 2065 2070
   Ser Lys Ala Val Thr Gly Leu Gln Thr Ile Asp Ser Lys Lys Tyr
   2075 2080 2085
   Tyr Phe Asn Thr Asn Thr Ala Glu Ala Ala Thr Gly Trp Gln Thr
   2090 2095 2100
   Ile Asp Gly Lys Lys Tyr Tyr Phe Asn Thr Asn Thr Ala Glu Ala
   2105 2110 2115
   Ala Thr Gly Trp Gln Thr Ile Asp Gly Lys Lys Tyr Tyr Phe Asn
   2120 2125 2130
   Thr Asn Thr Ala Ile Ala Ser Thr Gly Tyr Thr Ile Ile Asn Gly
   2135 2140 2145
   Lys His Phe Tyr Phe Asn Thr Asp Gly Ile Met Gln Ile Gly Val
   2150 2155 2160
   Phe Lys Gly Pro Asn Gly Phe Glu Tyr Phe Ala Pro Ala Asn Thr
   2165 2170 2175
   Asp Ala Asn Asn Ile Glu Gly Gln Ala Ile Leu Tyr Gln Asn Glu
   2180 2185 2190
   Phe Leu Thr Leu Asn Gly Lys Lys Tyr Tyr Phe Gly Ser Asp Ser
   2195 2200 2205
   Lys Ala Val Thr Gly Trp Arg Ile Ile Asn Asn Lys Lys Tyr Tyr
   2210 2215 2220
   Phe Asn Pro Asn Asn Ala Ile Ala Ala Ile His Leu Cys Thr Ile
   2225 2230 2235
   Asn Asn Asp Lys Tyr Tyr Phe Ser Tyr Asp Gly Ile Leu Gln Asn
   2240 2245 2250
   Gly Tyr Ile Thr Ile Glu Arg Asn Asn Phe Tyr Phe Asp Ala Asn
   2255 2260 2265
   Asn Glu Ser Lys Met Val Thr Gly Val Phe Lys Gly Pro Asn Gly
   2270 2275 2280
   Phe Glu Tyr Phe Ala Pro Ala Asn Thr His Asn Asn Asn Ile Glu
   2285 2290 2295
   Gly Gln Ala Ile Val Tyr Gln Asn Lys Phe Leu Thr Leu Asn Gly
   2300 2305 2310
   Lys Lys Tyr Tyr Phe Asp Asn Asp Ser Lys Ala Val Thr Gly Trp
   2315 2320 2325
   Gln Thr Ile Asp Gly Lys Lys Tyr Tyr Phe Asn Leu Asn Thr Ala
   2330 2335 2340
   Glu Ala Ala Thr Gly Trp Gln Thr Ile Asp Gly Lys Lys Tyr Tyr
   2345 2350 2355
   Phe Asn Leu Asn Thr Ala Glu Ala Ala Thr Gly Trp Gln Thr Ile
   2360 2365 2370
   Asp Gly Lys Lys Tyr Tyr Phe Asn Thr Asn Thr Phe Ile Ala Ser
   2375 2380 2385
   Thr Gly Tyr Thr Ser Ile Asn Gly Lys His Phe Tyr Phe Asn Thr
   2390 2395 2400
   Asp Gly Ile Met Gln Ile Gly Val Phe Lys Gly Pro Asn Gly Phe
   2405 2410 2415
   Glu Tyr Phe Ala Pro Ala Asn Thr Asp Ala Asn Asn Ile Glu Gly
   2420 2425 2430
   Gln Ala Ile Leu Tyr Gln Asn Lys Phe Leu Thr Leu Asn Gly Lys
   2435 2440 2445
   Lys Tyr Tyr Phe Gly Ser Asp Ser Lys Ala Val Thr Gly Leu Arg
   2450 2455 2460
   Thr Ile Asp Gly Lys Lys Tyr Tyr Phe Asn Thr Asn Thr Ala Val
   2465 2470 2475
   Ala Val Thr Gly Trp Gln Thr Ile Asn Gly Lys Lys Tyr Tyr Phe
   2480 2485 2490
   Asn Thr Asn Thr Ser Ile Ala Ser Thr Gly Tyr Thr Ile Ile Ser
   2495 2500 2505
   Gly Lys His Phe Tyr Phe Asn Thr Asp Gly Ile Met Gln Ile Gly
   2510 2515 2520
   Val Phe Lys Gly Pro Asp Gly Phe Glu Tyr Phe Ala Pro Ala Asn
   2525 2530 2535
   Thr Asp Ala Asn Asn Ile Glu Gly Gln Ala Ile Arg Tyr Gln Asn
   2540 2545 2550
   Arg Phe Leu Tyr Leu His Asp Asn Ile Tyr Tyr Phe Gly Asn Asn
   2555 2560 2565
   Ser Lys Ala Ala Thr Gly Trp Val Thr Ile Asp Gly Asn Arg Tyr
   2570 2575 2580
   Tyr Phe Glu Pro Asn Thr Ala Met Gly Ala Asn Gly Tyr Lys Thr
               2585                2590                2595
   Ile Asp  Asn Lys Asn Phe Tyr  Phe Arg Asn Gly Leu  Pro Gln Ile
       2600                2605                2610
   Gly Val  Phe Lys Gly Ser Asn  Gly Phe Glu Tyr Phe  Ala Pro Ala
       2615                2620                2625
   Asn Thr  Asp Ala Asn Asn Ile  Glu Gly Gln Ala Ile  Arg Tyr Gln
       2630                2635                2640
   Asn Arg  Phe Leu His Leu Leu  Gly Lys Ile Tyr Tyr  Phe Gly Asn
       2645                2650                2655
   Asn Ser  Lys Ala Val Thr Gly  Trp Gln Thr Ile Asn  Gly Lys Val
       2660                2665                2670
   Tyr Tyr  Phe Met Pro Asp Thr  Ala Met Ala Ala Ala  Gly Gly Leu
       2675                2680                2685
   Phe Glu  Ile Asp Gly Val Ile  Tyr Phe Phe Gly Val  Asp Gly Val
       2690                2695                2700
   Lys Ala  Pro Gly Ile Tyr Gly
       2705                2710
<210> 4
   <211> 3
   <212> PRT
   <213> Clostridium difficile
<300>
   <308> P18177
   <309> 1990-01-11
   <313> (1665)..(1667)
<400> 4
   Asp Ser Gly
   1
<210> 5
   <211> 26
   <212> PRT
   <213> Clostridium difficile
<300>
   <308> P18177
   <309> 1990-11-01
   <313> (1653)..(1678)
<400> 5
   Gln Asn Met Ile Val Glu Pro Asn Tyr Asp Leu Asp Asp Ser Gly Asp
   1               5                   10                  15
   Ile Ser Ser Thr Val Ile Asn Phe Ser Gln
                       20                  25
<210> 6
   <211> 301
   <212> PRT
   <213> Clostridium difficile
<300>
   <308> P18177
   <309> 1990-11-01
   <313> (1500)..(1800)
<400> 6
   Asp Asp Ser Lys Pro Ser Phe Gly Tyr Tyr Ser Asn Asn Leu Lys Asp
   1               5                   10                  15
   Val Lys Val Ile Thr Lys Asp Asn Val Asn Ile Leu Thr Gly Tyr Tyr
               20                  25                  30
   Leu Lys Asp Asp Ile Lys Ile Ser Leu Ser Leu Thr Leu Gln Asp Glu
           35                  40                  45
   Lys Thr Ile Lys Leu Asn Ser Val His Leu Asp Glu Ser Gly Val Ala
       50                  55                  60
   Glu Ile Leu Lys Phe Met Asn Arg Lys Gly Asn Thr Asn Thr Ser Asp
   65                  70                  75                  80
   Ser Leu Met Ser Phe Leu Glu Ser Met Asn Ile Lys Ser Ile Phe Val
                   85                  90                  95
   Asn Phe Leu Gln Ser Asn Ile Lys Phe Ile Leu Asp Ala Asn Phe Ile
               100                 105                 110
   Ile Ser Gly Thr Thr Ser Ile Gly Gln Phe Glu Phe Ile Cys Asp Glu
           115                 120                 125
   Asn Asp Asn Ile Gln Pro Tyr Phe Ile Lys Phe Asn Thr Leu Glu Thr
       130                 135                 140
   Asn Tyr Thr Leu Tyr Val Gly Asn Arg Gln Asn Met Ile Val Glu Pro
   145                 150                 155                 160
   Asn Tyr Asp Leu Asp Asp Ser Gly Asp Ile Ser Ser Thr Val Ile Asn
                   165                 170                 175
   Phe Ser Gln Lys Tyr Leu Tyr Gly Ile Asp Ser Cys Val Asn Lys Val
               180                 185                 190
   Val Ile Ser Pro Asn Ile Tyr Thr Asp Glu Ile Asn Ile Thr Pro Val
           195                 200                 205
   Tyr Glu Thr Asn Asn Thr Tyr Pro Glu Val Ile Val Leu Asp Ala Asn
   210 215 220
   Tyr Ile Asn Glu Lys Ile Asn Val Asn Ile Asn Asp Leu Ser Ile Arg
   225 230 235 240
   Tyr Val Trp Ser Asn Asp Gly Asn Asp Phe Ile Leu Met Ser Thr Ser
   245 250 255
   Glu Glu Asn Lys Val Ser Gln Val Lys Ile Arg Phe Val Asn Val Phe
   260 265 270
   Lys Asp Lys Thr Leu Ala Asn Lys Leu Ser Phe Asn Phe Ser Asp Lys
   275 280 285
   Gln Asp Val Pro Val Ser Glu Ile Ile Leu Ser Phe Thr
   290 295 300
<210> 7
   <211> 901
   <212> PRT
   <213> Clostridium difficile
<400> 7
   Gly Phe Val Ser Leu Thr Phe Ser Ile Leu Glu Gly Ile Asn Ala Ile
   1 5 10 15
   Ile Glu Val Asp Leu Leu Ser Lys Ser Tyr Lys Leu Leu Ile Ser Gly
   20 25 30
   Glu Leu Lys Ile Leu Met Leu Asn Ser Asn His Ile Gln Gln Lys Ile
   35 40 45
   Asp Tyr Ile Gly Phe Asn Ser Glu Leu Gln Lys Asn Ile Pro Tyr Ser
   50 55 60
   Phe Val Asp Ser Glu Gly Lys Glu Asn Gly Phe Ile Asn Gly Ser Thr
   65 70 75 80
   Lys Glu Gly Leu Phe Val Ser Glu Leu Pro Asp Val Val Leu Ile Ser
   85 90 95
   Lys Val Tyr Met Asp Asp Ser Lys Pro Ser Phe Gly Tyr Tyr Ser Asn
   100 105 110
   Asn Leu Lys Asp Val Lys Val Ile Thr Lys Asp Asn Val Asn Ile Leu
   115 120 125
   Thr Gly Tyr Tyr Leu Lys Asp Asp Ile Lys Ile Ser Leu Ser Leu Thr
   130 135 140
   Leu Gln Asp Glu Lys Thr Ile Lys Leu Asn Ser Val His Leu Asp Glu
   145 150 155 160
   Ser Gly Val Ala Glu Ile Leu Lys Phe Met Asn Arg Lys Gly Asn Thr
   165 170 175
   Asn Thr Ser Asp Ser Leu Met Ser Phe Leu Glu Ser Met Asn Ile Lys
   180 185 190
   Ser Ile Phe Val Asn Phe Leu Gln Ser Asn Ile Lys Phe Ile Leu Asp
   195 200 205
   Ala Asn Phe Ile Ile Ser Gly Thr Thr Ser Ile Gly Gln Phe Glu Phe
   210 215 220
   Ile Cys Asp Glu Asn Asp Asn Ile Gln Pro Tyr Phe Ile Lys Phe Asn
   225 230 235 240
   Thr Leu Glu Thr Asn Tyr Thr Leu Tyr Val Gly Asn Arg Gln Asn Met
   245 250 255
   Ile Val Glu Pro Asn Tyr Asp Leu Asp Asp Ser Gly Asp Ile Ser Ser
   260 265 270
   Thr Val Ile Asn Phe Ser Gln Lys Tyr Leu Tyr Gly Ile Asp Ser Cys
   275 280 285
   Val Asn Lys Val Val Ile Ser Pro Asn Ile Tyr Thr Asp Glu Ile Asn
   290 295 300
   Ile Thr Pro Val Tyr Glu Thr Asn Asn Thr Tyr Pro Glu Val Ile Val
   305 310 315 320
   Leu Asp Ala Asn Tyr Ile Asn Glu Lys Ile Asn Val Asn Ile Asn Asp
   325 330 335
   Leu Ser Ile Arg Tyr Val Trp Ser Asn Asp Gly Asn Asp Phe Ile Leu
   340 345 350
   Met Ser Thr Ser Glu Glu Asn Lys Val Ser Gln Val Lys Ile Arg Phe
   355 360 365
   Val Asn Val Phe Lys Asp Lys Thr Leu Ala Asn Lys Leu Ser Phe Asn
   370 375 380
   Phe Ser Asp Lys Gln Asp Val Pro Val Ser Glu Ile Ile Leu Ser Phe
   385 390 395 400
   Thr Pro Ser Tyr Tyr Glu Asp Gly Leu Ile Gly Tyr Asp Leu Gly Leu
   405 410 415
   Val Ser Leu Tyr Asn Glu Lys Phe Tyr Ile Asn Asn Phe Gly Met Met
   420 425 430
   Val Ser Gly Leu Ile Tyr Ile Asn Asp Ser Leu Tyr Tyr Phe Lys Pro
   435 440 445
   Pro Val Asn Asn Leu Ile Thr Gly Phe Val Thr Val Gly Asp Asp Lys
   450 455 460
   Tyr Tyr Phe Asn Pro Ile Asn Gly Gly Ala Ala Ser Ile Gly Glu Thr
   465 470 475 480
   Ile Ile Asp Asp Lys Asn Tyr Tyr Phe Asn Gln Ser Gly Val Leu Gln
   485 490 495
   Thr Gly Val Phe Ser Thr Glu Asp Gly Phe Lys Tyr Phe Ala Pro Ala
   500 505 510
   Asn Thr Leu Asp Glu Asn Leu Glu Gly Glu Ala Ile Asp Phe Thr Gly
   515 520 525
   Lys Leu Ile Ile Asp Glu Asn Ile Tyr Tyr Phe Asp Asp Asn Tyr Arg
   530 535 540
   Gly Ala Val Glu Trp Lys Glu Leu Asp Gly Glu Met His Tyr Phe Ser
   545 550 555 560
   Pro Glu Thr Gly Lys Ala Phe Lys Gly Leu Asn Gln Ile Gly Asp Tyr
   565 570 575
   Lys Tyr Tyr Phe Asn Ser Asp Gly Val Met Gln Lys Gly Phe Val Ser
   580 585 590
   Ile Asn Asp Asn Lys His Tyr Phe Asp Asp Ser Gly Val Met Lys Val
   595 600 605
   Gly Tyr Thr Glu Ile Asp Gly Lys His Phe Tyr Phe Ala Glu Asn Gly
   610 615 620
   Glu Met Gln Ile Gly Val Phe Asn Thr Glu Asp Gly Phe Lys Tyr Phe
   625 630 635 640
   Ala His His Asn Glu Asp Leu Gly Asn Glu Glu Gly Glu Glu Ile Ser
   645 650 655
   Tyr Ser Gly Ile Leu Asn Phe Asn Asn Lys Ile Tyr Tyr Phe Asp Asp
   660 665 670
   Ser Phe Thr Ala Val Val Gly Trp Lys Asp Leu Glu Asp Gly Ser Lys
   675 680 685
   Tyr Tyr Phe Asp Glu Asp Thr Ala Glu Ala Tyr Ile Gly Leu Ser Leu
   690 695 700
   Ile Asn Asp Gly Gln Tyr Tyr Phe Asn Asp Asp Gly Ile Met Gln Val
   705 710 715 720
   Gly Phe Val Thr Ile Asn Asp Lys Val Phe Tyr Phe Ser Asp Ser Gly
   725 730 735
   Ile Ile Glu Ser Gly Val Gln Asn Ile Asp Asp Asn Tyr Phe Tyr Ile
   740 745 750
   Asp Asp Asn Gly Ile Val Gln Ile Gly Val Phe Asp Thr Ser Asp Gly
   755 760 765
   Tyr Lys Tyr Phe Ala Pro Ala Asn Thr Val Asn Asp Asn Ile Tyr Gly
   770 775 780
   Gln Ala Val Glu Tyr Ser Gly Leu Val Arg Val Gly Glu Asp Val Tyr
   785 790 795 800
   Tyr Phe Gly Glu Thr Tyr Thr Ile Glu Thr Gly Trp Ile Tyr Asp Met
   805 810 815
   Glu Asn Glu Ser Asp Lys Tyr Tyr Phe Asn Pro Glu Thr Lys Lys Ala
   820 825 830
   Cys Lys Gly Ile Asn Leu Ile Asp Asp Ile Lys Tyr Tyr Phe Asp Glu
   835 840 845
   Lys Gly Ile Met Arg Thr Gly Leu Ile Ser Phe Glu Asn Asn Asn Tyr
   850 855 860
   Tyr Phe Asn Glu Asn Gly Glu Met Gln Phe Gly Tyr Ile Asn Ile Glu
   865 870 875 880
   Asp Lys Met Phe Tyr Phe Gly Glu Asp Gly Val Met Gln Ile Gly Val
   885 890 895
   Phe Asn Thr Pro Asp
   900
<210> 8
   <211> 626
   <212> PRT
   <213> Clostridium difficile
<400> 8
   Lys Val Ile Thr Lys Asp Asn Val Asn Ile Leu Thr Gly Tyr Tyr Leu
   1 5 10 15
   Lys Asp Asp Ile Lys Ile Ser Leu Ser Leu Thr Leu Gln Asp Glu Lys
   20 25 30
   Thr Ile Lys Leu Asn Ser Val His Leu Asp Glu Ser Gly Val Ala Glu
   35 40 45
   Ile Leu Lys Phe Met Asn Arg Lys Gly Asn Thr Asn Thr Ser Asp Ser
   50 55 60
   Leu Met Ser Phe Leu Glu Ser Met Asn Ile Lys Ser Ile Phe Val Asn
   65 70 75 80
   Phe Leu Gln Ser Asn Ile Lys Phe Ile Leu Asp Ala Asn Phe Ile Ile
   85 90 95
   Ser Gly Thr Thr Ser Ile Gly Gln Phe Glu Phe Ile Cys Asp Glu Asn
   100 105 110
   Asp Asn Ile Gln Pro Tyr Phe Ile Lys Phe Asn Thr Leu Glu Thr Asn
   115 120 125
   Tyr Thr Leu Tyr Val Gly Asn Arg Gln Asn Met Ile Val Glu Pro Asn
   130 135 140
   Tyr Asp Leu Asp Asp Ser Gly Asp Ile Ser Ser Thr Val Ile Asn Phe
   145 150 155 160
   Ser Gln Lys Tyr Leu Tyr Gly Ile Asp Ser Cys Val Asn Lys Val Val
   165 170 175
   Ile Ser Pro Asn Ile Tyr Thr Asp Glu Ile Asn Ile Thr Pro Val Tyr
   180 185 190
   Glu Thr Asn Asn Thr Tyr Pro Glu Val Ile Val Leu Asp Ala Asn Tyr
   195 200 205
   Ile Asn Glu Lys Ile Asn Val Asn Ile Asn Asp Leu Ser Ile Arg Tyr
   210 215 220
   Val Trp Ser Asn Asp Gly Asn Asp Phe Ile Leu Met Ser Thr Ser Glu
   225 230 235 240
   Glu Asn Lys Val Ser Gln Val Lys Ile Arg Phe Val Asn Val Phe Lys
   245 250 255
   Asp Lys Thr Leu Ala Asn Lys Leu Ser Phe Asn Phe Ser Asp Lys Gln
   260 265 270
   Asp Val Pro Val Ser Glu Ile Ile Leu Ser Phe Thr Pro Ser Tyr Tyr
   275 280 285
   Glu Asp Gly Leu Ile Gly Tyr Asp Leu Gly Leu Val Ser Leu Tyr Asn
   290 295 300
   Glu Lys Phe Tyr Ile Asn Asn Phe Gly Met Met Val Ser Gly Leu Ile
   305 310 315 320
   Tyr Ile Asn Asp Ser Leu Tyr Tyr Phe Lys Pro Pro Val Asn Asn Leu
   325 330 335
   Ile Thr Gly Phe Val Thr Val Gly Asp Asp Lys Tyr Tyr Phe Asn Pro
   340 345 350
   Ile Asn Gly Gly Ala Ala Ser Ile Gly Glu Thr Ile Ile Asp Asp Lys
   355 360 365
   Asn Tyr Tyr Phe Asn Gln Ser Gly Val Leu Gln Thr Gly Val Phe Ser
   370 375 380
   Thr Glu Asp Gly Phe Lys Tyr Phe Ala Pro Ala Asn Thr Leu Asp Glu
   385 390 395 400
   Asn Leu Glu Gly Glu Ala Ile Asp Phe Thr Gly Lys Leu Ile Ile Asp
   405 410 415
   Glu Asn Ile Tyr Tyr Phe Asp Asp Asn Tyr Arg Gly Ala Val Glu Trp
   420 425 430
   Lys Glu Leu Asp Gly Glu Met His Tyr Phe Ser Pro Glu Thr Gly Lys
   435 440 445
   Ala Phe Lys Gly Leu Asn Gln Ile Gly Asp Tyr Lys Tyr Tyr Phe Asn
   450 455 460
   Ser Asp Gly Val Met Gln Lys Gly Phe Val Ser Ile Asn Asp Asn Lys
   465 470 475 480
   His Tyr Phe Asp Asp Ser Gly Val Met Lys Val Gly Tyr Thr Glu Ile
   485 490 495
   Asp Gly Lys His Phe Tyr Phe Ala Glu Asn Gly Glu Met Gln Ile Gly
   500 505 510
   Val Phe Asn Thr Glu Asp Gly Phe Lys Tyr Phe Ala His His Asn Glu
   515 520 525
   Asp Leu Gly Asn Glu Glu Gly Glu Glu Ile Ser Tyr Ser Gly Ile Leu
   530 535 540
   Asn Phe Asn Asn Lys Ile Tyr Tyr Phe Asp Asp Ser Phe Thr Ala Val
   545 550 555 560
   Val Gly Trp Lys Asp Leu Glu Asp Gly Ser Lys Tyr Tyr Phe Asp Glu
   565 570 575
   Asp Thr Ala Glu Ala Tyr Ile Gly Leu Ser Leu Ile Asn Asp Gly Gln
               580                 585                 590
   Tyr Tyr Phe Asn Asp Asp Gly Ile Met Gln Val Gly Phe Val Thr Ile
           595                 600                 605
   Asn Asp Lys Val Phe Tyr Phe Ser Asp Ser Gly Ile Ile Glu Ser Gly
       610                 615                 620
   Val Gln
   625
<210> 9
   <211> 77
   <212> PRT
   <213> Clostridium difficile
<400> 9
   Lys Val Ile Thr Lys Asp Asn Val Asn Ile Leu Thr Gly Tyr Tyr Leu
   1               5                   10                  15
   Lys Asp Asp Ile Lys Ile Ser Leu Ser Leu Thr Leu Gln Asp Glu Lys
               20                  25                  30
   Thr Ile Lys Leu Asn Ser Val His Leu Asp Glu Ser Gly Val Ala Glu
           35                  40                  45
   Ile Leu Lys Phe Met Asn Arg Lys Gly Asn Thr Asn Thr Ser Asp Ser
       50                  55                  60
   Leu Met Ser Phe Leu Glu Ser Met Asn Ile Lys Ser Ile
   65                  70                  75
<210> 10
   <211> 225
   <212> PRT
   <213> Clostridium difficile
<400> 10
   Leu Glu Gly Glu Ala Ile Asp Phe Thr Gly Lys Leu Ile Ile Asp Glu
   1               5                   10                  15
   Asn Ile Tyr Tyr Phe Asp Asp Asn Tyr Arg Gly Ala Val Glu Trp Lys
               20                  25                  30
   Glu Leu Asp Gly Glu Met His Tyr Phe Ser Pro Glu Thr Gly Lys Ala
           35                  40                  45
   Phe Lys Gly Leu Asn Gln Ile Gly Asp Tyr Lys Tyr Tyr Phe Asn Ser
       50                  55                  60
   Asp Gly Val Met Gln Lys Gly Phe Val Ser Ile Asn Asp Asn Lys His
   65                  70                  75                  80
   Tyr Phe Asp Asp Ser Gly Val Met Lys Val Gly Tyr Thr Glu Ile Asp
                   85                  90                  95
   Gly Lys His Phe Tyr Phe Ala Glu Asn Gly Glu Met Gln Ile Gly Val
               100                 105                 110
   Phe Asn Thr Glu Asp Gly Phe Lys Tyr Phe Ala His His Asn Glu Asp
           115                 120                 125
   Leu Gly Asn Glu Glu Gly Glu Glu Ile Ser Tyr Ser Gly Ile Leu Asn
       130                 135                 140
   Phe Asn Asn Lys Ile Tyr Tyr Phe Asp Asp Ser Phe Thr Ala Val Val
   145                 150                 155                 160
   Gly Trp Lys Asp Leu Glu Asp Gly Ser Lys Tyr Tyr Phe Asp Glu Asp
                   165                 170                 175
   Thr Ala Glu Ala Tyr Ile Gly Leu Ser Leu Ile Asn Asp Gly Gln Tyr
               180                 185                 190
   Tyr Phe Asn Asp Asp Gly Ile Met Gln Val Gly Phe Val Thr Ile Asn
           195                 200                 205
   Asp Lys Val Phe Tyr Phe Ser Asp Ser Gly Ile Ile Glu Ser Gly Val
       210                 215                 220
   Gln
   225
<210> 11
   <211> 116
   <212> PRT
   <213> Clostridium difficile
<400> 11
   Asn Ile Lys Phe Ile Leu Asp Ala Asn Phe Ile Ile Ser Gly Thr Thr
   1               5                   10                  15
   Ser Ile Gly Gln Phe Glu Phe Ile Cys Asp Glu Asn Asp Asn Ile Gln
               20                  25                  30
   Pro Tyr Phe Ile Lys Phe Asn Thr Leu Glu Thr Asn Tyr Thr Leu Tyr
           35                  40                  45
   Val Gly Asn Arg Gln Asn Met Ile Val Glu Pro Asn Tyr Asp Leu Asp
       50                  55                  60
   Asp Ser Gly Asp Ile Ser Ser Thr Val Ile Asn Phe Ser Gln Lys Tyr
   65                  70                  75                  80
   Leu Tyr Gly Ile Asp Ser Cys Val Asn Lys Val Val Ile Ser Pro Asn
                   85                  90                  95
   Ile Tyr Thr Asp Glu Ile Asn Ile Thr Pro Val Tyr Glu Thr Asn Asn
               100                 105                 110
   Thr Tyr Pro Glu
           115
<210> 12
   <211> 94
   <212> PRT
   <213> Clostridium difficile
<400> 12
   Tyr Tyr Ser Asn Asn Leu Lys Asp Val Lys Val Ile Thr Lys Asp Asn
   1               5                   10                  15
   Val Asn Ile Leu Thr Gly Tyr Tyr Leu Lys Asp Asp Ile Lys Ile Ser
               20                  25                  30
   Leu Ser Leu Thr Leu Gln Asp Glu Lys Thr Ile Lys Leu Asn Ser Val
           35                  40                  45
   His Leu Asp Glu Ser Gly Val Ala Glu Ile Leu Lys Phe Met Asn Arg
       50                  55                  60
   Lys Gly Asn Thr Asn Thr Ser Asp Ser Leu Met Ser Phe Leu Glu Ser
   65                  70                  75                  80
   Met Asn Ile Lys Ser Ile Phe Val Asn Phe Leu Gln Ser Asn
                   85                  90
<210> 13
   <211> 650
   <212> PRT
   <213> Clostridium difficile
<400> 13
   Tyr Tyr Ser Asn Asn Leu Lys Asp Val Lys Val Ile Thr Lys Asp Asn
   1               5                   10                  15
   Val Asn Ile Leu Thr Gly Tyr Tyr Leu Lys Asp Asp Ile Lys Ile Ser
               20                  25                  30
   Leu Ser Leu Thr Leu Gln Asp Glu Lys Thr Ile Lys Leu Asn Ser Val
           35                  40                  45
   His Leu Asp Glu Ser Gly Val Ala Glu Ile Leu Lys Phe Met Asn Arg
       50                  55                  60
   Lys Gly Asn Thr Asn Thr Ser Asp Ser Leu Met Ser Phe Leu Glu Ser
   65                  70                  75                  80
   Met Asn Ile Lys Ser Ile Phe Val Asn Phe Leu Gln Ser Asn Ile Lys
                   85                  90                  95
   Phe Ile Leu Asp Ala Asn Phe Ile Ile Ser Gly Thr Thr Ser Ile Gly
               100                 105                 110
   Gln Phe Glu Phe Ile Cys Asp Glu Asn Asp Asn Ile Gln Pro Tyr Phe
           115                 120                 125
   Ile Lys Phe Asn Thr Leu Glu Thr Asn Tyr Thr Leu Tyr Val Gly Asn
       130                 135                 140
   Arg Gln Asn Met Ile Val Glu Pro Asn Tyr Asp Leu Asp Asp Ser Gly
   145                 150                 155                 160
   Asp Ile Ser Ser Thr Val Ile Asn Phe Ser Gln Lys Tyr Leu Tyr Gly
                   165                 170                 175
   Ile Asp Ser Cys Val Asn Lys Val Val Ile Ser Pro Asn Ile Tyr Thr
               180                 185                 190
   Asp Glu Ile Asn Ile Thr Pro Val Tyr Glu Thr Asn Asn Thr Tyr Pro
           195                 200                 205
   Glu Val Ile Val Leu Asp Ala Asn Tyr Ile Asn Glu Lys Ile Asn Val
       210                 215                 220
   Asn Ile Asn Asp Leu Ser Ile Arg Tyr Val Trp Ser Asn Asp Gly Asn
   225                 230                 235                 240
   Asp Phe Ile Leu Met Ser Thr Ser Glu Glu Asn Lys Val Ser Gln Val
                   245                 250                 255
   Lys Ile Arg Phe Val Asn Val Phe Lys Asp Lys Thr Leu Ala Asn Lys
               260                 265                 270
   Leu Ser Phe Asn Phe Ser Asp Lys Gln Asp Val Pro Val Ser Glu Ile
           275                 280                 285
   Ile Leu Ser Phe Thr Pro Ser Tyr Tyr Glu Asp Gly Leu Ile Gly Tyr
       290                 295                 300
   Asp Leu Gly Leu Val Ser Leu Tyr Asn Glu Lys Phe Tyr Ile Asn Asn
   305                 310                 315                 320
   Phe Gly Met Met Val Ser Gly Leu Ile Tyr Ile Asn Asp Ser Leu Tyr
                   325                 330                 335
   Tyr Phe Lys Pro Pro Val Asn Asn Leu Ile Thr Gly Phe Val Thr Val
   340 345 350
   Gly Asp Asp Lys Tyr Tyr Phe Asn Pro Ile Asn Gly Gly Ala Ala Ser
   355 360 365
   Ile Gly Glu Thr Ile Ile Asp Asp Lys Asn Tyr Tyr Phe Asn Gln Ser
   370 375 380
   Gly Val Leu Gln Thr Gly Val Phe Ser Thr Glu Asp Gly Phe Lys Tyr
   385 390 395 400
   Phe Ala Pro Ala Asn Thr Leu Asp Glu Asn Leu Glu Gly Glu Ala Ile
   405 410 415
   Asp Phe Thr Gly Lys Leu Ile Ile Asp Glu Asn Ile Tyr Tyr Phe Asp
   420 425 430
   Asp Asn Tyr Arg Gly Ala Val Glu Trp Lys Glu Leu Asp Gly Glu Met
   435 440 445
   His Tyr Phe Ser Pro Glu Thr Gly Lys Ala Phe Lys Gly Leu Asn Gln
   450 455 460
   Ile Gly Asp Tyr Lys Tyr Tyr Phe Asn Ser Asp Gly Val Met Gln Lys
   465 470 475 480
   Gly Phe Val Ser Ile Asn Asp Asn Lys His Tyr Phe Asp Asp Ser Gly
   485 490 495
   Val Met Lys Val Gly Tyr Thr Glu Ile Asp Gly Lys His Phe Tyr Phe
   500 505 510
   Ala Glu Asn Gly Glu Met Gln Ile Gly Val Phe Asn Thr Glu Asp Gly
   515 520 525
   Phe Lys Tyr Phe Ala His His Asn Glu Asp Leu Gly Asn Glu Glu Gly
   530 535 540
   Glu Glu Ile Ser Tyr Ser Gly Ile Leu Asn Phe Asn Asn Lys Ile Tyr
   545 550 555 560
   Tyr Phe Asp Asp Ser Phe Thr Ala Val Val Gly Trp Lys Asp Leu Glu
   565 570 575
   Asp Gly Ser Lys Tyr Tyr Phe Asp Glu Asp Thr Ala Glu Ala Tyr Ile
   580 585 590
   Gly Leu Ser Leu Ile Asn Asp Gly Gln Tyr Tyr Phe Asn Asp Asp Gly
   595 600 605
   Ile Met Gln Val Gly Phe Val Thr Ile Asn Asp Lys Val Phe Tyr Phe
       610                 615                 620
   Ser Asp Ser Gly Ile Ile Glu Ser Gly Val Gln Asn Ile Asp Asp Asn
   625                 630                 635                     640
   Tyr Phe Tyr Ile Asp Asp Asn Gly Ile Val
                   645                 650
<210> 14
   <211> 2366
   <212> PRT
   <213> Clostridium difficile
<220>
   <221> MISC_FEATURE
   <222> (1665)..(1667)
   <223> DXG motive
<300>
   <308> P18177
   <309> 1990-11-01
   <313> (1)..(2366)
<400> 14
   Met Ser Leu Val Asn Arg Lys Gln Leu Glu Lys Met Ala Asn Val Arg
   1               5                   10                  15
   Phe Arg Thr Gln Glu Asp Glu Tyr Val Ala Ile Leu Asp Ala Leu Glu
               20                  25                  30
   Glu Tyr His Asn Met Ser Glu Asn Thr Val Val Glu Lys Tyr Leu Lys
           35                  40                  45
   Leu Lys Asp Ile Asn Ser Leu Thr Asp Ile Tyr Ile Asp Thr Tyr Lys
       50                  55                  60
   Lys Ser Gly Arg Asn Lys Ala Leu Lys Lys Phe Lys Glu Tyr Leu Val
   65                  70                  75                  80
   Thr Glu Val Leu Glu Leu Lys Asn Asn Asn Leu Thr Pro Val Glu Lys
                   85                  90                  95
   Asn Leu His Phe Val Trp Ile Gly Gly Gln Ile Asn Asp Thr Ala Ile
               100                 105                 110
   Asn Tyr Ile Asn Gln Trp Lys Asp Val Asn Ser Asp Tyr Asn Val Asn
           115                 120                 125
   Val Phe Tyr Asp Ser Asn Ala Phe Leu Ile Asn Thr Leu Lys Lys Thr
       130                 135                 140
   Val Val Glu Ser Ala Ile Asn Asp Thr Leu Glu Ser Phe Arg Glu Asn
   145                 150                 155                 160
   Leu Asn Asp Pro Arg Phe Asp Tyr Asn Lys Phe Phe Arg Lys Arg Met
   165 170 175
   Glu Ile Ile Tyr Asp Lys Gln Lys Asn Phe Ile Asn Tyr Tyr Lys Ala
   180 185 190
   Gln Arg Glu Glu Asn Pro Glu Leu Ile Ile Asp Asp Ile Val Lys Thr
   195 200 205
   Tyr Leu Ser Asn Glu Tyr Ser Lys Glu Ile Asp Glu Leu Asn Thr Tyr
   210 215 220
   Ile Glu Glu Ser Leu Asn Lys Ile Thr Gln Asn Ser Gly Asn Asp Val
   225 230 235 240
   Arg Asn Phe Glu Glu Phe Lys Asn Gly Glu Ser Phe Asn Leu Tyr Glu
   245 250 255
   Gln Glu Leu Val Glu Arg Trp Asn Leu Ala Ala Ala Ser Asp Ile Leu
   260 265 270
   Arg Ile Ser Ala Leu Lys Glu Ile Gly Gly Met Tyr Leu Asp Val Asp
   275 280 285
   Met Leu Pro Gly Ile Gln Pro Asp Leu Phe Glu Ser Ile Glu Lys Pro
   290 295 300
   Ser Ser Val Thr Val Asp Phe Trp Glu Met Thr Lys Leu Glu Ala Ile
   305 310 315 320
   Met Lys Tyr Lys Glu Tyr Ile Pro Glu Tyr Thr Ser Glu His Phe Asp
   325 330 335
   Met Leu Asp Glu Glu Val Gln Ser Ser Phe Glu Ser Val Leu Ala Ser
   340 345 350
   Lys Ser Asp Lys Ser Glu Ile Phe Ser Ser Leu Gly Asp Met Glu Ala
   355 360 365
   Ser Pro Leu Glu Val Lys Ile Ala Phe Asn Ser Lys Gly Ile Ile Asn
   370 375 380
   Gln Gly Leu Ile Ser Val Lys Asp Ser Tyr Cys Ser Asn Leu Ile Val
   385 390 395 400
   Lys Gln Ile Glu Asn Arg Tyr Lys Ile Leu Asn Asn Ser Leu Asn Pro
   405 410 415
   Ala Ile Ser Glu Asp Asn Asp Phe Asn Thr Thr Thr Asn Thr Phe Ile
   420 425 430
   Asp Ser Ile Met Ala Glu Ala Asn Ala Asp Asn Gly Arg Phe Met Met
   435 440 445
   Glu Leu Gly Lys Tyr Leu Arg Val Gly Phe Phe Pro Asp Val Lys Thr
   450 455 460
   Thr Ile Asn Leu Ser Gly Pro Glu Ala Tyr Ala Ala Ala Tyr Gln Asp
   465 470 475 480
   Leu Leu Met Phe Lys Glu Gly Ser Met Asn Ile His Leu Ile Glu Ala
   485 490 495
   Asp Leu Arg Asn Phe Glu Ile Ser Lys Thr Asn Ile Ser Gln Ser Thr
   500 505 510
   Glu Gln Glu Met Ala Ser Leu Trp Ser Phe Asp Asp Ala Arg Ala Lys
   515 520 525
   Ala Gln Phe Glu Glu Tyr Lys Arg Asn Tyr Phe Glu Gly Ser Leu Gly
   530 535 540
   Glu Asp Asp Asn Leu Asp Phe Ser Gln Asn Ile Val Val Asp Lys Glu
   545 550 555 560
   Tyr Leu Leu Glu Lys Ile Ser Ser Leu Ala Arg Ser Ser Glu Arg Gly
   565 570 575
   Tyr Ile His Tyr Ile Val Gln Leu Gln Gly Asp Lys Ile Ser Tyr Glu
   580 585 590
   Ala Ala Cys Asn Leu Phe Ala Lys Thr Pro Tyr Asp Ser Val Leu Phe
   595 600 605
   Gln Lys Asn Ile Glu Asp Ser Glu Ile Ala Tyr Tyr Tyr Asn Pro Gly
   610 615 620
   Asp Gly Glu Ile Gln Glu Ile Asp Lys Tyr Lys Ile Pro Ser Ile Ile
   625 630 635 640
   Ser Asp Arg Pro Lys Ile Lys Leu Thr Phe Ile Gly His Gly Lys Asp
   645 650 655
   Glu Phe Asn Thr Asp Ile Phe Ala Gly Phe Asp Val Asp Ser Leu Ser
   660 665 670
   Thr Glu Ile Glu Ala Ala Ile Asp Leu Ala Lys Glu Asp Ile Ser Pro
   675 680 685
   Lys Ser Ile Glu Ile Asn Leu Leu Gly Cys Asn Met Phe Ser Tyr Ser
   690 695 700
   Ile Asn Val Glu Glu Thr Tyr Pro Gly Lys Leu Leu Leu Lys Val Lys
   705 710 715 720
   Asp Lys Ile Ser Glu Leu Met Pro Ser Ile Ser Gln Asp Ser Ile Ile
   725 730 735
   Val Ser Ala Asn Gln Tyr Glu Val Arg Ile Asn Ser Glu Gly Arg Arg
   740 745 750
   Glu Leu Leu Asp His Ser Gly Glu Trp Ile Asn Lys Glu Glu Ser Ile
   755 760 765
   Ile Lys Asp Ile Ser Ser Lys Glu Tyr Ile Ser Phe Asn Pro Lys Glu
   770 775 780
   Asn Lys Ile Thr Val Lys Ser Lys Asn Leu Pro Glu Leu Ser Thr Leu
   785 790 795 800
   Leu Gln Glu Ile Arg Asn Asn Ser Asn Ser Ser Asp Ile Glu Leu Glu
   805 810 815
   Glu Lys Val Met Leu Thr Glu Cys Glu Ile Asn Val Ile Ser Asn Ile
   820 825 830
   Asp Thr Gln Ile Val Glu Glu Arg Ile Glu Glu Ala Lys Asn Leu Thr
   835 840 845
   Ser Asp Ser Ile Asn Tyr Ile Lys Asp Glu Phe Lys Leu Ile Glu Ser
   850 855 860
   Ile Ser Asp Ala Leu Cys Asp Leu Lys Gln Gln Asn Glu Leu Glu Asp
   865 870 875 880
   Ser His Phe Ile Ser Phe Glu Asp Ile Ser Glu Thr Asp Glu Gly Phe
   885 890 895
   Ser Ile Arg Phe Ile Asn Lys Glu Thr Gly Glu Ser Ile Phe Val Glu
   900 905 910
   Thr Glu Lys Thr Ile Phe Ser Glu Tyr Ala Asn His Ile Thr Glu Glu
   915 920 925
   Ile Ser Lys Ile Lys Gly Thr Ile Phe Asp Thr Val Asn Gly Lys Leu
   930 935 940
   Val Lys Lys Val Asn Leu Asp Thr Thr His Glu Val Asn Thr Leu Asn
   945 950 955 960
   Ala Ala Phe Phe Ile Gln Ser Leu Ile Glu Tyr Asn Ser Ser Lys Glu
   965 970 975
   Ser Leu Ser Asn Leu Ser Val Ala Met Lys Val Gln Val Tyr Ala Gln
   980 985 990
   Leu Phe Ser Thr Gly Leu Asn Thr Ile Thr Asp Ala Ala Lys Val Val
   995 1000 1005
   Glu Leu Val Ser Thr Ala Leu Asp Glu Thr Ile Asp Leu Leu Pro
   1010 1015 1020
   Thr Leu Ser Glu Gly Leu Pro Ile Ile Ala Thr Ile Ile Asp Gly
   1025 1030 1035
   Val Ser Leu Gly Ala Ala Ile Lys Glu Leu Ser Glu Thr Ser Asp
   1040 1045 1050
   Pro Leu Leu Arg Gln Glu Ile Glu Ala Lys Ile Gly Ile Met Ala
   1055 1060 1065
   Val Asn Leu Thr Thr Ala Thr Thr Ala Ile Ile Thr Ser Ser Leu
   1070 1075 1080
   Gly Ile Ala Ser Gly Phe Ser Ile Leu Leu Val Pro Leu Ala Gly
   1085 1090 1095
   Ile Ser Ala Gly Ile Pro Ser Leu Val Asn Asn Glu Leu Val Leu
   1100 1105 1110
   Arg Asp Lys Ala Thr Lys Val Val Asp Tyr Phe Lys His Val Ser
   1115 1120 1125
   Leu Val Glu Thr Glu Gly Val Phe Thr Leu Leu Asp Asp Lys Ile
   1130 1135 1140
   Met Met Pro Gln Asp Asp Leu Val Ile Ser Glu Ile Asp Phe Asn
   1145 1150 1155
   Asn Asn Ser Ile Val Leu Gly Lys Cys Glu Ile Trp Arg Met Glu
   1160 1165 1170
   Gly Gly Ser Gly His Thr Val Thr Asp Asp Ile Asp His Phe Phe
   1175 1180 1185
   Ser Ala Pro Ser Ile Thr Tyr Arg Glu Pro His Leu Ser Ile Tyr
   1190 1195 1200
   Asp Val Leu Glu Val Gln Lys Glu Glu Leu Asp Leu Ser Lys Asp
   1205 1210 1215
   Leu Met Val Leu Pro Asn Ala Pro Asn Arg Val Phe Ala Trp Glu
   1220 1225 1230
   Thr Gly Trp Thr Pro Gly Leu Arg Ser Leu Glu Asn Asp Gly Thr
   1235 1240 1245
   Lys Leu Leu Asp Arg Ile Arg Asp Asn Tyr Glu Gly Glu Phe Tyr
   1250 1255 1260
   Trp Arg Tyr Phe Ala Phe Ile Ala Asp Ala Leu Ile Thr Thr Leu
   1265 1270 1275
   Lys Pro Arg Tyr Glu Asp Thr Asn Ile Arg Ile Asn Leu Asp Ser
   1280 1285 1290
   Asn Thr Arg Ser Phe Ile Val Pro Ile Ile Thr Thr Glu Tyr Ile
   1295 1300 1305
   Arg Glu Lys Leu Ser Tyr Ser Phe Tyr Gly Ser Gly Gly Thr Tyr
   1310 1315 1320
   Ala Leu Ser Leu Ser Gln Tyr Asn Met Gly Ile Asn Ile Glu Leu
   1325 1330 1335
   Ser Glu Ser Asp Val Trp Ile Ile Asp Val Asp Asn Val Val Arg
   1340 1345 1350
   Asp Val Thr Ile Glu Ser Asp Lys Ile Lys Lys Gly Asp Leu Ile
   1355 1360 1365
   Glu Gly Ile Leu Ser Thr Leu Ser Ile Glu Glu Asn Lys Ile Ile
   1370 1375 1380
   Leu Asn Ser His Glu Ile Asn Phe Ser Gly Glu Val Asn Gly Ser
   1385 1390 1395
   Asn Gly Phe Val Ser Leu Thr Phe Ser Ile Leu Glu Gly Ile Asn
   1400 1405 1410
   Ala Ile Ile Glu Val Asp Leu Leu Ser Lys Ser Tyr Lys Leu Leu
   1415 1420 1425
   Ile Ser Gly Glu Leu Lys Ile Leu Met Leu Asn Ser Asn His Ile
   1430 1435 1440
   Gln Gln Lys Ile Asp Tyr Ile Gly Phe Asn Ser Glu Leu Gln Lys
   1445 1450 1455
   Asn Ile Pro Tyr Ser Phe Val Asp Ser Glu Gly Lys Glu Asn Gly
   1460 1465 1470
   Phe Ile Asn Gly Ser Thr Lys Glu Gly Leu Phe Val Ser Glu Leu
   1475 1480 1485
   Pro Asp Val Val Leu Ile Ser Lys Val Tyr Met Asp Asp Ser Lys
   1490 1495 1500
   Pro Ser Phe Gly Tyr Tyr Ser Asn Asn Leu Lys Asp Val Lys Val
   1505 1510 1515
   Ile Thr Lys Asp Asn Val Asn Ile Leu Thr Gly Tyr Tyr Leu Lys
   1520 1525 1530
   Asp Asp Ile Lys Ile Ser Leu Ser Leu Thr Leu Gln Asp Glu Lys
   1535 1540 1545
   Thr Ile Lys Leu Asn Ser Val His Leu Asp Glu Ser Gly Val Ala
   1550 1555 1560
   Glu Ile Leu Lys Phe Met Asn Arg Lys Gly Asn Thr Asn Thr Ser
   1565 1570 1575
   Asp Ser Leu Met Ser Phe Leu Glu Ser Met Asn Ile Lys Ser Ile
   1580 1585 1590
   Phe Val Asn Phe Leu Gln Ser Asn Ile Lys Phe Ile Leu Asp Ala
   1595 1600 1605
   Asn Phe Ile Ile Ser Gly Thr Thr Ser Ile Gly Gln Phe Glu Phe
   1610 1615 1620
   Ile Cys Asp Glu Asn Asp Asn Ile Gln Pro Tyr Phe Ile Lys Phe
   1625 1630 1635
   Asn Thr Leu Glu Thr Asn Tyr Thr Leu Tyr Val Gly Asn Arg Gln
   1640 1645 1650
   Asn Met Ile Val Glu Pro Asn Tyr Asp Leu Asp Asp Ser Gly Asp
   1655 1660 1665
   Ile Ser Ser Thr Val Ile Asn Phe Ser Gln Lys Tyr Leu Tyr Gly
   1670 1675 1680
   Ile Asp Ser Cys Val Asn Lys Val Val Ile Ser Pro Asn Ile Tyr
   1685 1690 1695
   Thr Asp Glu Ile Asn Ile Thr Pro Val Tyr Glu Thr Asn Asn Thr
   1700 1705 1710
   Tyr Pro Glu Val Ile Val Leu Asp Ala Asn Tyr Ile Asn Glu Lys
   1715 1720 1725
   Ile Asn Val Asn Ile Asn Asp Leu Ser Ile Arg Tyr Val Trp Ser
   1730 1735 1740
   Asn Asp Gly Asn Asp Phe Ile Leu Met Ser Thr Ser Glu Glu Asn
   1745 1750 1755
   Lys Val Ser Gln Val Lys Ile Arg Phe Val Asn Val Phe Lys Asp
   1760 1765 1770
   Lys Thr Leu Ala Asn Lys Leu Ser Phe Asn Phe Ser Asp Lys Gln
   1775 1780 1785
   Asp Val Pro Val Ser Glu Ile Ile Leu Ser Phe Thr Pro Ser Tyr
   1790 1795 1800
   Tyr Glu Asp Gly Leu Ile Gly Tyr Asp Leu Gly Leu Val Ser Leu
   1805 1810 1815
   Tyr Asn Glu Lys Phe Tyr Ile Asn Asn Phe Gly Met Met Val Ser
   1820 1825 1830
   Gly Leu Ile Tyr Ile Asn Asp Ser Leu Tyr Tyr Phe Lys Pro Pro
   1835 1840 1845
   Val Asn Asn Leu Ile Thr Gly Phe Val Thr Val Gly Asp Asp Lys
   1850 1855 1860
   Tyr Tyr Phe Asn Pro Ile Asn Gly Gly Ala Ala Ser Ile Gly Glu
   1865 1870 1875
   Thr Ile Ile Asp Asp Lys Asn Tyr Tyr Phe Asn Gln Ser Gly Val
   1880 1885 1890
   Leu Gln Thr Gly Val Phe Ser Thr Glu Asp Gly Phe Lys Tyr Phe
   1895 1900 1905
   Ala Pro Ala Asn Thr Leu Asp Glu Asn Leu Glu Gly Glu Ala Ile
   1910 1915 1920
   Asp Phe Thr Gly Lys Leu Ile Ile Asp Glu Asn Ile Tyr Tyr Phe
   1925 1930 1935
   Asp Asp Asn Tyr Arg Gly Ala Val Glu Trp Lys Glu Leu Asp Gly
   1940 1945 1950
   Glu Met His Tyr Phe Ser Pro Glu Thr Gly Lys Ala Phe Lys Gly
   1955 1960 1965
   Leu Asn Gln Ile Gly Asp Tyr Lys Tyr Tyr Phe Asn Ser Asp Gly
   1970 1975 1980
   Val Met Gln Lys Gly Phe Val Ser Ile Asn Asp Asn Lys His Tyr
   1985 1990 1995
   Phe Asp Asp Ser Gly Val Met Lys Val Gly Tyr Thr Glu Ile Asp
   2000 2005 2010
   Gly Lys His Phe Tyr Phe Ala Glu Asn Gly Glu Met Gln Ile Gly
   2015 2020 2025
   Val Phe Asn Thr Glu Asp Gly Phe Lys Tyr Phe Ala His His Asn
   2030 2035 2040
   Glu Asp Leu Gly Asn Glu Glu Gly Glu Glu Ile Ser Tyr Ser Gly
   2045 2050 2055
   Ile Leu Asn Phe Asn Asn Lys Ile Tyr Tyr Phe Asp Asp Ser Phe
   2060 2065 2070
   Thr Ala Val Val Gly Trp Lys Asp Leu Glu Asp Gly Ser Lys Tyr
   2075 2080 2085
   Tyr Phe Asp Glu Asp Thr Ala Glu Ala Tyr Ile Gly Leu Ser Leu
   2090 2095 2100
   Ile Asn Asp Gly Gln Tyr Tyr Phe Asn Asp Asp Gly Ile Met Gln
   2105 2110 2115
   Val Gly Phe Val Thr Ile Asn Asp Lys Val Phe Tyr Phe Ser Asp
   2120 2125 2130
   Ser Gly Ile Ile Glu Ser Gly Val Gln Asn Ile Asp Asp Asn Tyr
   2135 2140 2145
   Phe Tyr Ile Asp Asp Asn Gly Ile Val Gln Ile Gly Val Phe Asp
   2150 2155 2160
   Thr Ser Asp Gly Tyr Lys Tyr Phe Ala Pro Ala Asn Thr Val Asn
   2165 2170 2175
   Asp Asn Ile Tyr Gly Gln Ala Val Glu Tyr Ser Gly Leu Val Arg
   2180 2185 2190
   Val Gly Glu Asp Val Tyr Tyr Phe Gly Glu Thr Tyr Thr Ile Glu
   2195 2200 2205
   Thr Gly Trp Ile Tyr Asp Met Glu Asn Glu Ser Asp Lys Tyr Tyr
   2210 2215 2220
   Phe Asn Pro Glu Thr Lys Lys Ala Cys Lys Gly Ile Asn Leu Ile
   2225 2230 2235
   Asp Asp Ile Lys Tyr Tyr Phe Asp Glu Lys Gly Ile Met Arg Thr
   2240 2245 2250
   Gly Leu  Ile Ser Phe Glu Asn  Asn Asn Tyr Tyr Phe  Asn Glu Asn
       2255                 2260                 2265
   Gly Glu  Met Gln Phe Gly Tyr  Ile Asn Ile Glu Asp  Lys Met Phe
       2270                 2275                 2280
   Tyr Phe  Gly Glu Asp Gly Val  Met Gln Ile Gly Val  Phe Asn Thr
       2285                 2290                 2295
   Pro Asp  Gly Phe Lys Tyr Phe  Ala His Gln Asn Thr  Leu Asp Glu
       2300                 2305                 2310
   Asn Phe  Glu Gly Glu Ser Ile  Asn Tyr Thr Gly Trp  Leu Asp Leu
       2315                 2320                 2325
   Asp Glu  Lys Arg Tyr Tyr Phe  Thr Asp Glu Tyr Ile  Ala Ala Thr
       2330                 2335                 2340
   Gly Ser  Val Ile Ile Asp Gly  Glu Glu Tyr Tyr Phe  Asp Pro Asp
       2345                 2350                 2355
   Thr Ala  Gln Leu Val Ile Ser  Glu
       2360                 2365
<210> 15
   <211> 25
   <212> PRT
   <213> Clostridium novyi
<300>
   <308> Q46149
   <309> 1996-11-01
   <313> (1641)..(1665)
<400> 15
   Cys Asn Val Ile Val Ser Gly Ser Asn Lys Leu Asn Ser Glu Gly Asp
   1               5                   10                  15
   Leu Ala Asp Thr Ile Asp Val Leu Asp
               20                  25
<210> 16
   <211> 2178
   <212> PRT
   <213> Clostridium novyi
<300>
   <308> Q46149
   <309> 1996-11-01
   <313> (1)..(2178)
<400> 16
   Met Leu Ile Thr Arg Glu Gln Leu Met Lys Ile Ala Ser Ile Pro Leu
   1               5                   10                  15
   Lys Arg Lys Glu Pro Glu Tyr Asn Leu Ile Leu Asp Ala Leu Glu Asn
               20                  25                  30
   Phe Asn Arg Asp Ile Glu Gly Thr Ser Val Lys Glu Ile Tyr Ser Lys
           35                  40                  45
   Leu Ser Lys Leu Asn Glu Leu Val Asp Asn Tyr Gln Thr Lys Tyr Pro
       50                  55                  60
   Ser Ser Gly Arg Asn Leu Ala Leu Glu Asn Phe Arg Asp Ser Leu Tyr
   65                  70                  75                  80
   Ser Glu Leu Arg Glu Leu Ile Lys Asn Ser Arg Thr Ser Thr Ile Ala
                   85                  90                  95
   Ser Lys Asn Leu Ser Phe Ile Trp Ile Gly Gly Pro Ile Ser Asp Gln
               100                 105                 110
   Ser Leu Glu Tyr Tyr Asn Met Trp Lys Met Phe Asn Lys Asp Tyr Asn
           115                 120                 125
   Ile Arg Leu Phe Tyr Asp Lys Asn Ser Leu Leu Val Asn Thr Leu Lys
       130                 135                 140
   Thr Ala Ile Ile Gln Glu Ser Ser Lys Val Ile Ile Glu Gln Asn Gln
   145                 150                 155                 160
   Ser Asn Ile Leu Asp Gly Thr Tyr Gly His Asn Lys Phe Tyr Ser Asp
                   165                 170                 175
   Arg Met Lys Leu Ile Tyr Arg Tyr Lys Arg Glu Leu Lys Met Leu Tyr
               180                 185                 190
   Glu Asn Met Lys Gln Asn Asn Ser Val Asp Asp Ile Ile Ile Asn Phe
           195                 200                 205
   Leu Ser Asn Tyr Phe Lys Tyr Asp Ile Gly Lys Leu Asn Asn Gln Lys
       210                 215                 220
   Glu Asn Asn Asn Asn Lys Met Ile Ala Ile Gly Ala Thr Asp Ile Asn
   225                 230                 235                 240
   Thr Glu Asn Ile Leu Thr Asn Lys Leu Lys Ser Tyr Tyr Tyr Gln Glu
                   245                 250                 255
   Leu Ile Gln Thr Asn Asn Leu Ala Ala Ala Ser Asp Ile Leu Arg Ile
               260                 265                 270
   Ala Ile Leu Lys Lys Tyr Gly Gly Val Tyr Cys Asp Leu Asp Phe Leu
           275                 280                 285
   Pro Gly Val Asn Leu Ser Leu Phe Asn Asp Ile Ser Lys Pro Asn Gly
   290 295 300
   Met Asp Ser Asn Tyr Trp Glu Ala Ala Ile Phe Glu Ala Ile Ala Asn
   305 310 315 320
   Glu Lys Lys Leu Met Asn Asn Tyr Pro Tyr Lys Tyr Met Glu Gln Val
   325 330 335
   Pro Ser Glu Ile Lys Glu Arg Ile Leu Ser Phe Val Arg Asn His Asp
   340 345 350
   Ile Asn Asp Leu Ile Leu Pro Leu Gly Asp Ile Lys Ile Ser Gln Leu
   355 360 365
   Glu Ile Leu Leu Ser Arg Leu Lys Ala Ala Thr Gly Lys Lys Thr Phe
   370 375 380
   Ser Asn Ala Phe Ile Ile Ser Asn Asn Asp Ser Leu Thr Leu Asn Asn
   385 390 395 400
   Leu Ile Ser Gln Leu Glu Asn Arg Tyr Glu Ile Leu Asn Ser Ile Ile
   405 410 415
   Gln Glu Lys Phe Lys Ile Cys Glu Thr Tyr Asp Ser Tyr Ile Asn Ser
   420 425 430
   Val Ser Glu Leu Val Leu Glu Thr Thr Pro Lys Asn Leu Ser Met Asp
   435 440 445
   Gly Ser Ser Phe Tyr Gln Gln Ile Ile Gly Tyr Leu Ser Ser Gly Phe
   450 455 460
   Lys Pro Glu Val Asn Ser Thr Val Phe Phe Ser Gly Pro Asn Ile Tyr
   465 470 475 480
   Ser Ser Ala Thr Cys Asp Thr Tyr His Phe Ile Lys Asn Thr Phe Asp
   485 490 495
   Met Leu Ser Ser Gln Asn Gln Glu Ile Phe Glu Ala Ser Asn Asn Leu
   500 505 510
   Tyr Phe Ser Lys Thr His Asp Glu Phe Lys Ser Ser Trp Leu Leu Arg
   515 520 525
   Ser Asn Ile Ala Glu Lys Glu Phe Gln Lys Leu Ile Lys Thr Tyr Ile
   530 535 540
   Gly Arg Thr Leu Asn Tyr Glu Asp Gly Leu Asn Phe Asn Lys Trp Lys
   545 550 555 560
   Arg Val Thr Thr Ser Glu Leu Leu Lys Val Ile Glu Glu Val Asn Ser
   565 570 575
   Thr Lys Ile Tyr Glu Asn Tyr Asp Leu Asn Met Ile Leu Gln Ile Gln
   580 585 590
   Gly Asp Asp Ile Ser Tyr Glu Ser Ala Val Asn Val Phe Gly Lys Asn
   595 600 605
   Pro Asn Lys Ser Ile Leu Ile Gln Gly Val Asp Asp Phe Ala Asn Val
   610 615 620
   Phe Tyr Phe Glu Asn Gly Ile Val Gln Ser Asp Asn Ile Asn Asn Ile
   625 630 635 640
   Leu Ser Arg Phe Asn Asp Ile Lys Lys Ile Lys Leu Thr Leu Ile Gly
   645 650 655
   His Gly Glu Asn Val Phe Asn Pro Lys Leu Phe Gly Gly Lys Thr Val
   660 665 670
   Asn Asp Leu Tyr Thr Asn Ile Ile Lys Pro Lys Leu Gln His Leu Leu
   675 680 685
   Glu Arg Glu Gly Val Ile Leu Lys Asn Lys Tyr Leu Lys Ile Asn Ile
   690 695 700
   Leu Gly Cys Tyr Met Phe Thr Pro Lys Val Asp Ile Asn Ser Thr Phe
   705 710 715 720
   Val Gly Lys Leu Phe Asn Lys Ile Ser Arg Asp Leu Gln Pro Lys Gly
   725 730 735
   Phe Ser Lys Asn Gln Leu Glu Ile Ser Ala Asn Lys Tyr Ala Ile Arg
   740 745 750
   Ile Asn Arg Glu Gly Lys Arg Glu Val Leu Asp Tyr Phe Gly Lys Trp
   755 760 765
   Val Ser Asn Thr Asp Leu Ile Ala Glu Gln Ile Ser Asn Lys Tyr Val
   770 775 780
   Val Tyr Trp Asn Glu Val Glu Asn Thr Leu Ser Ala Arg Val Glu Gln
   785 790 795 800
   Leu Asn Lys Val Ala Glu Phe Ala Lys Asp Ile Asn Ser Ile Ile Gln
   805 810 815
   Thr Thr Asn Asn Gln Glu Leu Lys Gln Ser Leu Val Asn Thr Tyr Ala
   820 825 830
   Asp Leu Ile Thr Thr Leu Tyr Ser Glu Leu Leu Lys Glu Asp Ile Pro
   835 840 845
   Phe Glu Leu Asp Asn Ile Gln Ile Lys Glu Arg Ile Ile Leu Asn Glu
   850 855 860
   Ile Ser Arg Leu His Asp Phe Ser Asn Ile Ile Leu Asp Phe Tyr Gln
   865 870 875 880
   Lys Asn Asn Ile Ser Asn Asn Met Ile Ile Leu Phe Asp Ser Ile Ile
   885 890 895
   Lys Glu Lys Asp Tyr Tyr Asn Val Lys Leu Ala Asn Lys Ile Thr Gly
   900 905 910
   Glu Thr Ser Val Ile Lys Thr Tyr Ser Asp Ser Leu Trp Asn Phe Thr
   915 920 925
   Asn Lys Tyr Lys Lys Ile Val Asp Asp Ile Lys Gly Ile Ile Val Lys
   930 935 940
   Asp Ile Asn Gly Glu Phe Ile Lys Lys Ala Asp Phe Glu Ile Glu Gln
   945 950 955 960
   Asn Pro Ser Leu Leu Asn Ser Ala Met Leu Met Gln Leu Leu Ile Asp
   965 970 975
   Tyr Lys Pro Tyr Thr Glu Ile Leu Thr Asn Met Asn Thr Ser Leu Lys
   980 985 990
   Val Gln Ala Tyr Ala Gln Ile Phe Gln Leu Ser Ile Gly Ala Ile Gln
   995 1000 1005
   Glu Ala Thr Glu Ile Val Thr Ile Ile Ser Asp Ala Leu Asn Ala
   1010 1015 1020
   Asn Phe Asn Ile Leu Ser Lys Leu Lys Val Gly Ser Ser Val Ala
   1025 1030 1035
   Ser Val Ile Ile Asp Gly Ile Asn Leu Ile Ala Ala Leu Thr Glu
   1040 1045 1050
   Leu Lys Asn Val Lys Thr Asn Phe Glu Arg Lys Leu Ile Glu Ala
   1055 1060 1065
   Lys Val Gly Met Tyr Ser Ile Gly Phe Ile Leu Glu Ser Ser Ser
   1070 1075 1080
   Leu Ile Ser Gly Leu Leu Gly Ala Thr Ala Val Ser Glu Ile Leu
   1085 1090 1095
   Gly Val Ile Ser Val Pro Val Ala Gly Ile Leu Val Gly Leu Pro
   1100 1105 1110
   Ser Leu Val Asn Asn Ile Leu Val Leu Gly Glu Lys Tyr Asn Gln
   1115 1120 1125
   Ile Leu Asp Tyr Phe Ser Lys Phe Tyr Pro Ile Val Gly Lys Asn
   1130 1135 1140
   Pro Phe Ser Ile Gln Asp Asn Ile Ile Ile Pro Tyr Asp Asp Ile
   1145 1150 1155
   Ala Ile Thr Glu Leu Asn Phe Lys Tyr Asn Lys Phe Lys Tyr Gly
   1160 1165 1170
   Tyr Ala Lys Ile Ser Gly Leu Lys Val Gly Leu Val Thr His Ile
   1175 1180 1185
   Gly Glu Asn Ile Asp His Tyr Phe Ser Ala Pro Ser Leu Asp His
   1190 1195 1200
   Tyr Ile Glu Leu Ser Ile Tyr Pro Ala Leu Lys Leu Asn Asp Thr
   1205 1210 1215
   Asn Leu Pro Lys Gly Asn Val Val Leu Leu Pro Ser Gly Leu Asn
   1220 1225 1230
   Lys Val Tyr Lys Pro Glu Ile Ser Ala Ile Ala Gly Ala Asn Ser
   1235 1240 1245
   Gln Glu Gly Asn Gly Val Glu Val Leu Asn Leu Ile Arg Asn Tyr
   1250 1255 1260
   Tyr Val Asp Ser Asn Gly Asn Thr Lys Phe Pro Trp Lys Tyr Glu
   1265 1270 1275
   Ala Pro Phe Glu Tyr Ser Phe Ser Tyr Met Arg Val Glu Tyr Phe
   1280 1285 1290
   Asp Thr Lys Val Asn Val Ile Leu Asp Asn Glu Asn Lys Thr Leu
   1295 1300 1305
   Ile Ile Pro Val Leu Thr Ile Asp Glu Met Arg Asn Lys Ile Ser
   1310 1315 1320
   Tyr Glu Ile Leu Gly Asp Gly Gly Gln Tyr Asn Val Ile Leu Pro
   1325 1330 1335
   Val Asn Gln Thr Asn Ile Asn Ile Val Ser Asn Lys Asn Asp Ile
   1340 1345 1350
   Trp Asn Phe Asp Val Ser Tyr Ile Val Lys Glu Ser Lys Ile Glu
   1355 1360 1365
   Asp Asn Lys Phe Val Leu Asp Gly Phe Ile Asn Asn Ile Phe Ser
   1370 1375 1380
   Thr Leu Lys Val Ser Asn Asp Gly Phe Lys Ile Gly Lys Gln Phe
   1385 1390 1395
   Ile Ser Ile Lys Asn Thr Pro Arg Ala Ile Asn Leu Ser Phe Lys
   1400 1405 1410
   Ile Asn Asn Asn Ile Val Ile Val Ser Ile Tyr Leu Asn His Glu
   1415 1420 1425
   Lys Ser Asn Ser Ile Thr Ile Ile Ser Ser Asp Leu Asn Asp Ile
   1430 1435 1440
   Lys Asn Asn Phe Asp Asn Leu Leu Asp Asn Ile Asn Tyr Ile Gly
   1445 1450 1455
   Leu Gly Ser Ile Ser Asp Asn Thr Ile Asn Cys Ile Val Arg Asn
   1460 1465 1470
   Asp Glu Val Tyr Met Glu Gly Lys Ile Phe Leu Asn Glu Lys Lys
   1475 1480 1485
   Leu Val Phe Ile Gln Asn Glu Leu Glu Leu His Leu Tyr Asp Ser
   1490 1495 1500
   Val Asn Lys Asp Ser Gln Tyr Leu Ile Asn Asn Pro Ile Asn Asn
   1505 1510 1515
   Val Val Lys Tyr Lys Asp Gly Tyr Ile Val Glu Gly Thr Phe Leu
   1520 1525 1530
   Ile Asn Ser Thr Glu Asn Lys Tyr Ser Leu Tyr Ile Glu Asn Asn
   1535 1540 1545
   Lys Ile Met Leu Lys Gly Leu Tyr Leu Glu Ser Ser Val Phe Lys
   1550 1555 1560
   Thr Ile Gln Asp Lys Ile Tyr Ser Lys Glu Lys Val Asn Asp Tyr
   1565 1570 1575
   Ile Leu Ser Leu Ile Lys Lys Phe Phe Thr Val Asn Ile Gln Leu
   1580 1585 1590
   Cys Pro Phe Met Ile Val Ser Gly Val Asp Glu Asn Asn Arg Tyr
   1595 1600 1605
   Leu Glu Tyr Met Leu Ser Thr Asn Asn Lys Trp Ile Ile Asn Gly
       1610                1615                1620
   Gly Tyr Trp Glu Asn Asp Phe Asn Asn Tyr Lys Ile Val Asp Phe
       1625                1630                1635
   Glu Lys Cys Asn Val Ile Val Ser Gly Ser Asn Lys Leu Asn Ser
       1640                1645                1650
   Glu Gly Asp Leu Ala Asp Thr Ile Asp Val Leu Asp Lys Asp Leu
       1655                1660                1665
   Glu Asn Leu Tyr Ile Asp Ser Val Ile Ile Ile Pro Lys Val Tyr
       1670                1675                1680
   Thr Lys Lys Ile Ile Ile His Pro Ile Pro Asn Asn Pro Gln Ile
       1685                1690                1695
   Asn Ile Ile Asn Thr Gln Ser Ile His Asp Lys Cys His Leu Ile
       1700                1705                1710
   Ile Asp Ser Val Leu Thr Asn Asn Tyr His Trp Glu Ser Asp Gly
       1715                1720                1725
   Asp Asp Leu Ile Ile Thr Asn Gly Leu Asp Ile Asn Ile Arg Ile
       1730                1735                1740
   Leu Gln Gly Leu Ser Phe Gly Phe Lys Tyr Lys Asn Ile Tyr Leu
       1745                1750                1755
   Lys Phe Ser Asn Tyr Asp Glu Leu Ser Leu Asn Asp Phe Leu Leu
       1760                1765                1770
   Gln Asn Tyr Asn Val Lys Gly Leu Tyr Tyr Ile Asn Gly Glu Leu
       1775                1780                1785
   His Tyr Lys Asn Ile Pro Gly Asp Thr Phe Glu Tyr Gly Trp Ile
       1790                1795                1800
   Asn Ile Asp Ser Arg Trp Tyr Phe Phe Asp Ser Ile Asn Leu Ile
       1805                1810                1815
   Ala Lys Lys Gly Tyr Gln Glu Ile Glu Gly Glu Arg Tyr Tyr Phe
       1820                1825                1830
   Asn Pro Asn Thr Gly Val Gln Glu Ser Gly Val Phe Leu Thr Pro
       1835                1840                1845
   Asn Gly Leu Glu Tyr Phe Thr Asn Lys His Ala Ser Ser Lys Arg
       1850                1855                1860
   Trp Gly Arg Ala Ile Asn Tyr Thr Gly Trp Leu Thr Leu Asp Gly
   1865 1870 1875
   Asn Lys Tyr Tyr Phe Gln Ser Asn Ser Lys Ala Val Thr Gly Leu
   1880 1885 1890
   Gln Lys Ile Ser Asp Lys Tyr Tyr Tyr Phe Asn Asp Asn Gly Gln
   1895 1900 1905
   Met Gln Ile Lys Trp Gln Ile Ile Asn Asn Asn Lys Tyr Tyr Phe
   1910 1915 1920
   Asp Gly Asn Thr Gly Glu Ala Ile Ile Gly Trp Phe Asn Asn Asn
   1925 1930 1935
   Lys Glu Arg Tyr Tyr Phe Asp Ser Glu Gly Arg Leu Leu Thr Gly
   1940 1945 1950
   Tyr Gln Val Ile Gly Asp Lys Ser Tyr Tyr Phe Ser Asp Asn Ile
   1955 1960 1965
   Asn Gly Asn Trp Glu Glu Gly Ser Gly Val Leu Lys Ser Gly Ile
   1970 1975 1980
   Phe Lys Thr Pro Ser Gly Phe Lys Leu Phe Ser Ser Glu Gly Asp
   1985 1990 1995
   Lys Ser Ala Ile Asn Tyr Lys Gly Trp Leu Asp Leu Asn Gly Asn
   2000 2005 2010
   Lys Tyr Tyr Phe Asn Ser Asp Ser Ile Ala Val Thr Gly Ser Tyr
   2015 2020 2025
   Asn Ile Lys Gly Ile Gln Tyr Tyr Phe Asn Pro Lys Thr Ala Val
   2030 2035 2040
   Leu Thr Asn Gly Trp Tyr Thr Leu Asp Asn Asn Asn Tyr Tyr Val
   2045 2050 2055
   Ser Asn Gly His Asn Val Leu Gly Tyr Gln Asp Ile Asp Gly Lys
   2060 2065 2070
   Gly Tyr Tyr Phe Asp Pro Ser Thr Gly Ile Gln Lys Ala Gly Val
   2075 2080 2085
   Phe Pro Thr Pro Asn Gly Leu Arg Tyr Phe Thr Met Lys Pro Ile
   2090 2095 2100
   Asp Gly Gln Arg Trp Gly Gln Cys Ile Asp Tyr Thr Gly Trp Leu
   2105 2110 2115
   His Leu Asn Gly Asn Lys Tyr Tyr Phe Gly Tyr Tyr Asn Ser Ala
   2120 2125 2130
   Val Thr Gly Trp Arg Val Leu Gly Gly Lys Arg Tyr Phe Phe Asn
   2135 2140 2145
   Ile Lys Thr Gly Ala Ala Thr Thr Gly Leu Leu Thr Leu Ser Gly
   2150 2155 2160
   Lys Arg Tyr Tyr Phe Asn Glu Lys Gly Glu Gln Leu Thr Leu Val
   2165 2170 2175
<210> 17
   <211> 3
   <212> PRT
   <213> Clostridium sordellii
<300>
   <308> Q46342
   <309> 1996-11-01
   <313> (1666)..(1668)
<400> 17
   Asp Ser Gly
   1
<210> 18
   <211> 26
   <212> PRT
   <213> Clostridium sordellii
<300>
   <308> Q46342
   <309> 1996-11-01
   <313> (1654)..(1679)
<400> 18
   Gln Asn Leu Ile Val Glu Pro Ser Tyr His Leu Asp Asp Ser Gly Asn
   1 5 10 15
   Ile Ser Ser Thr Val Ile Asn Phe Ser Gln
   20 25
<210> 19
   <211> 2364
   <212> PRT
   <213> Clostridium sordellii
<220>
   <221> MISC_FEATURE
   <222> (1666)..(1668)
   <223> DXG motive
<300>
   <308> Q46342
   <309> 1996-11-01
   <313> (1)..(2364)
<400> 19
   Met Asn Leu Val Asn Lys Ala Gln Leu Gln Lys Met Val Tyr Val Lys
   1               5                   10                  15
   Phe Arg Ile Gln Glu Asp Glu Tyr Val Ala Ile Leu Asn Ala Leu Glu
               20                  25                  30
   Glu Tyr His Asn Met Ser Glu Ser Ser Val Val Glu Lys Tyr Leu Lys
           35                  40                  45
   Leu Lys Asp Ile Asn Asn Leu Thr Asp Asn Tyr Leu Asn Thr Tyr Lys
       50                  55                  60
   Lys Ser Gly Arg Asn Lys Ala Leu Lys Lys Phe Lys Glu Tyr Leu Thr
   65                  70                  75                  80
   Met Glu Val Leu Glu Leu Lys Asn Asn Ser Leu Thr Pro Val Glu Lys
                   85                  90                  95
   Asn Leu His Phe Ile Trp Ile Gly Gly Gln Ile Asn Asp Thr Ala Ile
               100                 105                 110
   Asn Tyr Ile Asn Gln Trp Lys Asp Val Asn Ser Asp Tyr Thr Val Lys
           115                 120                 125
   Val Phe Tyr Asp Ser Asn Ala Phe Leu Ile Asn Thr Leu Lys Lys Thr
       130                 135                 140
   Ile Val Glu Ser Ala Thr Asn Asn Thr Leu Glu Ser Phe Arg Glu Asn
   145                 150                 155                 160
   Leu Asn Asp Pro Glu Phe Asp Tyr Asn Lys Phe Tyr Arg Lys Arg Met
                   165                 170                 175
   Glu Ile Ile Tyr Asp Lys Gln Lys His Phe Ile Asp Tyr Tyr Lys Ser
               180                 185                 190
   Gln Ile Glu Glu Asn Pro Glu Phe Ile Ile Asp Asn Ile Ile Lys Thr
           195                 200                 205
   Tyr Leu Ser Asn Glu Tyr Ser Lys Asp Leu Glu Ala Leu Asn Lys Tyr
       210                 215                 220
   Ile Glu Glu Ser Leu Asn Lys Ile Thr Ala Asn Asn Gly Asn Asp Ile
   225                 230                 235                 240
   Arg Asn Leu Glu Lys Phe Ala Asp Glu Asp Leu Val Arg Leu Tyr Asn
                   245                 250                 255
   Gln Glu Leu Val Glu Arg Trp Asn Leu Ala Ala Ala Ser Asp Ile Leu
               260                 265                 270
   Arg Ile Ser Met Leu Lys Glu Asp Gly Gly Val Tyr Leu Asp Val Asp
           275                 280                 285
   Ile Leu Pro Gly Ile Gln Pro Asp Leu Phe Lys Ser Ile Asn Lys Pro
       290                 295                 300
   Asp Ser Ile Thr Asn Thr Ser Trp Glu Met Ile Lys Leu Glu Ala Ile
   305                 310                 315                 320
   Met Lys Tyr Lys Glu Tyr Ile Pro Gly Tyr Thr Ser Lys Asn Phe Asp
                   325                 330                 335
   Met Leu Asp Glu Glu Val Gln Arg Ser Phe Glu Ser Ala Leu Ser Ser
               340                 345                 350
   Lys Ser Asp Lys Ser Glu Ile Phe Leu Pro Leu Asp Asp Ile Lys Val
           355                 360                 365
   Ser Pro Leu Glu Val Lys Ile Ala Phe Ala Asn Asn Ser Val Ile Asn
       370                 375                 380
   Gln Ala Leu Ile Ser Leu Lys Asp Ser Tyr Cys Ser Asp Leu Val Ile
   385                 390                 395                 400
   Asn Gln Ile Lys Asn Arg Tyr Lys Ile Leu Asn Asp Asn Leu Asn Pro
                   405                 410                 415
   Ser Ile Asn Glu Gly Thr Asp Phe Asn Thr Thr Met Lys Ile Phe Ser
               420                 425                 430
   Asp Lys Leu Ala Ser Ile Ser Asn Glu Asp Asn Met Met Phe Met Ile
           435                 440                 445
   Lys Ile Thr Asn Tyr Leu Lys Val Gly Phe Ala Pro Asp Val Arg Ser
       450                 455                 460
   Thr Ile Asn Leu Ser Gly Pro Gly Val Tyr Thr Gly Ala Tyr Gln Asp
   465                 470                 475                 480
   Leu Leu Met Phe Lys Asp Asn Ser Thr Asn Ile His Leu Leu Glu Pro
                   485                 490                 495
   Glu Leu Arg Asn Phe Glu Phe Pro Lys Thr Lys Ile Ser Gln Leu Thr
               500                 505                 510
   Glu Gln Glu Ile Thr Ser Leu Trp Ser Phe Asn Gln Ala Arg Ala Lys
           515                 520                 525
   Ser Gln Phe Glu Glu Tyr Lys Lys Gly Tyr Phe Glu Gly Ala Leu Gly
   530 535 540
   Glu Asp Asp Asn Leu Asp Phe Ala Gln Asn Thr Val Leu Asp Lys Asp
   545 550 555 560
   Tyr Val Ser Lys Lys Ile Leu Ser Ser Met Lys Thr Arg Asn Lys Glu
   565 570 575
   Tyr Ile His Tyr Ile Val Gln Leu Gln Gly Asp Lys Ile Ser Tyr Glu
   580 585 590
   Ala Ser Cys Asn Leu Phe Ser Lys Asp Pro Tyr Ser Ser Ile Leu Tyr
   595 600 605
   Gln Lys Asn Ile Glu Gly Ser Glu Thr Ala Tyr Tyr Tyr Tyr Val Ala
   610 615 620
   Asp Ala Glu Ile Lys Glu Ile Asp Lys Tyr Arg Ile Pro Tyr Gln Ile
   625 630 635 640
   Ser Asn Lys Arg Asn Ile Lys Leu Thr Phe Ile Gly His Gly Lys Ser
   645 650 655
   Glu Phe Asn Thr Asp Thr Phe Ala Asn Leu Asp Val Asp Ser Leu Ser
   660 665 670
   Ser Glu Ile Glu Thr Ile Leu Asn Leu Ala Lys Ala Asp Ile Ser Pro
   675 680 685
   Lys Tyr Ile Glu Ile Asn Leu Leu Gly Cys Asn Met Phe Ser Tyr Ser
   690 695 700
   Ile Ser Ala Glu Glu Thr Tyr Pro Gly Lys Leu Leu Leu Lys Ile Lys
   705 710 715 720
   Asp Arg Val Ser Glu Leu Met Pro Ser Ile Ser Gln Asp Ser Ile Thr
   725 730 735
   Val Ser Ala Asn Gln Tyr Glu Val Arg Ile Asn Glu Glu Gly Lys Arg
   740 745 750
   Glu Ile Leu Asp His Ser Gly Lys Trp Ile Asn Lys Glu Glu Ser Ile
   755 760 765
   Ile Lys Asp Ile Ser Ser Lys Glu Tyr Ile Ser Phe Asn Pro Lys Glu
   770 775 780
   Asn Lys Ile Ile Val Lys Ser Lys Tyr Leu His Glu Leu Ser Thr Leu
   785 790 795 800
   Leu Gln Glu Ile Arg Asn Asn Ala Asn Ser Ser Asp Ile Asp Leu Glu
   805 810 815
   Lys Lys Val Met Leu Thr Glu Cys Glu Ile Asn Val Ala Ser Asn Ile
   820 825 830
   Asp Arg Gln Ile Val Glu Gly Arg Ile Glu Glu Ala Lys Asn Leu Thr
   835 840 845
   Ser Asp Ser Ile Asn Tyr Ile Lys Asn Glu Phe Lys Leu Ile Glu Ser
   850 855 860
   Ile Ser Asp Ser Leu Tyr Asp Leu Lys His Gln Asn Gly Leu Asp Asp
   865 870 875 880
   Ser His Phe Ile Ser Phe Glu Asp Ile Ser Lys Thr Glu Asn Gly Phe
   885 890 895
   Arg Ile Arg Phe Ile Asn Lys Glu Thr Gly Asn Ser Ile Phe Ile Glu
   900 905 910
   Thr Glu Lys Glu Ile Phe Ser Glu Tyr Ala Thr His Ile Ser Lys Glu
   915 920 925
   Ile Ser Asn Ile Lys Asp Thr Ile Phe Asp Asn Val Asn Gly Lys Leu
   930 935 940
   Val Lys Lys Val Asn Leu Asp Ala Ala His Glu Val Asn Thr Leu Asn
   945 950 955 960
   Ser Ala Phe Phe Ile Gln Ser Leu Ile Glu Tyr Asn Thr Thr Lys Glu
   965 970 975
   Ser Leu Ser Asn Leu Ser Val Ala Met Lys Val Gln Val Tyr Ala Gln
   980 985 990
   Leu Phe Ser Thr Gly Leu Asn Thr Ile Thr Asp Ala Ser Lys Val Val
   995 1000 1005
   Glu Leu Val Ser Thr Ala Leu Asp Glu Thr Ile Asp Leu Leu Pro
   1010 1015 1020
   Thr Leu Ser Glu Gly Leu Pro Ile Ile Ala Thr Ile Ile Asp Gly
   1025 1030 1035
   Val Ser Leu Gly Ala Ala Ile Lys Glu Leu Ser Glu Thr Asn Asp
   1040 1045 1050
   Pro Leu Leu Arg Gln Glu Ile Glu Ala Lys Ile Gly Ile Met Ala
   1055 1060 1065
   Val Asn Leu Thr Ala Ala Ser Thr Ala Ile Val Thr Ser Ala Leu
   1070 1075 1080
   Gly Ile Ala Ser Gly Phe Ser Ile Leu Leu Val Pro Leu Ala Gly
   1085 1090 1095
   Ile Ser Ala Gly Ile Pro Ser Leu Val Asn Asn Glu Leu Ile Leu
   1100 1105 1110
   Gln Asp Lys Ala Thr Lys Val Ile Asp Tyr Phe Lys His Ile Ser
   1115 1120 1125
   Leu Ala Glu Thr Glu Gly Ala Phe Thr Leu Leu Asp Asp Lys Ile
   1130 1135 1140
   Ile Met Pro Gln Asp Asp Leu Val Leu Ser Glu Ile Asp Phe Asn
   1145 1150 1155
   Asn Asn Ser Ile Thr Leu Gly Lys Cys Glu Ile Trp Arg Ala Glu
   1160 1165 1170
   Gly Gly Ser Gly His Thr Leu Thr Asp Asp Ile Asp His Phe Phe
   1175 1180 1185
   Ser Ser Pro Ser Ile Thr Tyr Arg Lys Pro Trp Leu Ser Ile Tyr
   1190 1195 1200
   Asp Val Leu Asn Ile Lys Lys Glu Lys Ile Asp Phe Ser Lys Asp
   1205 1210 1215
   Leu Met Val Leu Pro Asn Ala Pro Asn Arg Val Phe Gly Tyr Glu
   1220 1225 1230
   Met Gly Trp Thr Pro Gly Phe Arg Ser Leu Asp Asn Asp Gly Thr
   1235 1240 1245
   Lys Leu Leu Asp Arg Ile Arg Asp His Tyr Glu Gly Gln Phe Tyr
   1250 1255 1260
   Trp Arg Tyr Phe Ala Phe Ile Ala Asp Ala Leu Ile Thr Lys Leu
   1265 1270 1275
   Lys Pro Arg Tyr Glu Asp Thr Asn Val Arg Ile Asn Leu Asp Gly
   1280 1285 1290
   Asn Thr Arg Ser Phe Ile Val Pro Val Ile Thr Thr Glu Gln Ile
   1295 1300 1305
   Arg Lys Asn Leu Ser Tyr Ser Phe Tyr Gly Ser Gly Gly Ser Tyr
   1310 1315 1320
   Ser Leu Ser Leu Ser Pro Tyr Asn Met Asn Ile Asp Leu Asn Leu
   1325 1330 1335
   Val Glu Asn Asp Thr Trp Val Ile Asp Val Asp Asn Val Val Lys
   1340 1345 1350
   Asn Ile Thr Ile Glu Ser Asp Glu Ile Gln Lys Gly Glu Leu Ile
   1355 1360 1365
   Glu Asn Ile Leu Ser Lys Leu Asn Ile Glu Asp Asn Lys Ile Ile
   1370 1375 1380
   Leu Asn Asn His Thr Ile Asn Phe Tyr Gly Asp Ile Asn Glu Ser
   1385 1390 1395
   Asn Arg Phe Ile Ser Leu Thr Phe Ser Ile Leu Glu Asp Ile Asn
   1400 1405 1410
   Ile Ile Ile Glu Ile Asp Leu Val Ser Lys Ser Tyr Lys Ile Leu
   1415 1420 1425
   Leu Ser Gly Asn Cys Met Lys Leu Ile Glu Asn Ser Ser Asp Ile
   1430 1435 1440
   Gln Gln Lys Ile Asp His Ile Gly Phe Asn Gly Glu His Gln Lys
   1445 1450 1455
   Tyr Ile Pro Tyr Ser Tyr Ile Asp Asn Glu Thr Lys Tyr Asn Gly
   1460 1465 1470
   Phe Ile Asp Tyr Ser Lys Lys Glu Gly Leu Phe Thr Ala Glu Phe
   1475 1480 1485
   Ser Asn Glu Ser Ile Ile Arg Asn Ile Tyr Met Pro Asp Ser Asn
   1490 1495 1500
   Asn Leu Phe Ile Tyr Ser Ser Lys Asp Leu Lys Asp Ile Arg Ile
   1505 1510 1515
   Ile Asn Lys Gly Asp Val Lys Leu Leu Ile Gly Asn Tyr Phe Lys
   1520 1525 1530
   Asp Asp Met Lys Val Ser Leu Ser Phe Thr Ile Glu Asp Thr Asn
   1535 1540 1545
   Thr Ile Lys Leu Asn Gly Val Tyr Leu Asp Glu Asn Gly Val Ala
   1550 1555 1560
   Gln Ile Leu Lys Phe Met Asn Asn Ala Lys Ser Ala Leu Asn Thr
   1565 1570 1575
   Ser Asn Ser Leu Met Asn Phe Leu Glu Ser Ile Asn Ile Lys Asn
   1580 1585 1590
   Ile Phe Tyr Asn Asn Leu Asp Pro Asn Ile Glu Phe Ile Leu Asp
   1595 1600 1605
   Thr Asn Phe Ile Ile Ser Gly Ser Asn Ser Ile Gly Gln Phe Glu
   1610 1615 1620
   Leu Ile Cys Asp Lys Asp Lys Asn Ile Gln Pro Tyr Phe Ile Asn
   1625 1630 1635
   Phe Lys Ile Lys Glu Thr Ser Tyr Thr Leu Tyr Val Gly Asn Arg
   1640 1645 1650
   Gln Asn Leu Ile Val Glu Pro Ser Tyr His Leu Asp Asp Ser Gly
   1655 1660 1665
   Asn Ile Ser Ser Thr Val Ile Asn Phe Ser Gln Lys Tyr Leu Tyr
   1670 1675 1680
   Gly Ile Asp Arg Tyr Val Asn Lys Val Ile Ile Ala Pro Asn Leu
   1685 1690 1695
   Tyr Thr Asp Glu Ile Asn Ile Thr Pro Val Tyr Lys Pro Asn Tyr
   1700 1705 1710
   Ile Cys Pro Glu Val Ile Ile Leu Asp Ala Asn Tyr Ile Asn Glu
   1715 1720 1725
   Lys Ile Asn Val Asn Ile Asn Asp Leu Ser Ile Arg Tyr Val Trp
   1730 1735 1740
   Asp Asn Asp Gly Ser Asp Leu Ile Leu Ile Ala Asn Ser Glu Glu
   1745 1750 1755
   Asp Asn Gln Pro Gln Val Lys Ile Arg Phe Val Asn Val Phe Lys
   1760 1765 1770
   Ser Asp Thr Ala Ala Asp Lys Leu Ser Phe Asn Phe Ser Asp Lys
   1775 1780 1785
   Gln Asp Val Ser Val Ser Lys Ile Ile Ser Thr Phe Ser Leu Ala
   1790 1795 1800
   Ala Tyr Ser Asp Gly Phe Phe Asp Tyr Glu Phe Gly Leu Val Ser
   1805 1810 1815
   Leu Asp Asn Asp Tyr Phe Tyr Ile Asn Ser Phe Gly Asn Met Val
   1820 1825 1830
   Ser Gly Leu Ile Tyr Ile Asn Asp Ser Leu Tyr Tyr Phe Lys Pro
   1835 1840 1845
   Pro Lys Asn Asn Leu Ile Thr Gly Phe Thr Thr Ile Asp Gly Asn
   1850 1855 1860
   Lys Tyr Tyr Phe Asp Pro Thr Lys Ser Gly Ala Ala Ser Ile Gly
   1865 1870 1875
   Glu Ile Thr Ile Asp Gly Lys Asp Tyr Tyr Phe Asn Lys Gln Gly
   1880 1885 1890
   Ile Leu Gln Val Gly Val Ile Asn Thr Ser Asp Gly Leu Lys Tyr
   1895 1900 1905
   Phe Ala Pro Ala Gly Thr Leu Asp Glu Asn Leu Glu Gly Glu Ser
   1910 1915 1920
   Val Asn Phe Ile Gly Lys Leu Asn Ile Asp Gly Lys Ile Tyr Tyr
   1925 1930 1935
   Phe Glu Asp Asn Tyr Arg Ala Ala Val Glu Trp Lys Leu Leu Asp
   1940 1945 1950
   Asp Glu Thr Tyr Tyr Phe Asn Pro Lys Thr Gly Glu Ala Leu Lys
   1955 1960 1965
   Gly Leu His Gln Ile Gly Asp Asn Lys Tyr Tyr Phe Asp Asp Asn
   1970 1975 1980
   Gly Ile Met Gln Thr Gly Phe Ile Thr Ile Asn Asp Lys Val Phe
   1985 1990 1995
   Tyr Phe Asn Asn Asp Gly Val Met Gln Val Gly Tyr Ile Glu Val
   2000 2005 2010
   Asn Gly Lys Tyr Phe Tyr Phe Gly Lys Asn Gly Glu Arg Gln Leu
   2015 2020 2025
   Gly Val Phe Asn Thr Pro Asp Gly Phe Lys Phe Phe Gly Pro Lys
   2030 2035 2040
   Asp Asp Asp Leu Gly Thr Glu Glu Gly Glu Leu Thr Leu Tyr Asn
   2045 2050 2055
   Gly Ile Leu Asn Phe Asn Gly Lys Ile Tyr Phe Phe Asp Ile Ser
   2060 2065 2070
   Asn Thr Ala Val Val Gly Trp Gly Thr Leu Asp Asp Gly Ser Thr
   2075 2080 2085
   Tyr Tyr Phe Asp Asp Asn Arg Ala Glu Ala Cys Ile Gly Leu Thr
   2090 2095 2100
   Val Ile Asn Asp Cys Lys Tyr Tyr Phe Asp Asp Asn Gly Ile Arg
   2105 2110 2115
   Gln Leu Gly Phe Ile Thr Ile Asn Asp Asn Ile Phe Tyr Phe Ser
   2120 2125 2130
   Glu Ser Gly Lys Ile Glu Leu Gly Tyr Gln Asn Ile Asn Gly Asn
   2135 2140 2145
   Tyr Phe Tyr Ile Asp Glu Ser Gly Leu Val Leu Ile Gly Val Phe
   2150 2155 2160
   Asp Thr Pro Asp Gly Tyr Lys Tyr Phe Ala Pro Leu Asn Thr Val
   2165 2170 2175
   Asn Asp Asn Ile Tyr Gly Gln Ala Val Lys Tyr Ser Gly Leu Val
   2180 2185 2190
   Arg Val Asn Glu Asp Val Tyr Tyr Phe Gly Glu Thr Tyr Lys Ile
   2195 2200 2205
   Glu Thr Gly Trp Ile Glu Asn Glu Thr Asp Lys Tyr Tyr Phe Asp
   2210 2215 2220
   Pro Glu Thr Lys Lys Ala Tyr Lys Gly Ile Asn Val Val Asp Asp
   2225 2230 2235
   Ile Lys Tyr Tyr Phe Asp Glu Asn Gly Ile Met Arg Thr Gly Leu
   2240 2245 2250
   Ile Ser Phe Glu Asn Asn Asn Tyr Tyr Phe Asn Glu Asp Gly Lys
   2255 2260 2265
   Met Gln Phe Gly Tyr Leu Asn Ile Lys Asp Lys Met Phe Tyr Phe
   2270 2275 2280
   Gly Lys Asp Gly Lys Met Gln Ile Gly Val Phe Asn Thr Pro Asp
   2285 2290 2295
   Gly Phe Lys Tyr Phe Ala His Gln Asn Thr Leu Asp Glu Asn Phe
   2300 2305 2310
   Glu Gly Glu Ser Ile Asn Tyr Thr Gly Trp Leu Asp Leu Asp Gly
   2315 2320 2325
   Lys Arg Tyr Tyr Phe Thr Asp Glu Tyr Ile Ala Ala Thr Gly Ser
   2330 2335 2340
   Leu Thr  Ile Asp Gly Tyr Asn  Tyr Tyr Phe Asp Pro  Asp Thr Ala
       2345                 2350                 2355
   Glu Leu  Val Val Ser Glu
       2360